# EUROPÄISCHE PATENTSCHRIFT

(11) **EP 3 880 682 B1**
(45) Veröffentlichungstag und Bekanntmachung des Hinweises auf die Patenterteilung: **14.06.2023**
(21) Anmeldenummer: 19798108.7
(22) Anmeldetag: 11.11.2019
(51) Int. Cl.: C07D 495/04, C07D 495/14, C07D 495/22, C07D 491/14, C07D 487/14, C07D 519/00, C09K 11/06, H10K 50/00

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 15.11.2018 EP 18206493
(43) Veröffentlichungstag der Anmeldung: 22.09.2021
(73) Patentinhaber: Merck Patent GmbH, 64293 Darmstadt (DE)
(72) Erfinder: PARHAM, Amir, 60486 FRANKFURT AM MAIN (DE); EICKHOFF, Christian, 68259 MANNHEIM (DE); LINGE, Rouven, 64291 DARMSTADT (DE); KAISER, Jens, 60489 FRANKFURT AM MAIN (DE); MEKIC, Amel, 64283 DARMSTADT (DE)
(86) Internationale Anmeldenummer: PCT/EP2019/080844
(87) Internationale Veröffentlichungsnummer: WO 2020/099307

(56) Entgegenhaltungen:
- WO-A1-2010/151006
- KR-A- 20180 097 955
- US-A1- 2018 205 022

## Beschreibung

Die vorliegende Erfindung betrifft Materialien für die Verwendung in elektronischen Vorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig phosphoreszierende metallorganische Komplexe eingesetzt. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter, aber auch als Elektronentransportmaterialien, Lochblockiermaterialien, Lochtransportmaterialien, Elektronenblockiermaterialien oder als fluoreszierende Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen und sich gut für die Verwendung in OLEDs eignen. Dabei weisen die OLEDs insbesondere eine lange Lebensdauer, eine hohe Effizienz und eine geringe Betriebsspannung auf. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß Formel (1), 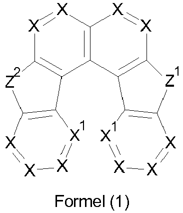 wobei für die verwendeten Symbole gilt:
- X: ist bei jedem Auftreten gleich oder verschieden CR oder N oder zwei benachbarte X stehen für eine Gruppe der folgenden Formel (2), 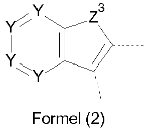 wobei die gestrichelten Bindungen die Verknüpfung dieser Gruppe in der Formel (1) kennzeichnen, mit der Maßgabe, dass die Verbindung der Formel (1) eine oder zwei Gruppen der Formel (2) enthält;
- X¹: ist CH;
- Y: ist bei jedem Auftreten gleich oder verschieden CR oder N;
- Z¹, Z², Z³: sind bei jedem Auftreten gleich oder verschieden O, S, N-Ar oder CR₂;
- Ar: ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann;
- R: ist bei jedem Auftreten gleich oder verschieden H, D, F, N(Ar')₂, CN, OR¹, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl- oder Alkenylgruppe jeweils mit einem oder mehreren Resten R¹ substituiert sein kann, wobei eine oder mehrere nicht benachbarte CH₂-Gruppen durch O ersetzt sein können, oder ein aromatisches oder heteroaromatisches Ringsystem mit 6 bis 30 aromatischen Ringatomen, das jeweils durch einen oder mehrere Reste R¹ substituiert sein kann; dabei können zwei Reste R auch miteinander ein aliphatisches Ringsystem bilden;
- Ar': ist bei jedem Auftreten gleich oder verschieden ein aromatisches oder heteroaromatisches Ringsystem mit 6 bis 30 aromatischen Ringatomen, das durch einen oder mehrere Reste R¹ substituiert sein kann;
- R¹: ist bei jedem Auftreten gleich oder verschieden H, D, F, CN, OR², eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl- oder Alkenylgruppe jeweils mit einem oder mehreren Resten R² substituiert sein kann, wobei eine oder mehrere nicht benachbarte CH₂-Gruppen durch O ersetzt sein können, oder ein aromatisches oder heteroaromatisches Ringsystem mit 6 bis 30 aromatischen Ring-atomen, das jeweils durch einen oder mehrere Reste R² substituiert sein kann; dabei können zwei oder mehrere Reste R¹ miteinander ein aliphatisches Ringsystem bilden;
- R²: ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können.

Eine Arylgruppe im Allgemeinen enthält 6 bis 40 C-Atome; eine Heteroarylgruppe im Allgemeinen enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Arvl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Allgemeinen enthält 6 bis 60 C-Atome, bevorzugt 6 bis 40 C-Atome im Ringsystem. Ein heteroaromatisches Ringsystem im Allgemeinen enthält 2 bis 60 C-Atome, bevorzugt 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. Ebenso sollen hierunter Systeme verstanden werden, in denen zwei oder mehr Aryl- bzw. Heteroarylgruppen direkt miteinander verknüpft sind, wie z. B. Biphenyl, Terphenyl, Bipyridin oder Phenylpyridin. So sollen beispielsweise auch Systeme wie Fluoren, 9,9'-Spirobifluoren, 9,9-Diarylfluoren, Triarylamin, Diarylether, Stilben, etc. als aromatische Ringsysteme verstanden werden, und ebenso Systeme, in denen zwei oder mehrere Arylgruppen beispielsweise durch eine kurze Alkylgruppe verbunden sind. Bevorzugte aromatische bzw. heteroaromatische Ringsysteme sind einfache Aryl- bzw. Heteroarylqruppen sowie Gruppen, in denen zwei oder mehr Aryl- bzw. Heteroarylgruppen direkt miteinander verknüpft sind, beispielsweise Biphenyl oder Bipyridin, sowie Fluoren oder Spirobifluoren.

Im Allgemeinen werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentvl, Cvclopentvl, n-Hexvl, neo-Hexyl, Cyclohexyl, n-Heptvl, Cvcloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe OR¹ mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe SR¹ mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, besonders bevorzugt F oder CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropvren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin. Benzo-6.7-chinolin. Benzo-7.8-chinolin. Phenothiazin. Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen. Benzopvridazin, Pyrimidin. Benzpvrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter der Formulierung, dass zwei oder mehr Reste miteinander einen aliphatischen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

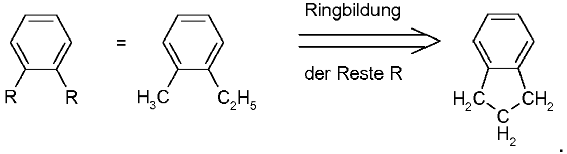

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

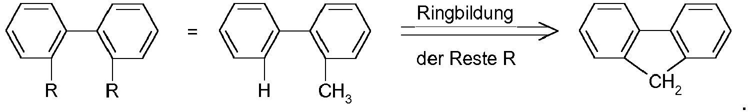

Je nach Position der Bindung der Gruppe der Formel (2) ergeben sich unterschiedliche Isomere, die nachfolgend durch die Formeln (3) bis (8) dargestellt sind, 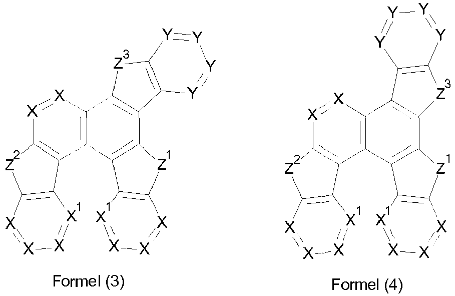 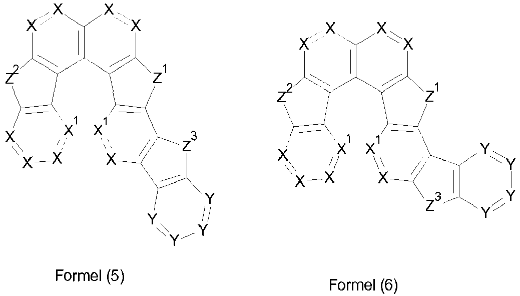 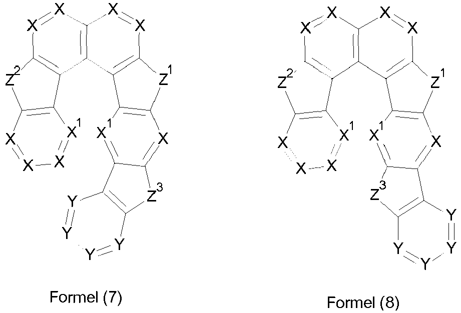 wobei die verwendeten Symbole die oben genannten Bedeutungen aufweisen.

In einer bevorzugten Ausführungsform der Erfindung enthält die Verbindung der Formel (1) genau eine Gruppe der Formel (2). Gemäß der Erfindung steht in Formel (1) und den Formeln (3) bis (8) das Symbol X' für CH.

In einer bevorzugten Ausführungsform der Erfindung steht in der Gruppe der Formel (2) maximal ein Symbol Y für N, und die anderen Symbole Y stehen gleich oder verschieden für CR. Besonders bevorzugt stehen alle Symbole Y in Formel (2) für CR. Die Gruppe der Formel (2) ist dann eine Gruppe der folgenden Formel (2a), 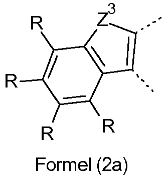 wobei die verwendeten Symbole die oben genannten Bedeutungen aufweisen.

In nochmals einer weiteren bevorzugten Ausführungsform der Erfindung stehen maximal zwei Symbole X für N, besonders bevorzugt maximal ein Symbol X. Ganz besonders bevorzugt stehen die Symbole X, die nicht für eine Gruppe der Formel (2) stehen, gleich oder verschieden bei iedem Auftreten für CR. In einer besonders bevorzugten Ausführungsform der Erfindung stehen die Symbole Y gleich oder verschieden bei jedem Auftreten für CR, so dass es sich bevorzugt um eine Gruppe der Formel (2a) handelt, und die Symbole X, die nicht für eine Gruppe der Formel (2) stehen, stehen gleich oder verschieden bei jedem Auftreten für CR, und die Symbole X' stehen für CH.

Bevorzugt sind somit die Strukturen der folgenden Formeln (3a) bis (8a), 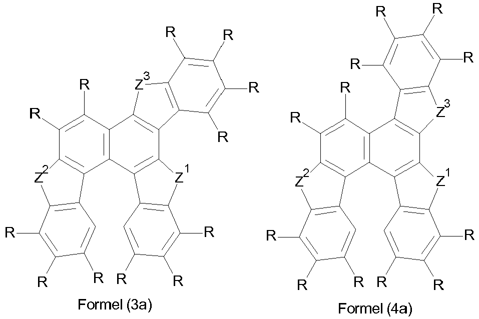 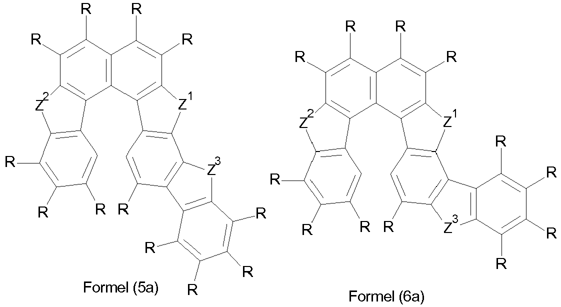 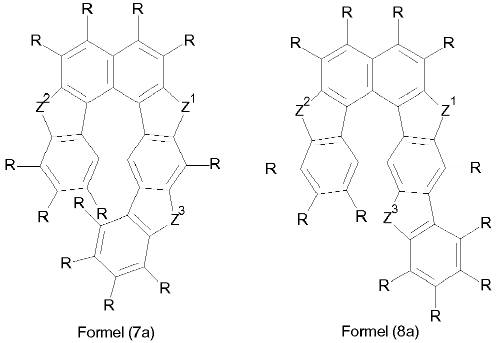 wobei die verwendeten Symbole die oben genannten Bedeutungen aufweisen.

Besonders bevorzugt sind die Strukturen der folgenden Formeln (3b) bis (8b), 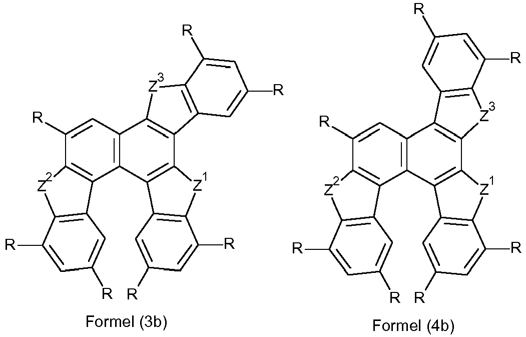 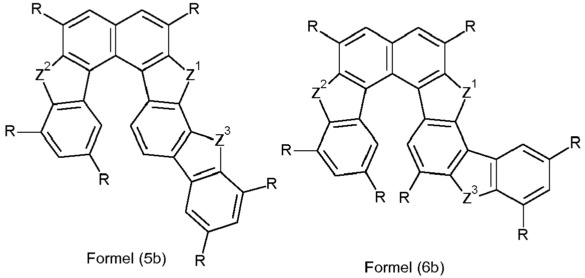 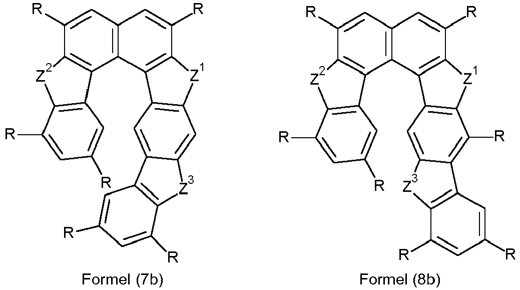 wobei die verwendeten Symbole die oben genannten Bedeutungen aufweisen.

In einer bevorzugten Ausführungsform der Erfindung stehen insgesamt maximal drei Reste R, besonders bevorzugt maximal zwei Reste R und ganz besonders bevorzugt maximal ein Rest R in der Verbindung der Formel (1) bzw. den oben und nachfolgend aufgeführten bevorzugten Strukturen für eine Gruppe ungleich Wasserstoff.

Ganz besonders bevorzugt sind die Strukturen der folgenden Formeln (3c) bis (8c), 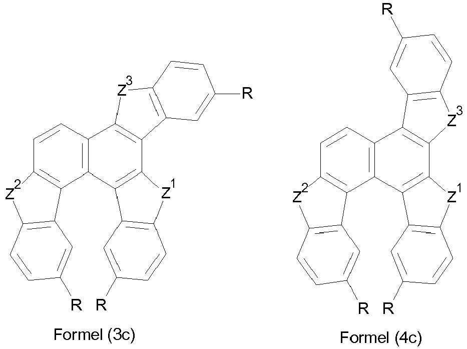 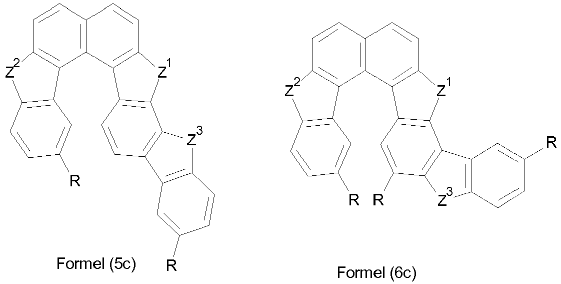 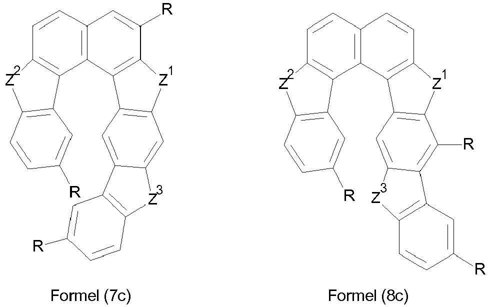 wobei die verwendeten Symbole die oben genannten Bedeutungen aufweisen.

Besonders bevorzugt sind die Strukturen der Formeln (3b) und (4b) bzw. (3c) und (4c).

Z¹, Z² und Z³ sind unabhängig voneinander gewählt aus der Gruppe bestehend aus O, S, N-Ar und CR₂, bevorzugt aus der Gruppe bestehend aus O, S und N-Ar. Dabei sind geeignete Kombinationen Z¹, Z² und Z³ die in der folgenden Tabelle aufgeführten Kombinationen, wobei jede dieser Kombinationen für jede der Strukturen der Formeln (1), (3), (4), (5), (6), (7), (8), (3a), (4a), (5a), (6a), (7a), (8a), (3b), (4b), (5b), (6b), (7b), (8b), (3c), (4c), (5c), (6c), (7c) und (8c) eingesetzt werden kann:

| **Z¹** | **Z²** | **Z³** |
|---|---|---|
| O | O | O |
| O | O | S |
| O | O | N-Ar |
| O | O | CR₂ |
| O | S | O |
| O | S | S |
| O | S | N-Ar |
| O | S | CR₂ |
| O | N-Ar | O |
| O | N-Ar | S |
| O | N-Ar | N-Ar |
| O | N-Ar | CR₂ |
| O | CR₂ | O |
| O | CR₂ | S |
| O | CR₂ | N-Ar |
| O | CR₂ | CR₂ |
| S | O | O |
| S | O | S |
| S | O | N-Ar |
| S | O | CR₂ |
| S | S | O |
| S | S | S |
| S | S | N-Ar |
| S | S | CR₂ |
| S | N-Ar | O |
| S | N-Ar | S |
| S | N-Ar | N-Ar |
| S | N-Ar | CR₂ |
| S | CR₂ | O |
| S | CR₂ | S |
| S | CR₂ | N-Ar |
| S | CR₂ | CR₂ |
| N-Ar | O | O |
| N-Ar | O | S |
| N-Ar | O | N-Ar |
| N-Ar | O | CR₂ |
| N-Ar | S | O |
| N-Ar | S | S |
| N-Ar | S | N-Ar |
| N-Ar | S | CR₂ |
| N-Ar | N-Ar | O |
| N-Ar | N-Ar | S |
| N-Ar | N-Ar | N-Ar |
| N-Ar | N-Ar | CR₂ |
| N-Ar | CR₂ | O |
| N-Ar | CR₂ | S |
| N-Ar | CR₂ | N-Ar |
| N-Ar | CR₂ | CR₂ |
| CR₂ | O | O |
| CR₂ | O | S |
| CR₂ | O | N-Ar |
| CR₂ | O | CR₂ |
| CR₂ | S | O |
| CR₂ | S | S |
| CR₂ | S | N-Ar |
| CR₂ | S | CR₂ |
| CR₂ | N-Ar | O |
| CR₂ | N-Ar | S |
| CR₂ | N-Ar | N-Ar |
| CR₂ | N-Ar | CR₂ |
| CR₂ | CR₂ | O |
| CR₂ | CR₂ | S |
| CR₂ | CR₂ | N-Ar |
| CR₂ | CR₂ | CR₂ |

In einer Ausführungsform der Erfindung sind Z¹ und Z² gleich gewählt. Dabei ist es bevorzugt, wenn Z¹ und Z² = O oder Z¹ und Z² = S sind und gleichzeitig Z³ = NAr ist. In einer weiteren Ausführungsform der Erfindung stehen zwei der Symbole Z¹, Z² und Z³ für NAr und das dritte der Symbole steht für S.

Wenn die erfindungsgemäße Verbindung als Matrixmaterial für phosphoreszierende Emitter eingesetzt wird, ist es bevorzugt, wenn mindestens eines der Symbole Z¹, Z² und/oder Z³ für N-Ar steht. In diesem Fall ist es besonders bevorzugt, wenn Z³ für N-Ar steht. Besonders bevorzugt sind daher die Verbindungen der folgenden Formeln (3d) bis (8d), 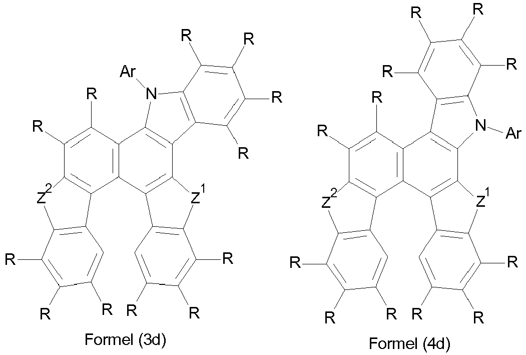 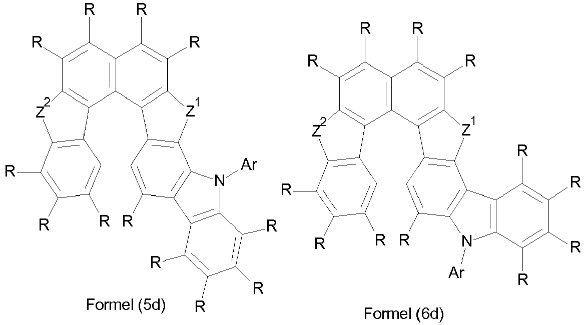 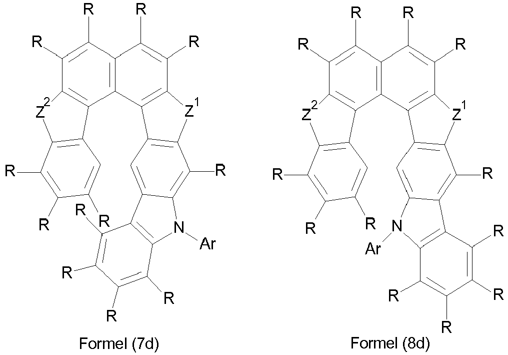 wobei die verwendeten Symbole die oben genannten Bedeutungen aufweisen.

Gand besonders bevorzugt sind die Strukturen der folgenden Formeln (3e) bis (8e), 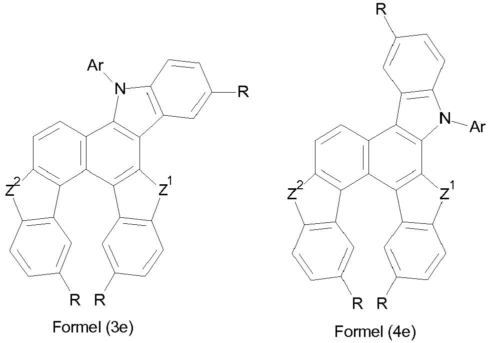 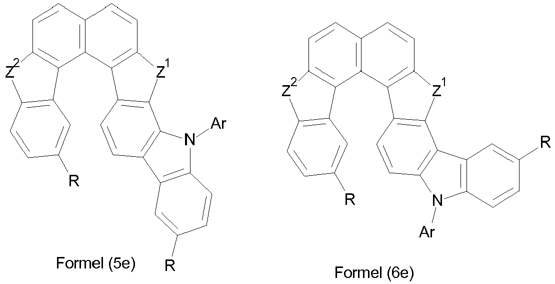 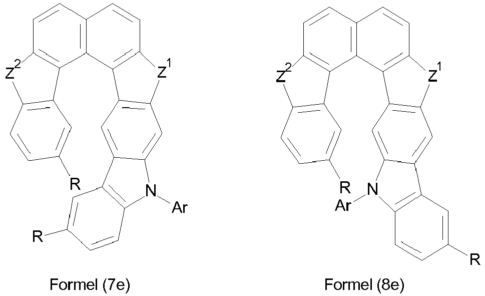 wobei die verwendeten Symbole die oben genannten Bedeutungen aufweisen.

Im Folgenden werden bevorzugte Substituenten Ar, R, Ar', R¹ und R² beschrieben. In einer besonders bevorzugten Ausführungsform der Erfindung treten die nachfolgend genannten Bevorzugungen für Ar, R, Ar', R¹ und R² gleichzeitig auf und gelten für die Strukturen der Formel (1) sowie für alle oben aufgeführten bevorzugten Ausführungsformen.

In einer bevorzugten Ausführungsform der Erfindung ist Ar bei jedem Auftreten gleich oder verschieden ein aromatisches oder heteroaromatisches Ringsystem mit 6 bis 30 aromatischen Ringatomen, das durch einen oder mehrere Reste R substituiert sein kann, wobei es sich bei den Resten R bevorzugt um nicht-aromatische Reste handelt. Besonders bevorzugt ist Ar bei jedem Auftreten gleich oder verschieden ein aromatisches oder heteroaromatisches Ringsystem mit 6 bis 24 aromatischen Ringatomen, insbesondere mit 6 bis 13 aromatischen Ringatomen, das durch einen oder mehrere, bevorzugt nicht-aromatische, Reste R substituiert sein kann. Wenn Ar für eine Heteroarylgruppe, insbesondere für Triazin, Pyrimidin, Chinazolin oder Carbazol steht, können auch aromatische oder heteroaromatische Substituenten R an dieser Heteroarylgruppe bevorzugt sein. Weiterhin kann es bevorzugt sein, wenn Ar mit einer Gruppe N(Ar')₂ substituiert ist, so dass der Substituent Ar insgesamt eine Triarylamin- bzw. Triheteroarylamin-Gruppe darstellt.

Geeignete aromatische bzw. heteroaromatische Ringsystem Ar sind bei jedem Auftreten gleich oder verschieden ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R, bevorzugt nicht-aromatischen Resten R, substituiert sein können. Wenn Ar für eine Heteroarylgruppe, insbesondere für Triazin, Pyrimidin oder Chinazolin steht, können auch aromatische oder heteroaromatische Reste R an dieser Heteroarylgruppe bevorzugt sein.

Dabei ist Ar bevorzugt bei jedem Auftreten gleich oder verschieden gewählt aus den Gruppen der folgenden Formeln Ar-1 bis Ar-81, 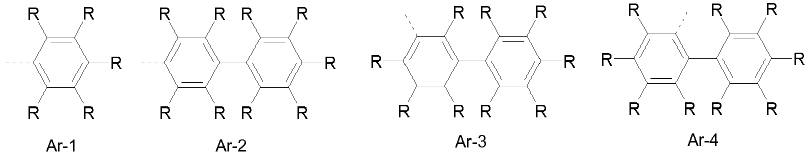 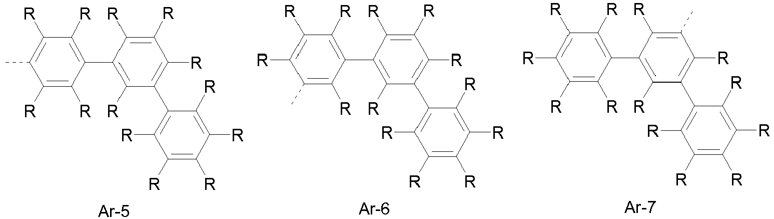 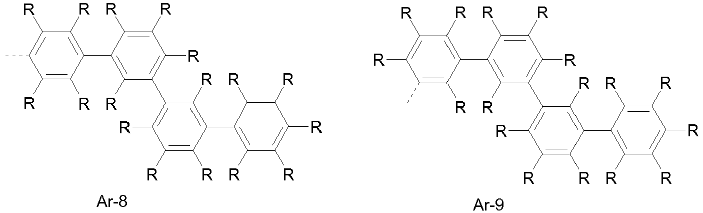 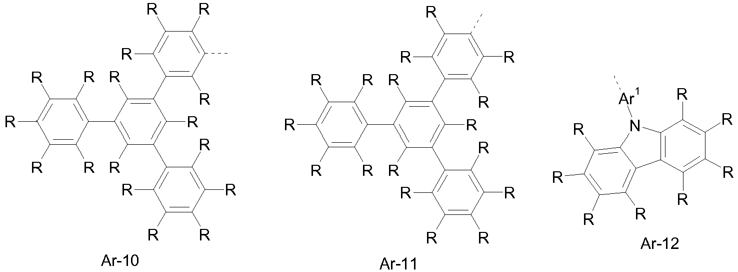 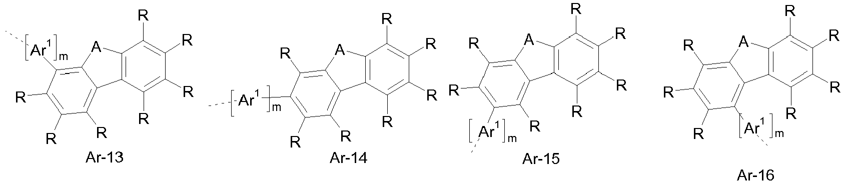 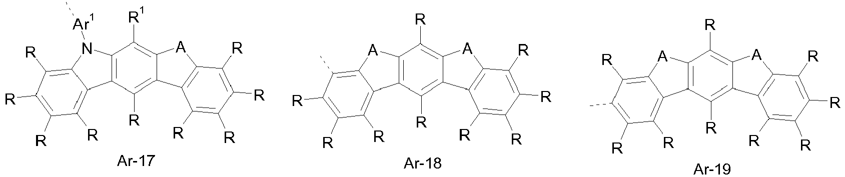 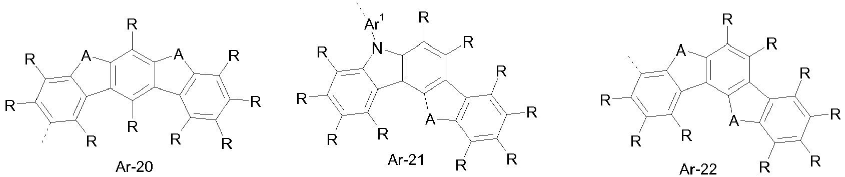 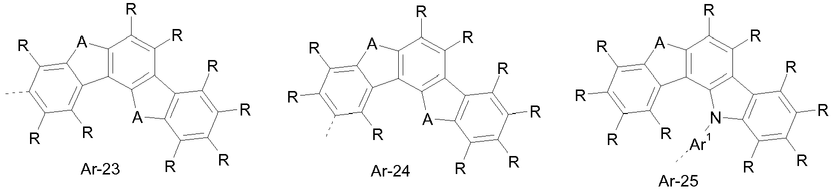 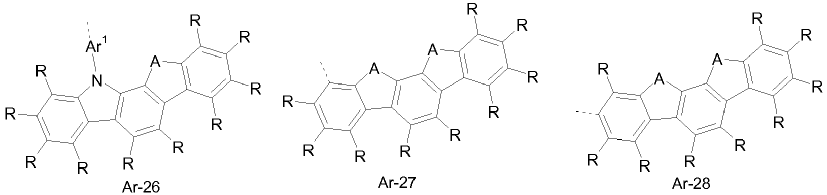 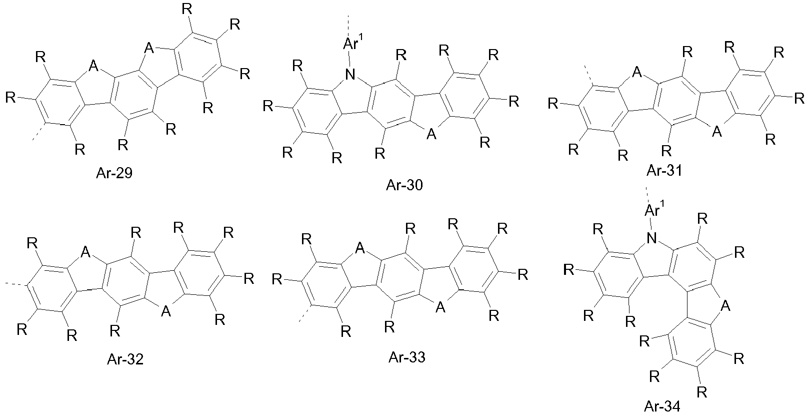 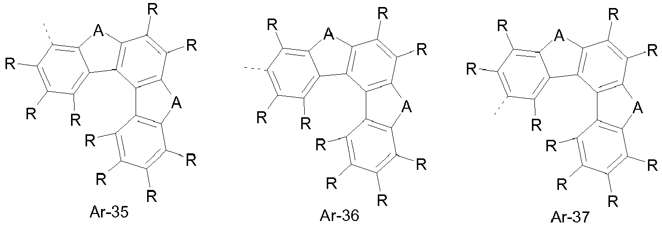 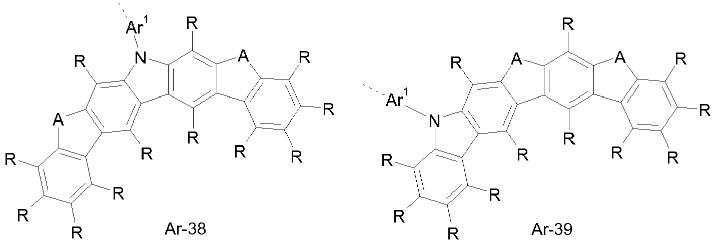 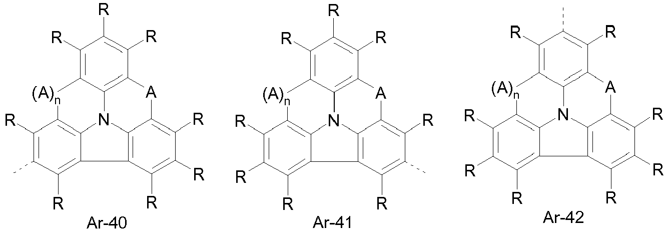 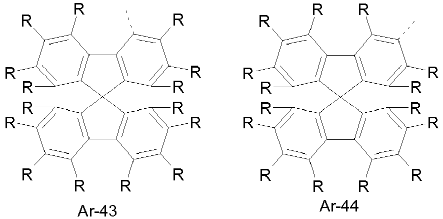 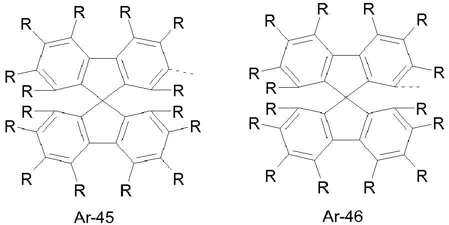 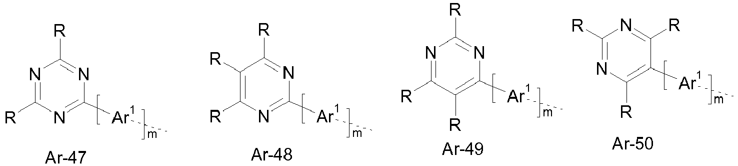 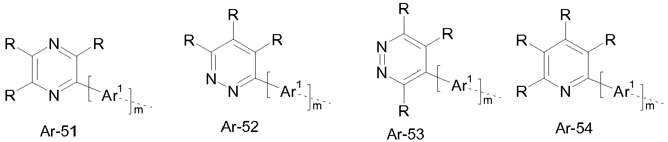 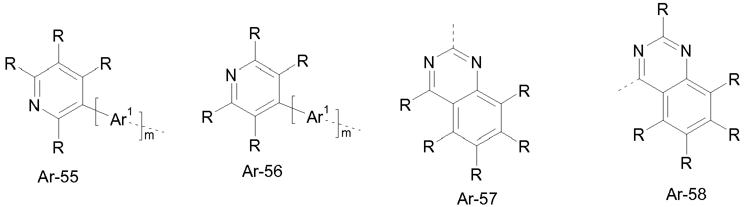 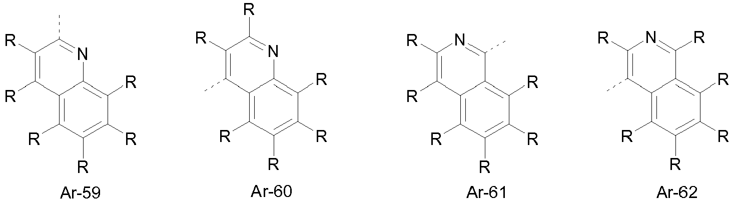 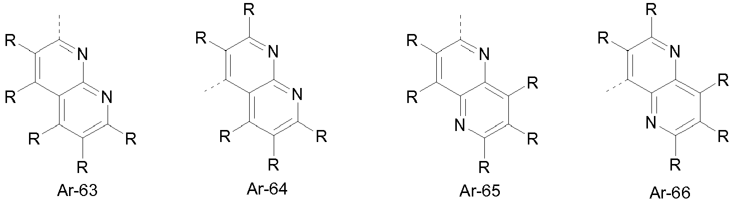 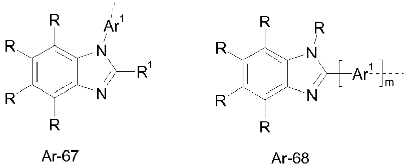 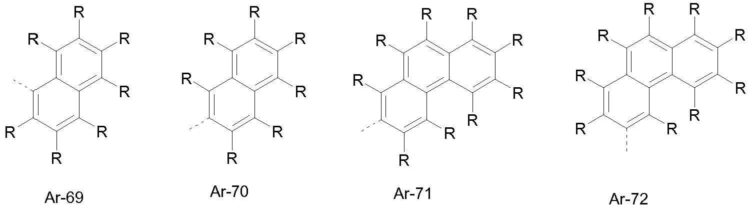 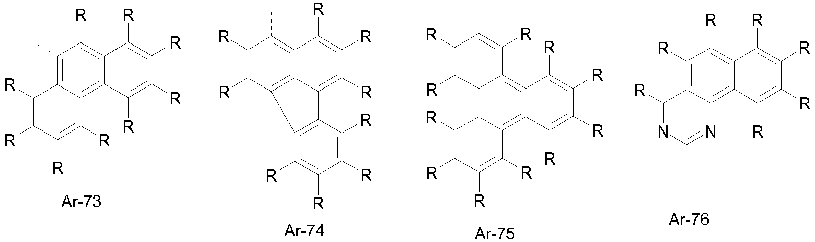 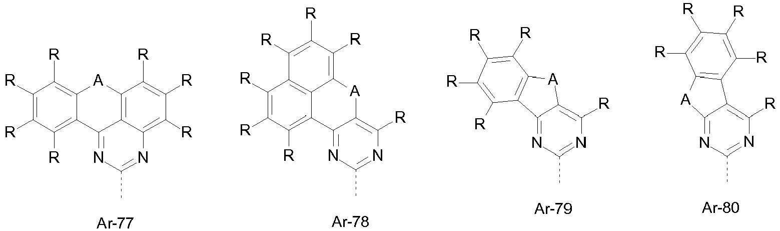 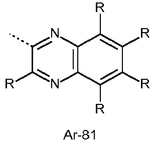 wobei R die oben genannten Bedeutungen aufweist, die gestrichelte Bindung die Bindung an das Stickstoffatom darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;
- A: ist bei jedem Auftreten gleich oder verschieden CR₂, NR, O oder S;
- n: ist 0 oder 1, wobei n = 0 bedeutet, dass an dieser Position keine Gruppe A gebunden ist und an die entsprechenden Kohlenstoffatome statt dessen Reste R gebunden sind;
- m: ist 0 oder 1, wobei m = 0 bedeutet, dass die Gruppe Ar¹ nicht vorhanden ist und dass die entsprechende aromatische bzw. heteroaromatische Gruppe direkt an das Stickstoffatom gebunden ist.

Gemäß der Erfindung ist R bei jedem Auftreten gleich oder verschieden ausgewählt aus der Gruppe bestehend aus H, D, F, N(Ar')₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann, wobei es sich bei den Resten R¹ dann bevorzugt um nicht-aromatische Reste handelt; dabei können zwei Reste R auch miteinander ein aliphatisches Ringsystem bilden. Besonders bevorzugt ist R bei jedem Auftreten gleich oder verschieden ausgewählt aus der Gruppe bestehend aus H, N(Ar')₂, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Ganz besonders bevorzugt ist R bei jedem Auftreten gleich oder verschieden ausgewählt aus der Gruppe bestehend aus H oder einem aromatischen oder heteroaromatischen Ringsystem mit 6 bis 24 aromatischen Ringatomen, bevorzugt mit 6 bis 13 aromatischen Ringatomen, das jeweils durch einen oder mehrere Reste R¹, bevorzugt nicht-aromatische Reste R¹, substituiert sein kann. Weiterhin kann es bevorzugt sein, wenn R für eine Triaryl- bzw. -heteroarylamingruppe steht, welche durch einen oder mehrere Reste R¹ substituiert sein kann. Diese Gruppe ist eine Ausführungsform eines aromatischen oder heteroaromatischen Ringsystems, wobei dann mehrere Aryl- bzw. Heteroarylgruppen durch ein Stickstoffatom miteinander verknüpft sind. Wenn R für eine Triaryl- bzw. -heteroarylamingruppe steht, weist diese Gruppe bevorzugt 18 bis 30 aromatische Ringatome auf und kann durch einen oder mehrere Reste R¹, bevorzugt nicht-aromatische Reste R¹, substituiert sein.

Wenn Z¹, Z² und/oder Z³ für CR₂ stehen, sind die Substituenten R, die an dieses Kohlenstoffatom gebunden sind, bevorzugt gleich oder verschieden bei jedem Auftreten ausgewählt aus der Gruppe bestehend aus einer linearen Alkylgruppe mit 1 bis 10 C-Atomen oder einer verzweigten oder cyclischen Alkylgruppe mit 3 bis 10 C-Atomen oder einem aromatischen oder heteroaromatischen Ringsystem mit 5 bis 24 aromatischen Ringatomen, welches auch durch einen oder mehrere Reste R¹ substituiert sein kann. Ganz besonders bevorzugt steht R für eine Methylgruppe oder für eine Phenylgruppe. Dabei können die Reste R auch miteinander ein Ringsystem bilden, was zu einem Spirosystem führt.

Gemäß der Erfindung ist Ar' ein aromatisches oder heteroaromatisches Ringsystem mit 6 bis 30 aromatischen Ringatomen, das durch einen oder mehrere Reste R¹ substituiert sein kann. In einer besonders bevorzugten Ausführungsform der Erfindung ist Ar' ein aromatisches oder heteroaromatisches Ringsystem mit 6 bis 24 aromatischen Ringatomen, insbesondere mit 6 bis 13 aromatischen Ringatomen, das durch einen oder mehrere, bevorzugt nicht-aromatische, Reste R¹ substituiert sein kann.

Gemäß der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, bevorzugt mit 6 bis 13 aromatischen Ringatomen, das jeweils durch einen oder mehrere, bevorzugt nicht-aromatische Reste R² substituiert sein kann, bevorzugt aber unsubstituiert ist.

In einer weiteren bevorzugten Ausführungsform der Erfindung ist R² gleich oder verschieden bei jedem Auftreten H, F, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Geeignete aromatische bzw. heteroaromatische Ringsysteme R, wenn R für ein aromatisches oder heteroaromatisches Ringsystem steht, bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R¹ substituiert sein können. Wenn R bzw. Ar' für eine Heteroarylgruppe, insbesondere für Triazin, Pyrimidin, Chinazolin oder Carbazol steht, können auch aromatische oder heteroaromatische Reste R¹ an dieser Heteroarylgruppe bevorzugt sein.

Dabei sind die Gruppen R, wenn sie für ein aromatisches bzw. heteroaromatisches Ringsystem stehen, bzw. Ar' bevorzugt gewählt aus den Gruppen der folgenden Formeln R-1 bis R-81, 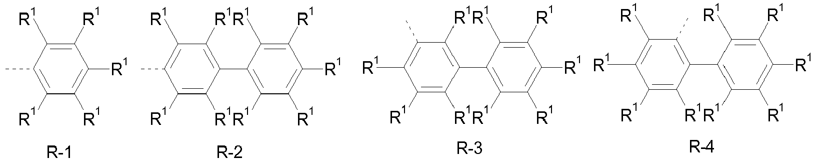 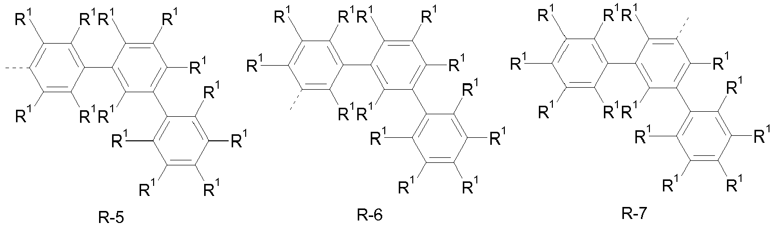 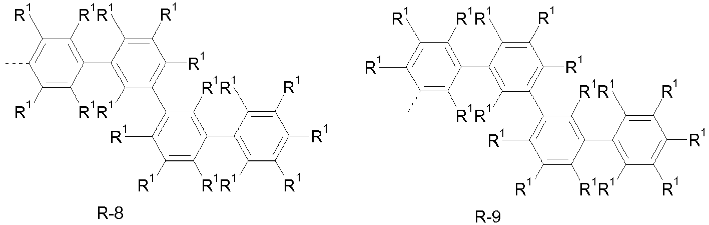 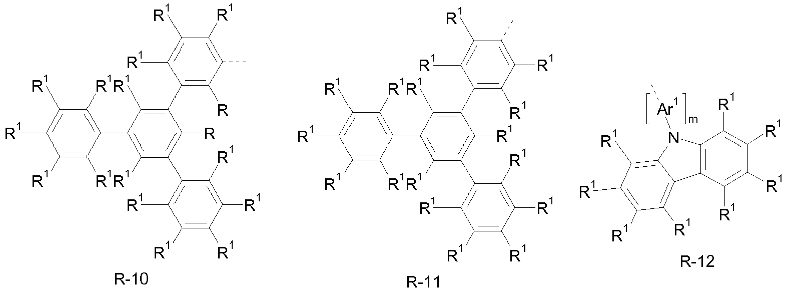 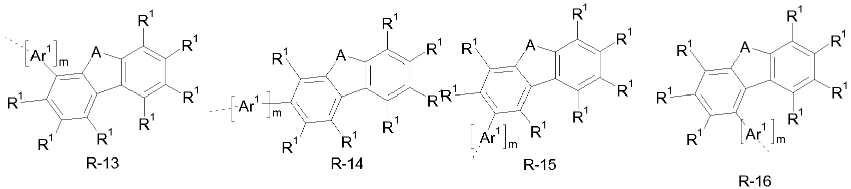 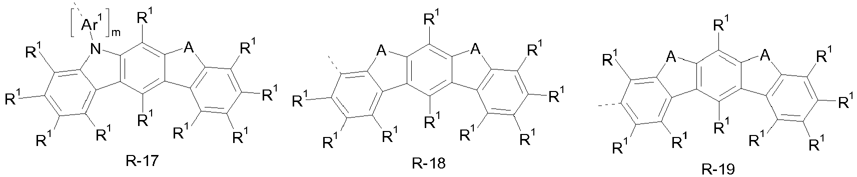 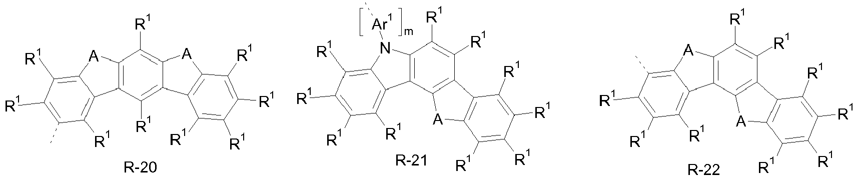 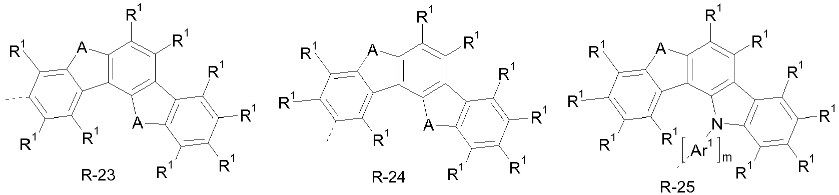 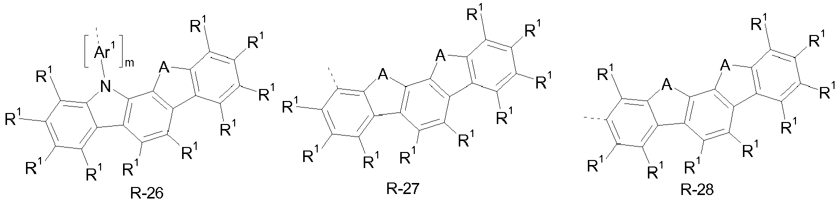 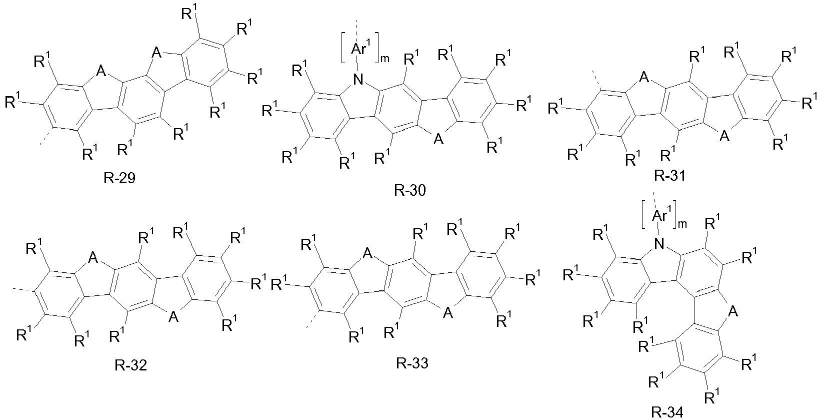 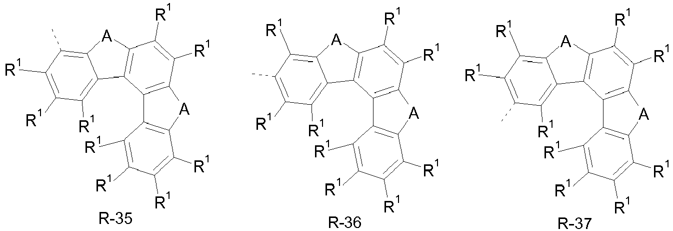 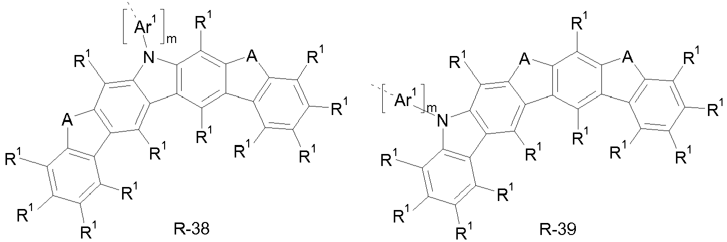 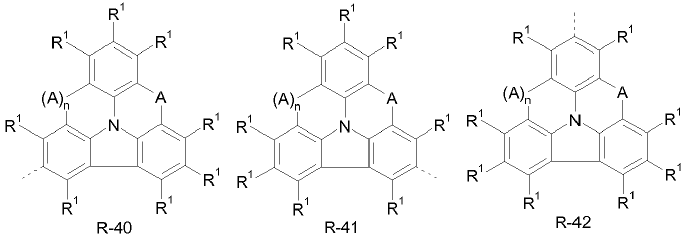 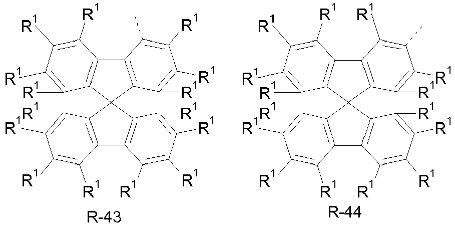 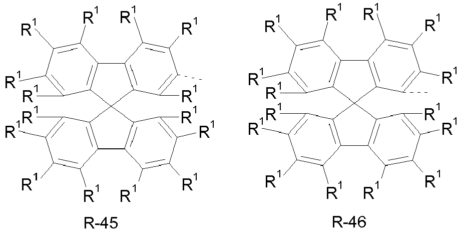 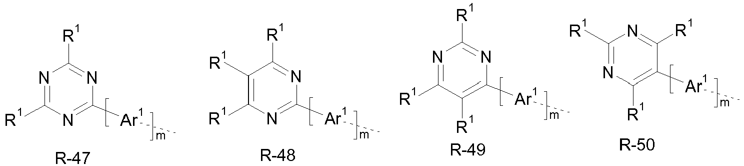 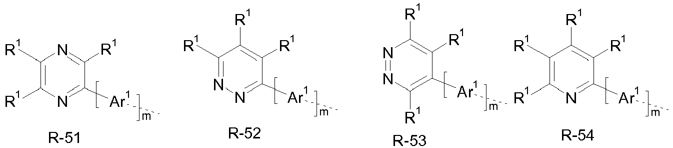 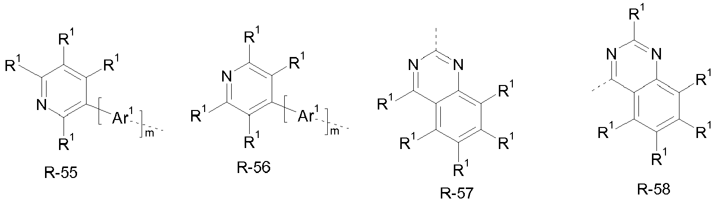 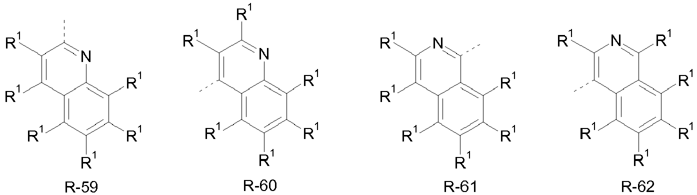 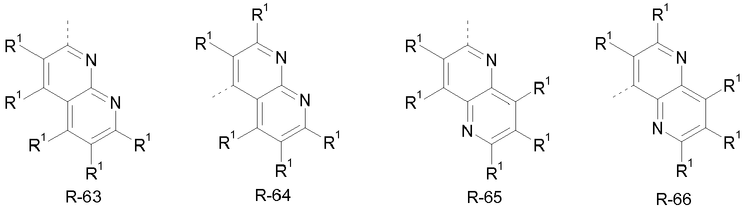 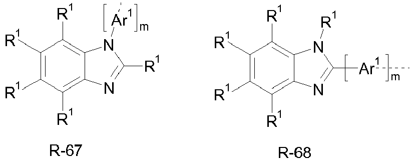 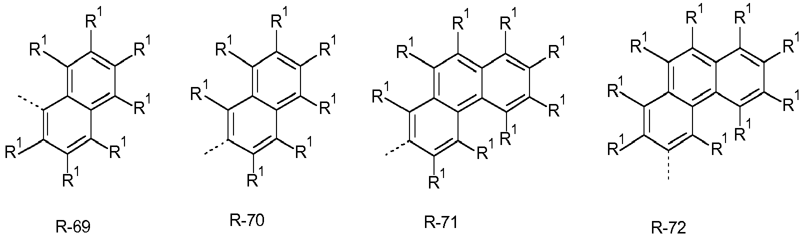 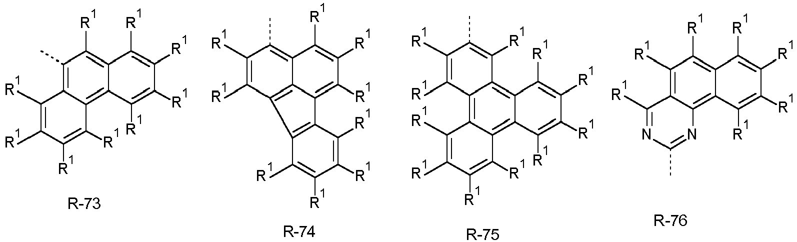 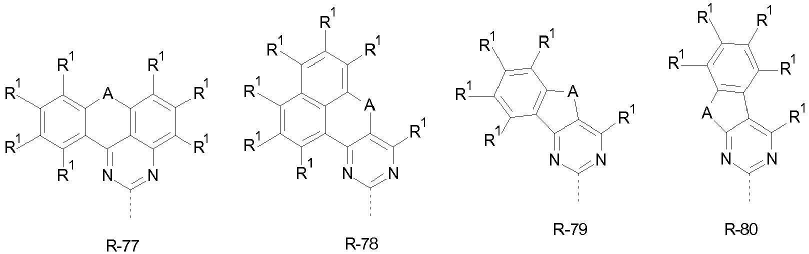 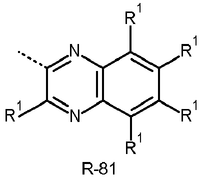 wobei R¹ die oben genannten Bedeutungen aufweist, die gestrichelte Bindung die Bindung an ein Kohlenstoffatom des Grundgerüsts in Formel (1) und (2) bzw. in den bevorzugten Ausführungsformen bzw. an das Stickstoffatom in der Gruppe N(Ar')₂ darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR', O oder S;
- n: ist 0 oder 1, wobei n = 0 bedeutet, dass an dieser Position keine Gruppe A gebunden ist und an den entsprechenden Kohlenstoffatomen statt dessen Reste R¹ gebunden sind;
- m: ist 0 oder 1, wobei m = 0 bedeutet, dass die Gruppe Ar¹ nicht vorhanden ist und dass die entsprechende aromatische bzw. heteroaromatische Gruppe direkt an ein Kohlenstoffatom des Grundgerüsts in Formel (1) bzw. in den bevorzugten Ausführungsformen bzw. an das Stickstoffatom in der Gruppe N(Ar')₂ gebunden ist; mit der Maßgabe, dass m = 1 ist für die Strukturen (R-12), (R-17), (R-21), (R-25), (R-26), (R-30), (R-34), (R-38) und (R-39), wenn es sich bei diesen Gruppen um Ausführungsformen von Ar' handelt.

Wenn die oben genannten Gruppen Ar-1 bis Ar-81 für Ar bzw. R-1 bis R-81 für R, R' bzw. Ar' mehrere Gruppen A aufweisen, so kommen hierfür alle Kombinationen aus der Definition von A in Frage. Bevorzugte Ausführungsformen sind dann solche, in denen eine Gruppe A für NR bzw. NR¹ und die andere Gruppe A für C(R)₂ bzw. C(R¹)₂ steht oder in denen beide Gruppen A für NR bzw. NR' stehen oder in denen beide Gruppen A für O stehen. In einer besonders bevorzugten Ausführungsform der Erfindung steht in Gruppen Ar, R bzw. Ar`, die mehrere Gruppen A aufweisen, mindestens eine Gruppe A für C(R)₂ bzw. C(R¹)₂ oder für NR bzw. NR¹.

Wenn in den Gruppen Ar-1 bis Ar-81 bzw. R-1 bis R-81 A für NR bzw. NR' steht, steht der Substituent R bzw. R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R¹ bzw. R² substituiert sein kann. In einer besonders bevorzugten Ausführungsform steht dieser Substituent R bzw. R¹ gleich oder verschieden bei jedem Auftreten für ein aromatisches oder heteroaromatisches Ringsystem mit 6 bis 24 aromatischen Ringatomen, bevorzugt mit 6 bis 12 aromatischen Ringatomen, welches keine kondensierten Arylgruppen oder Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R¹ bzw. R² substituiert sein kann. Besonders bevorzugt sind Phenyl, Biphenyl, Terphenyl und Quaterphenyl mit Verknüpfungsmustern, wie vorne für Ar-1 bis Ar-11 bzw. R-1 bis R-11 aufgeführt, wobei diese Strukturen durch einen oder mehrere Reste R¹ bzw. R² substituiert sein können, bevorzugt aber unsubstituiert sind.

Wenn in den Gruppen Ar-1 bis Ar-81 bzw. R-1 bis R-81 A für CR₂ bzw. C(R¹)₂ steht, stehen die Substituenten R bzw. R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R¹ bzw. R² substituiert sein kann. Ganz besonders bevorzugt steht R bzw. R¹ für eine Methylgruppe oder für eine Phenylgruppe. Dabei können die Reste R bzw. R¹ auch miteinander ein Ringsystem bilden, was zu einem Spirosystem führt.

Weitere geeignete Gruppen Ar, R bzw. Ar' sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Für Ar ergibt sich eine solche Gruppe, indem die Gruppe Ar mit einer Gruppe N(Ar')₂ substituiert ist. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe, ausgewählt aus C(R¹)₂, NR', O oder S, verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, insbesondere mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe. Dies gilt insbesondere, wenn Ar⁴ mit Ar² durch einen Einfachbindung verbunden ist.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar² und Ar³ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben die Alkylgruppen in erfindungsgemäßen Verbindungen, die durch Vakuumverdampfung verarbeitet werden, bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste Ar, R, Ar', R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

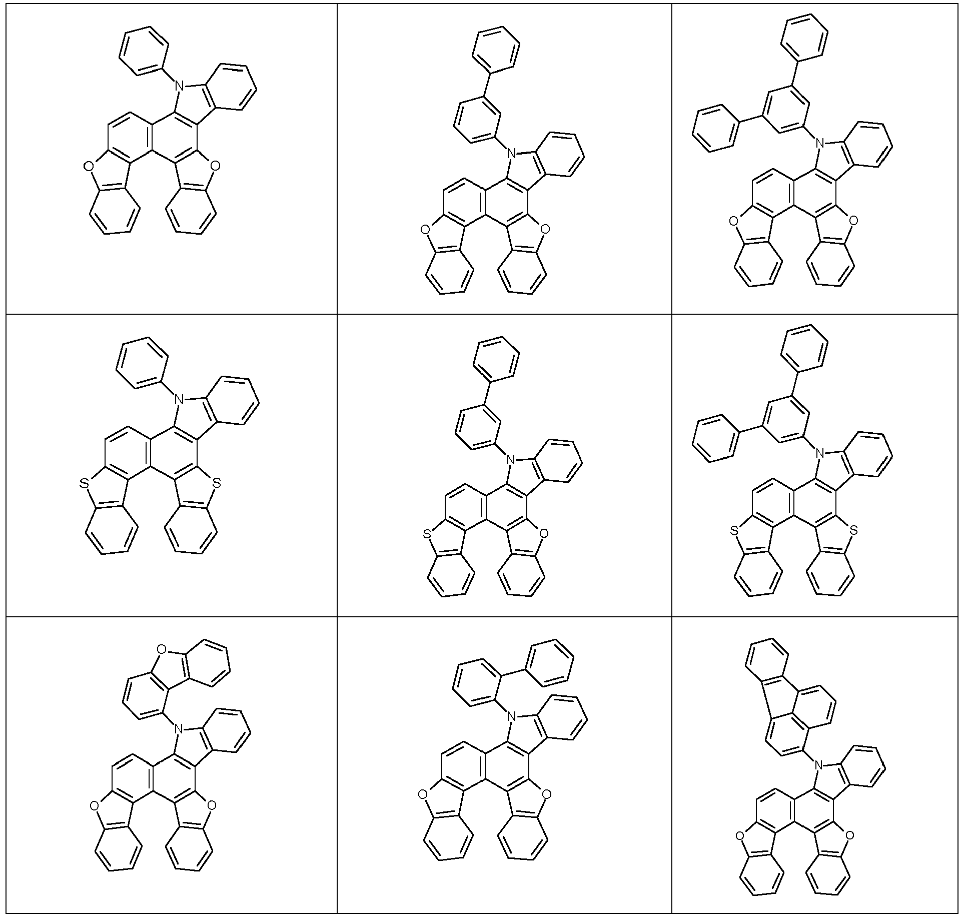

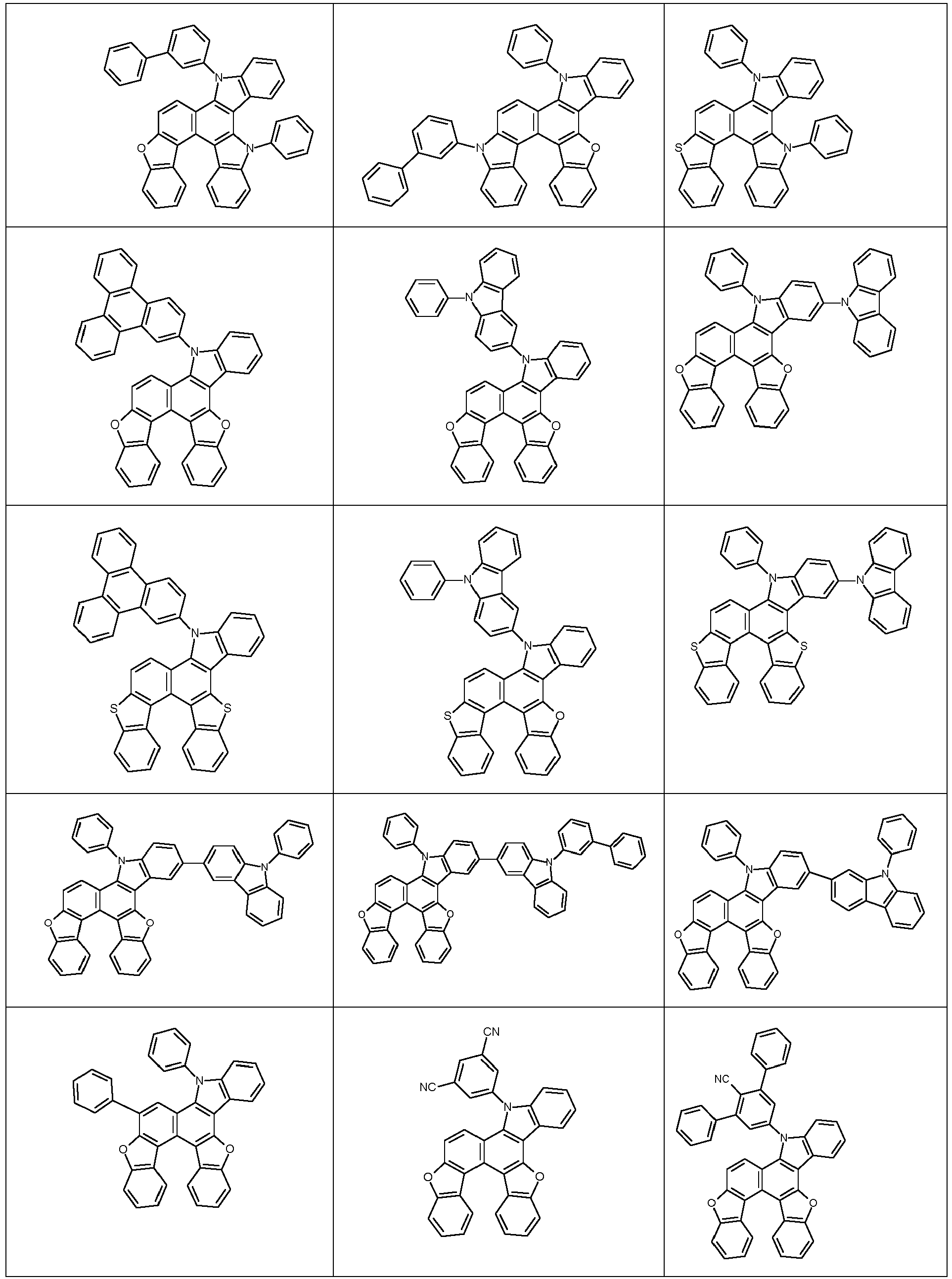

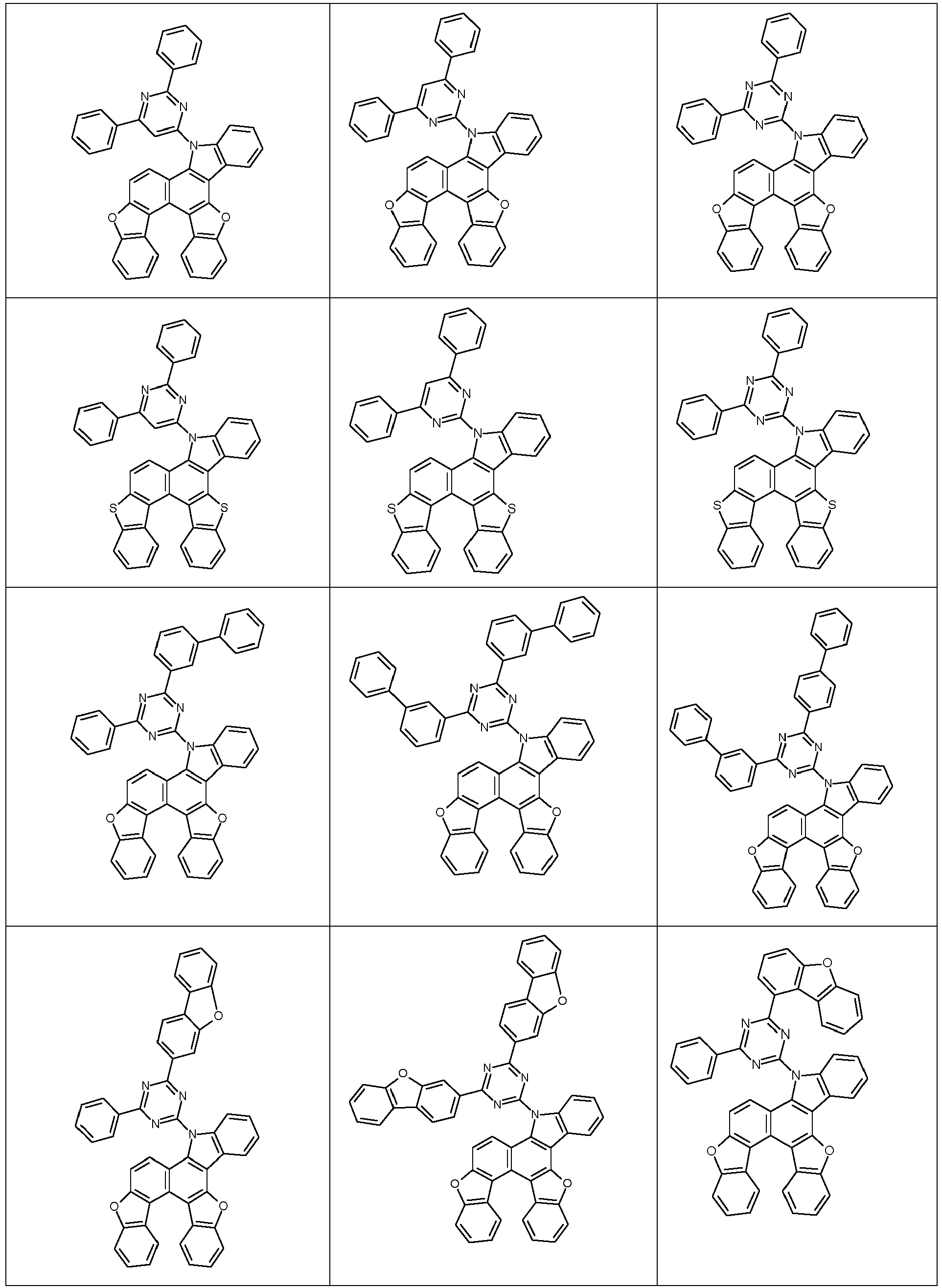

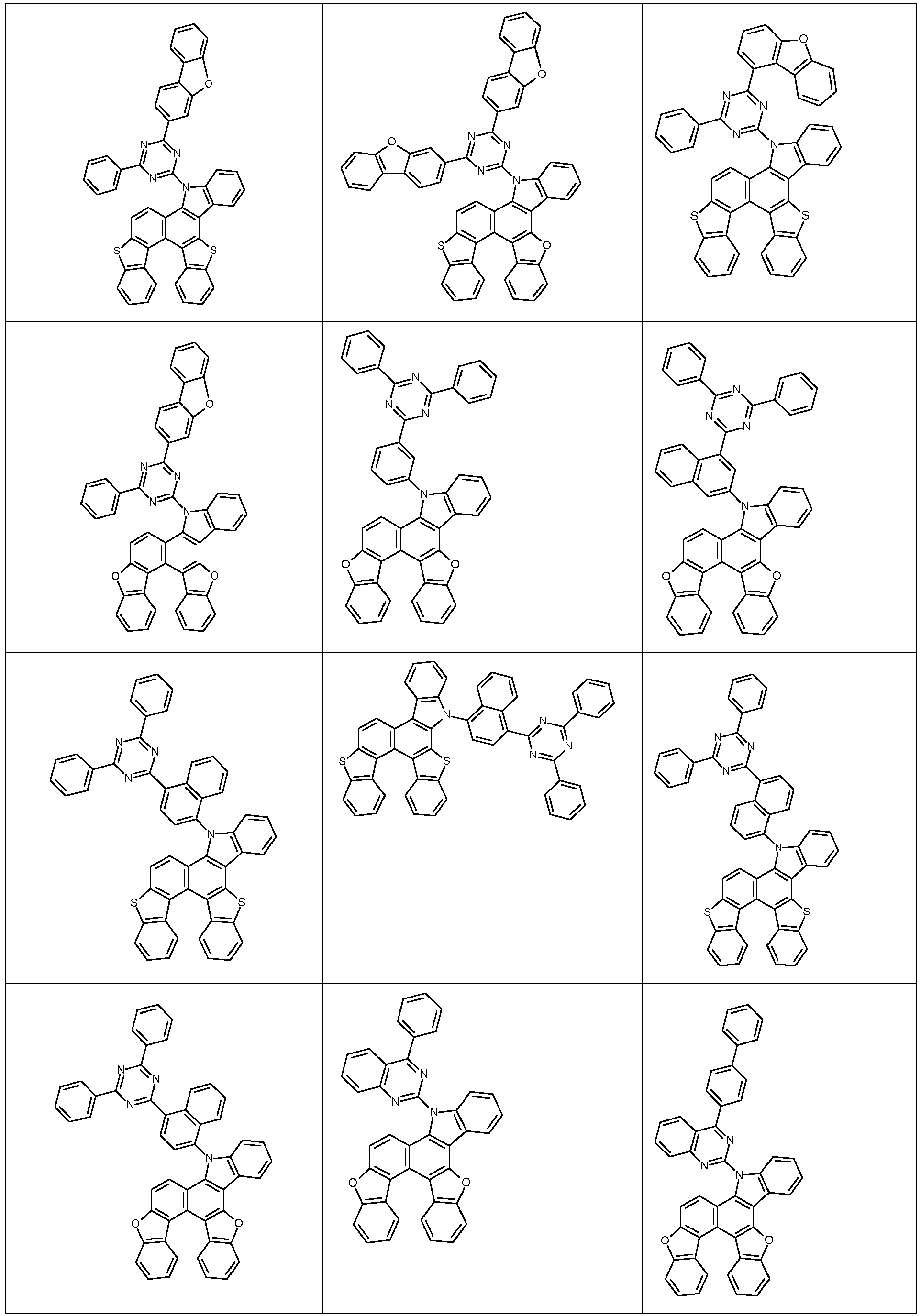

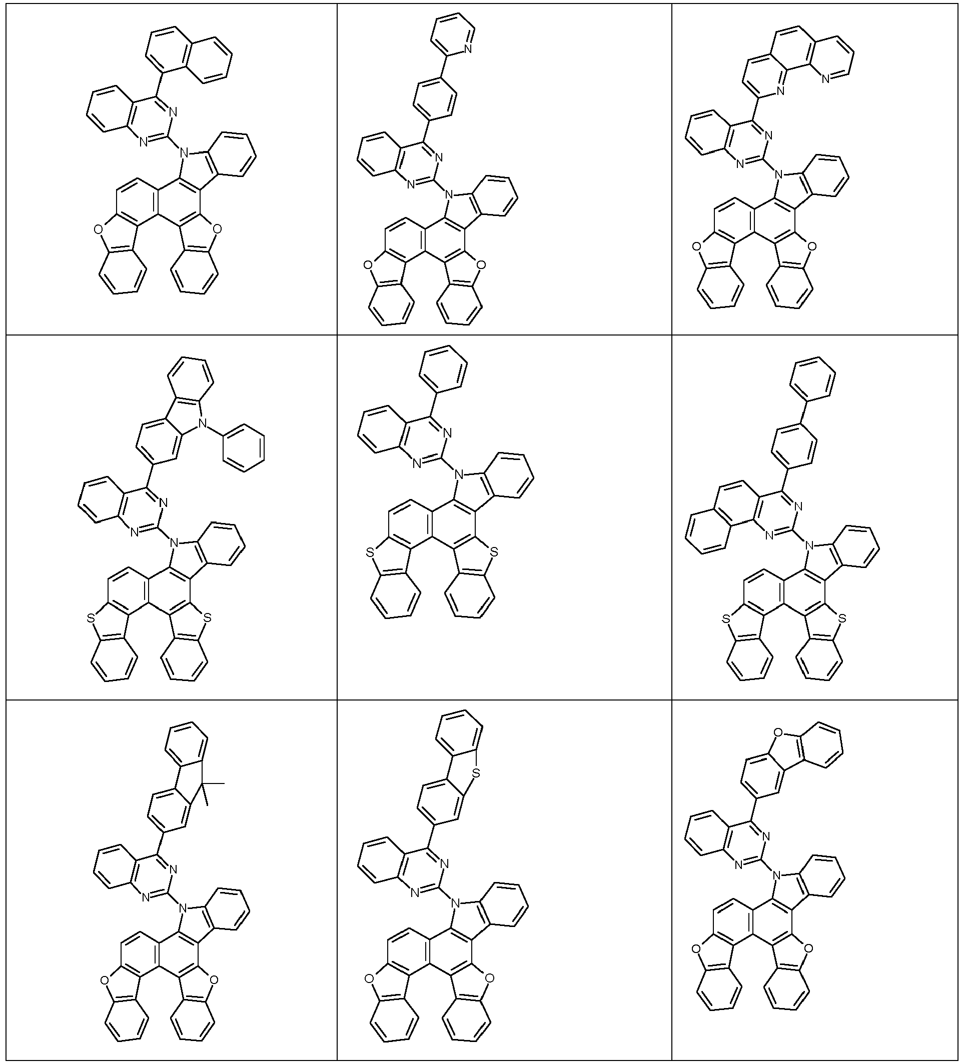

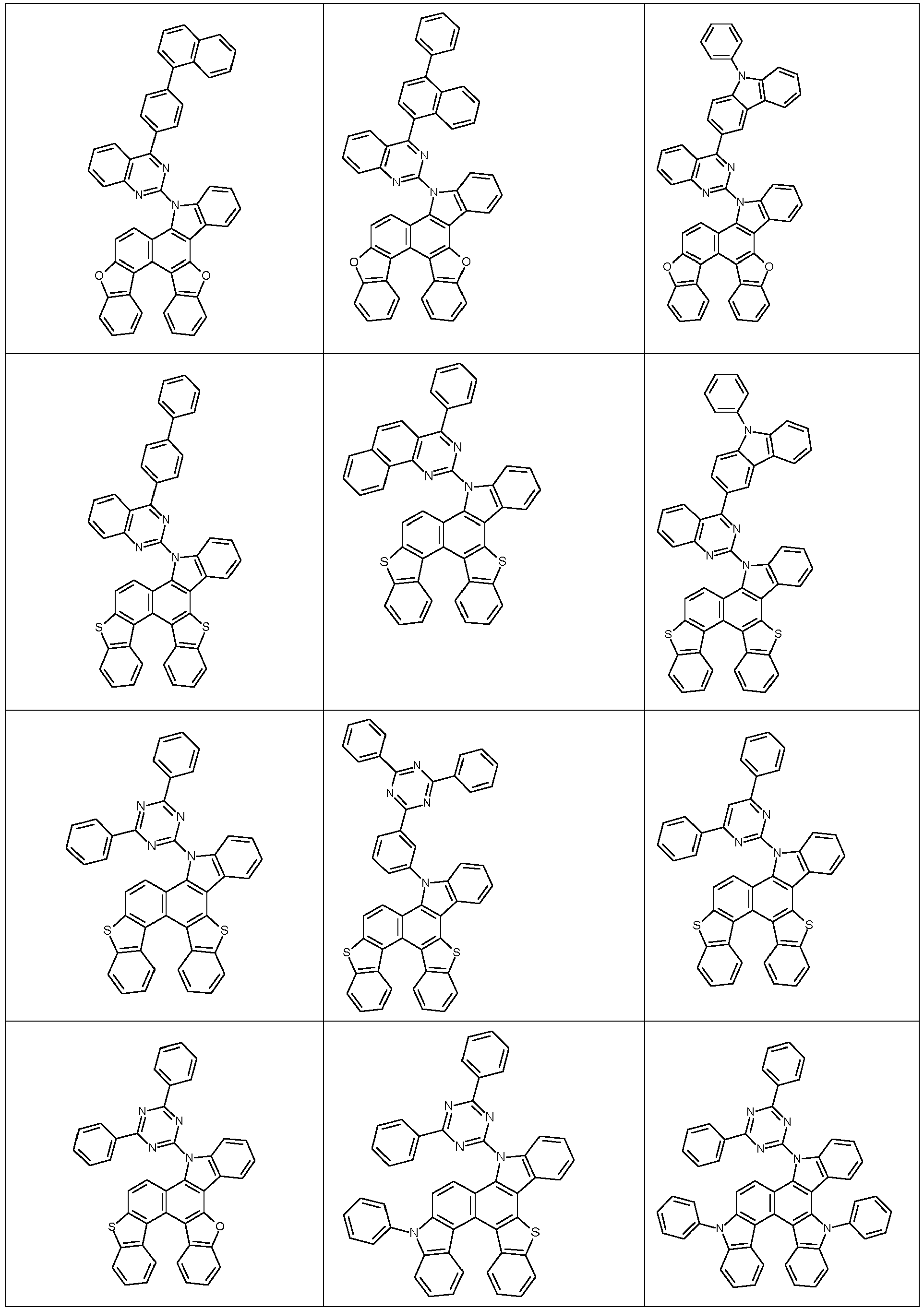

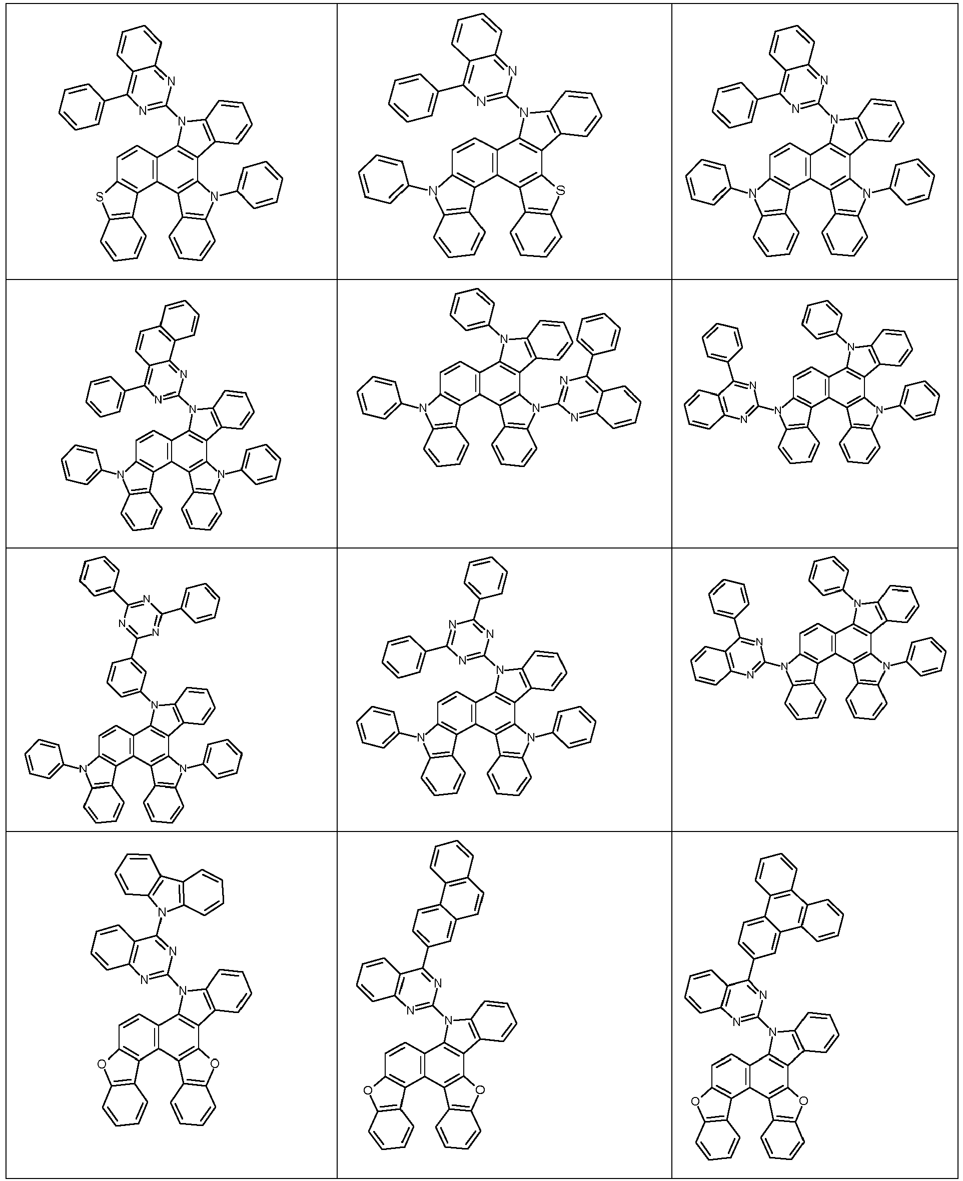

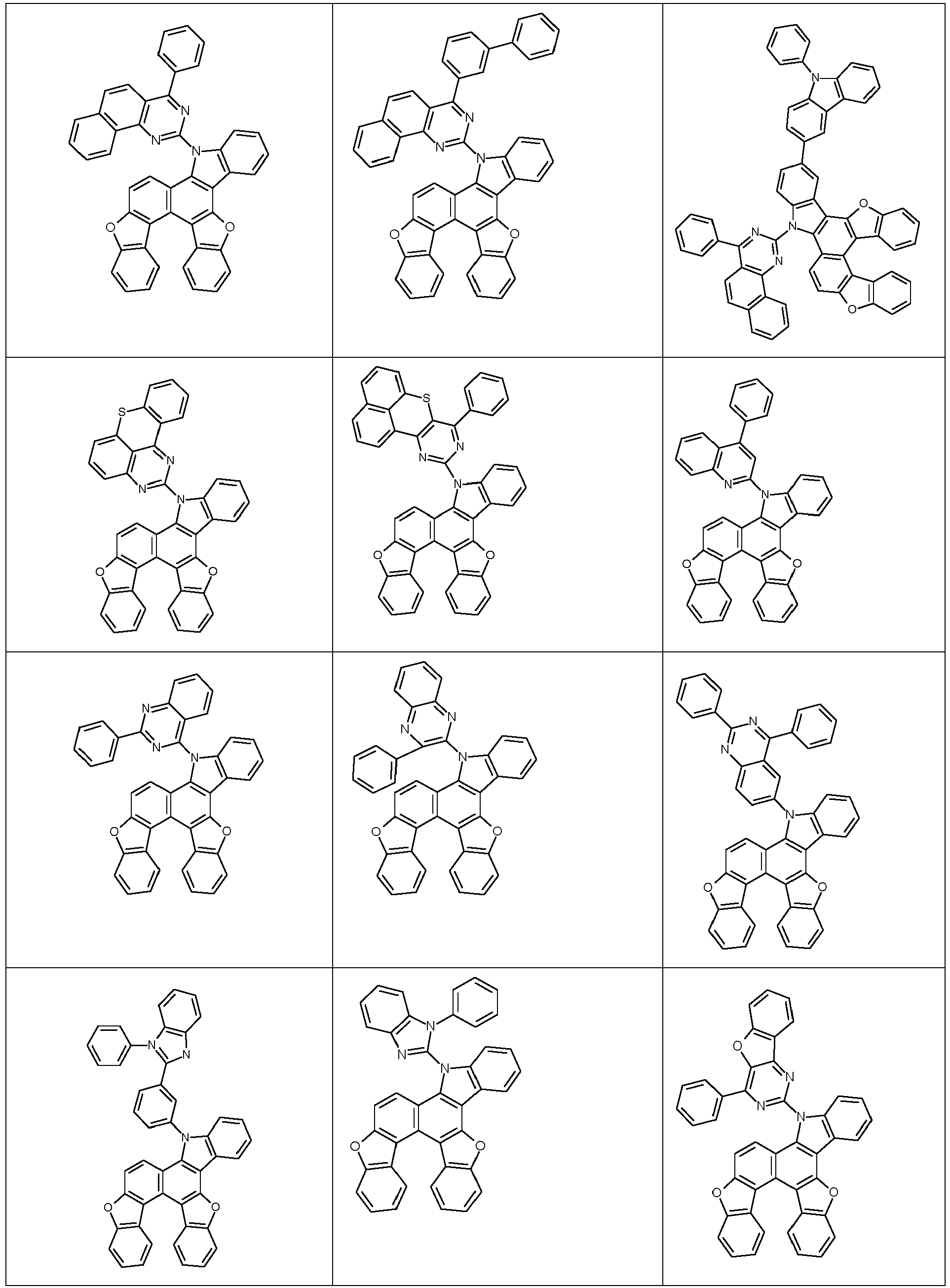

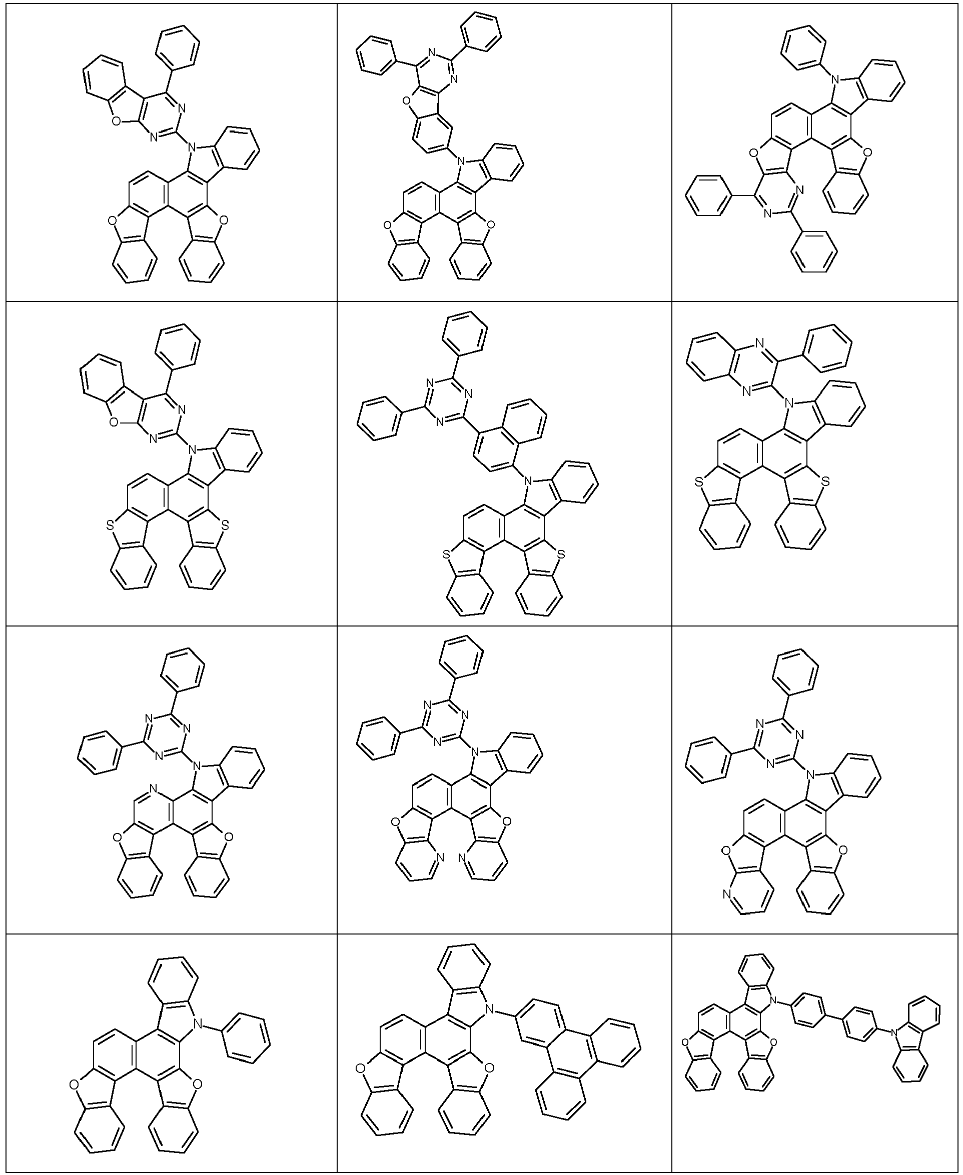

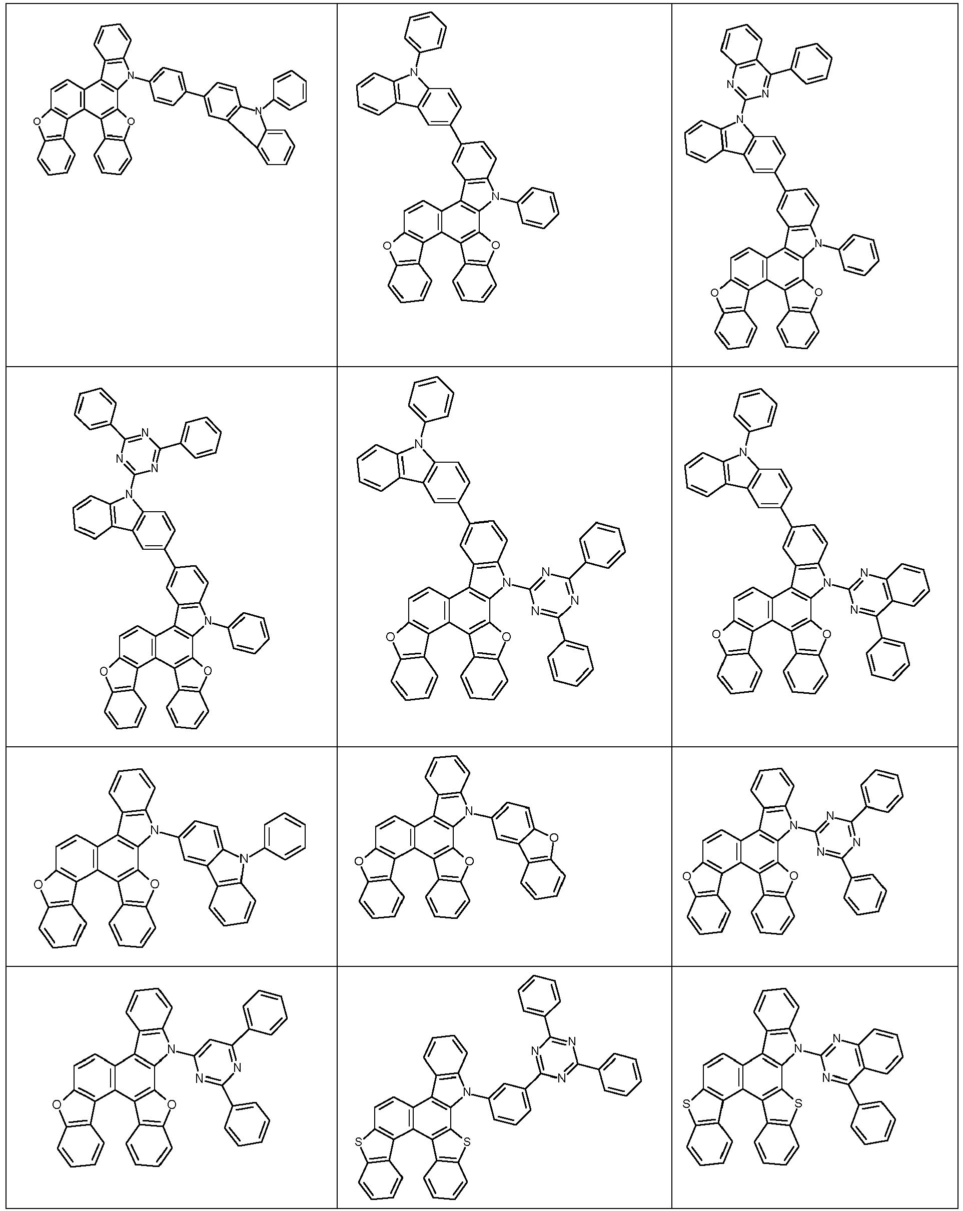

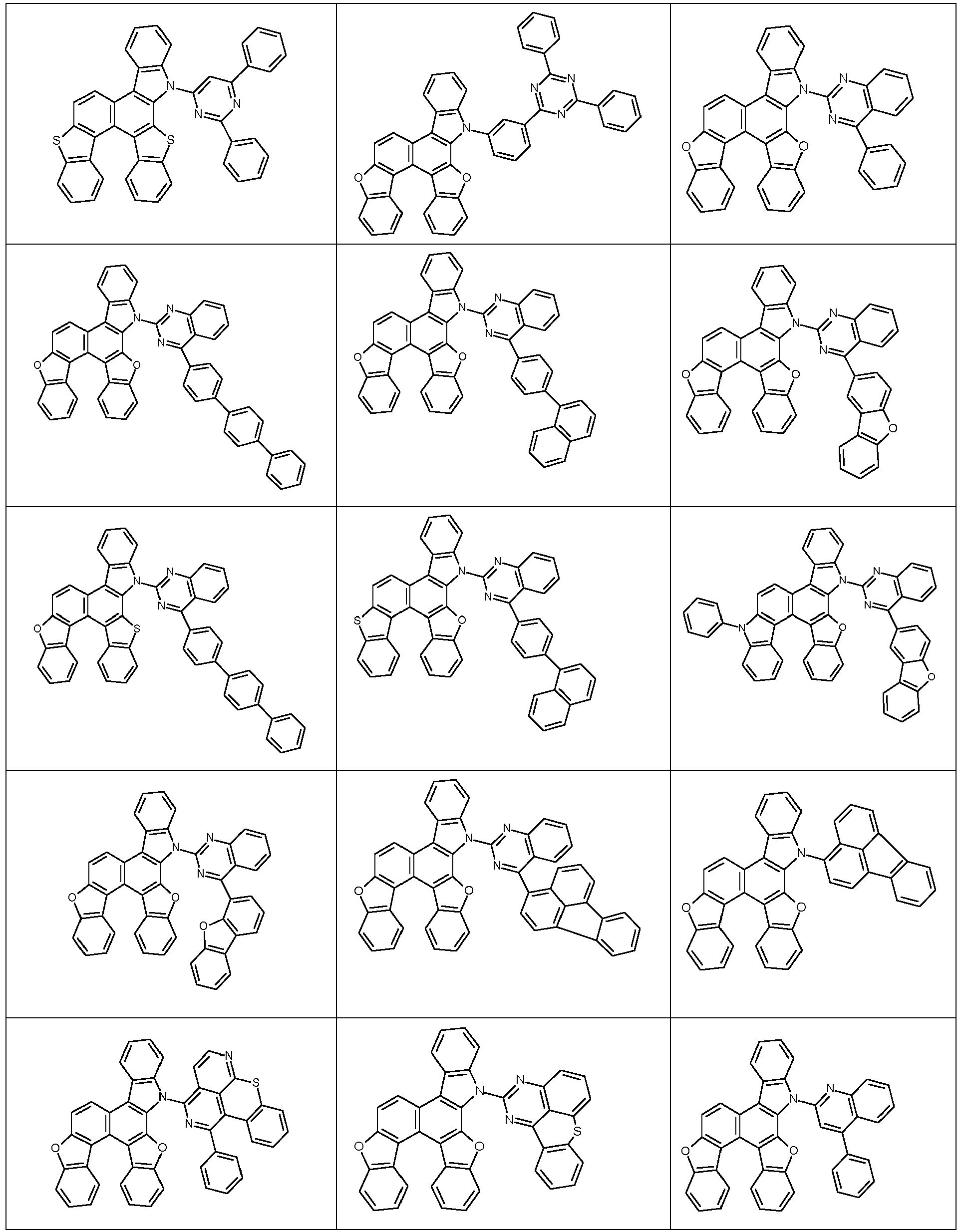

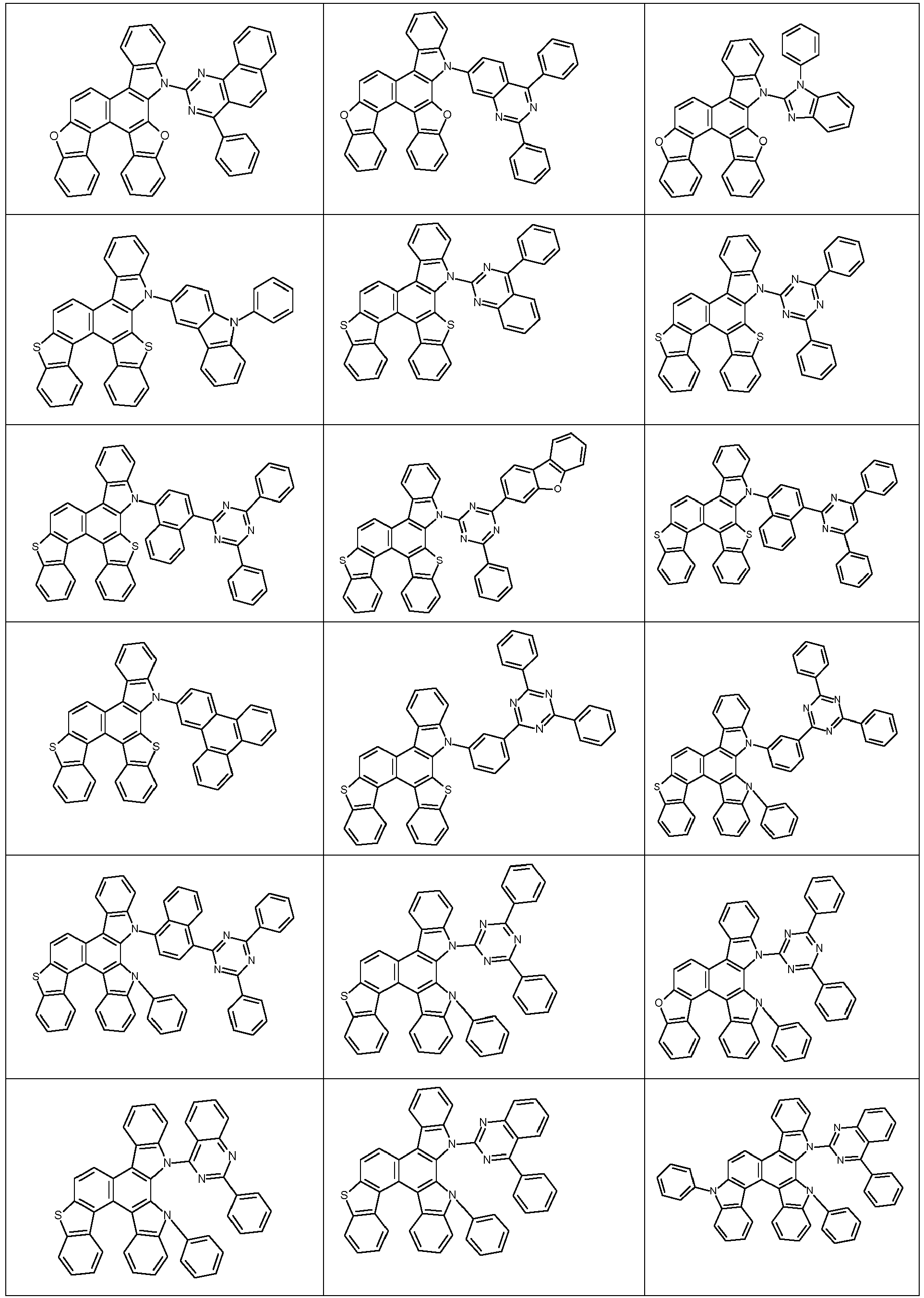

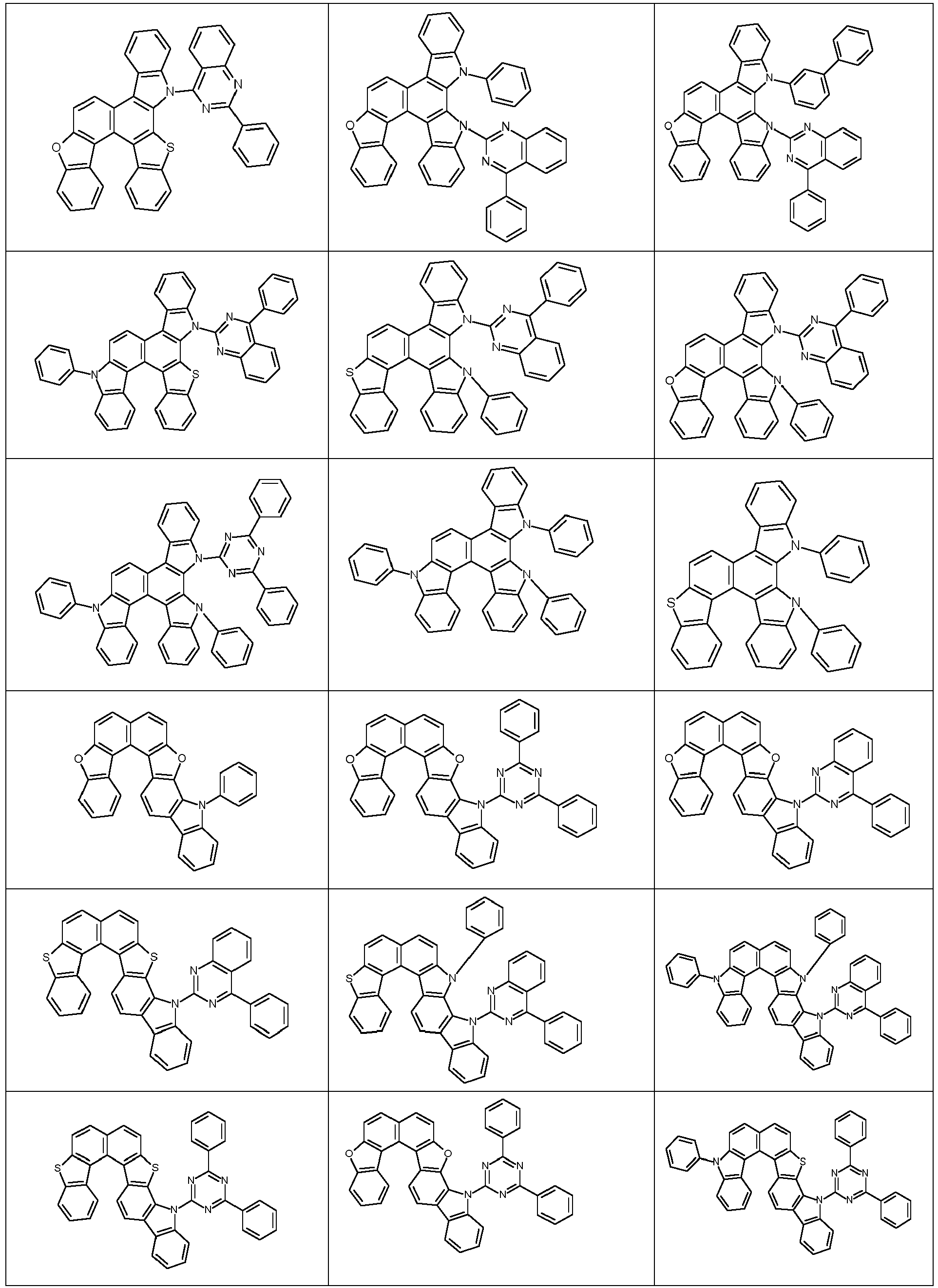

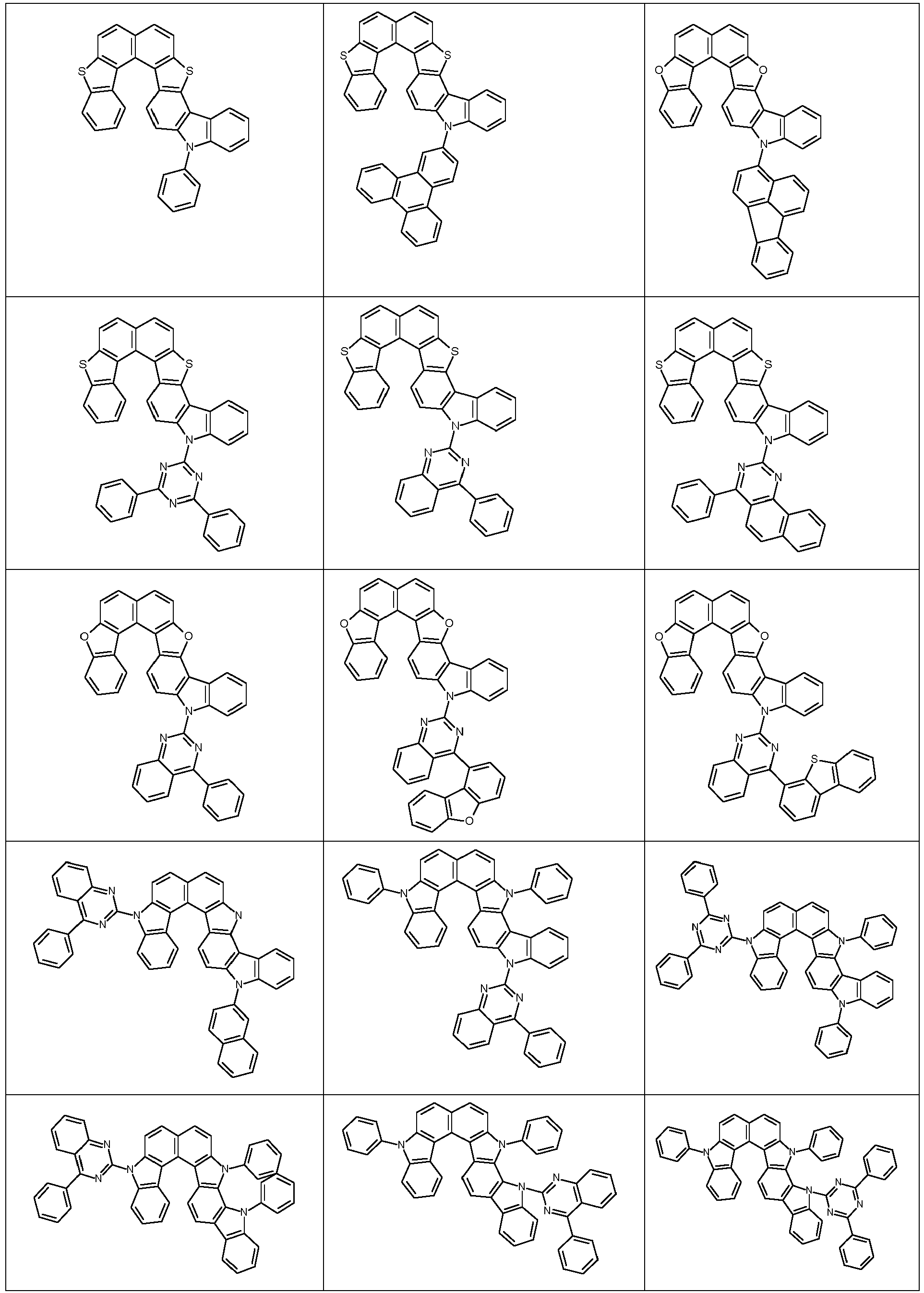

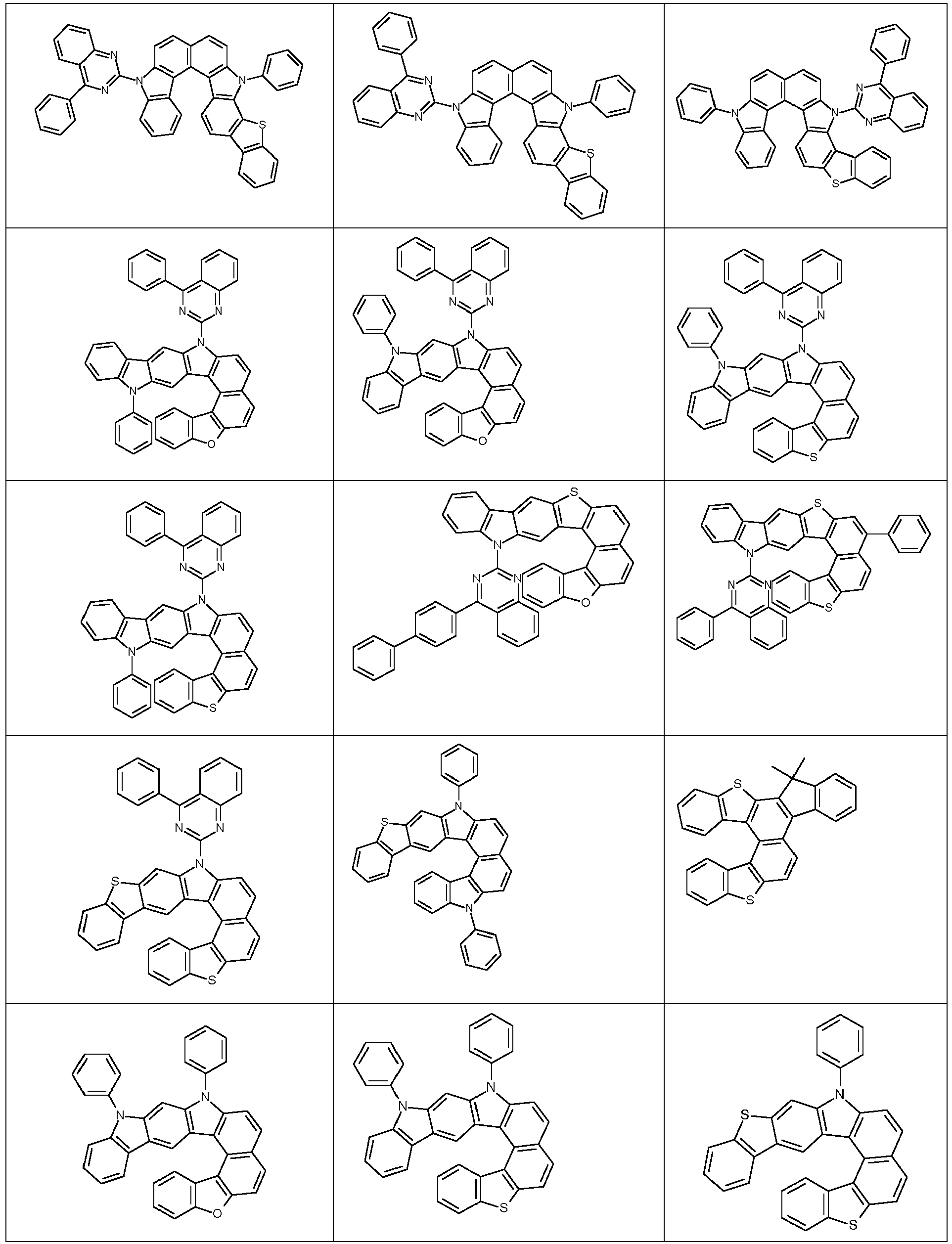

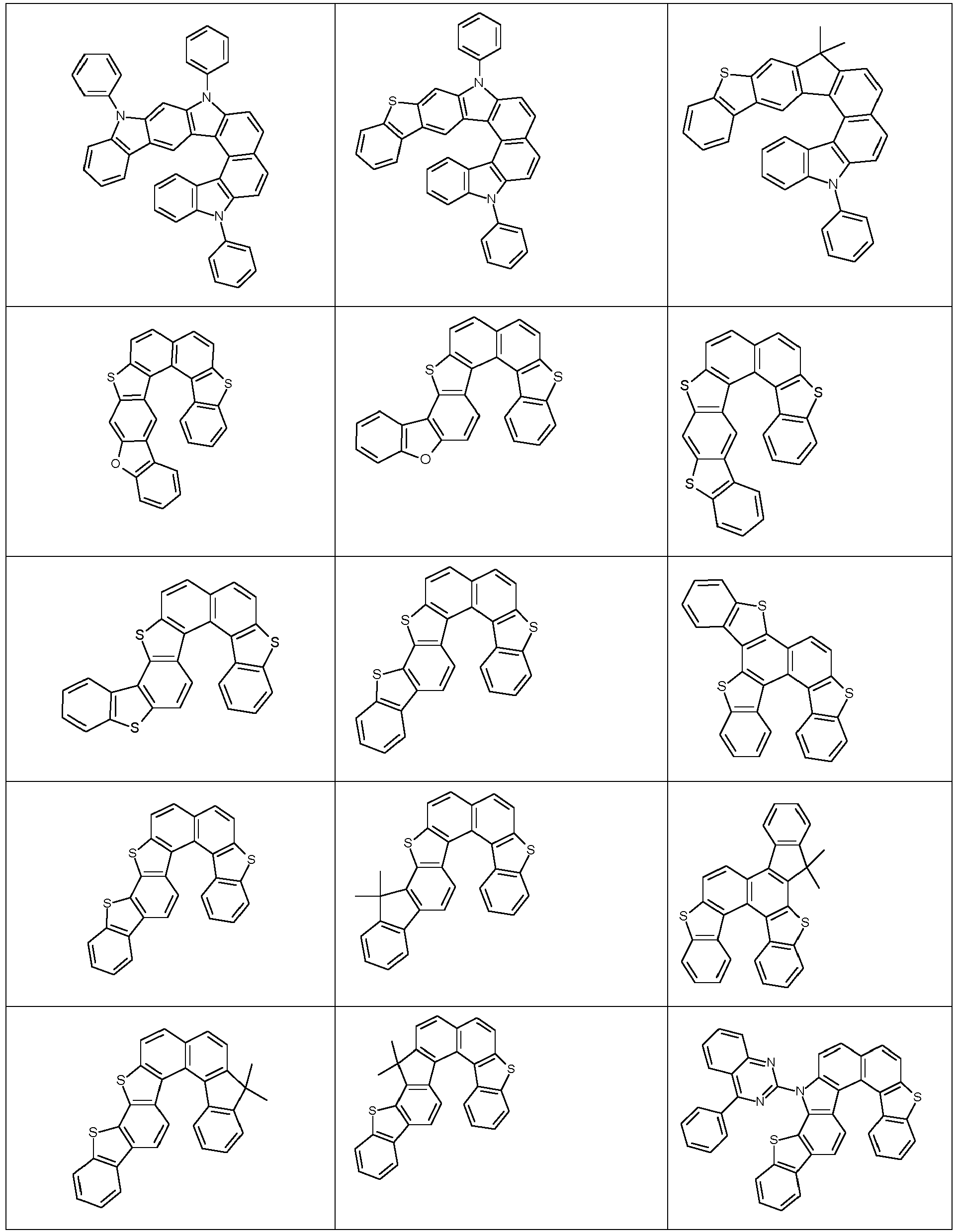

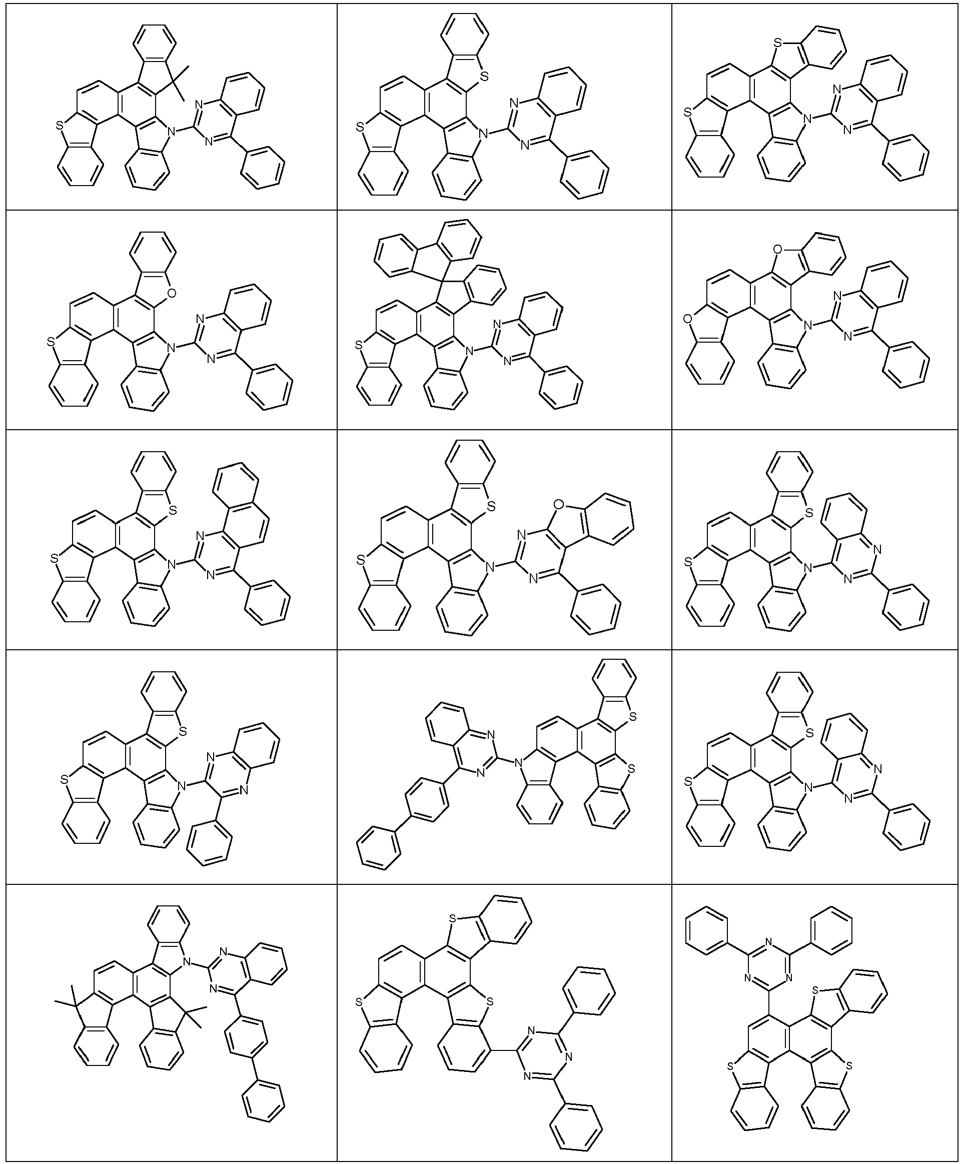

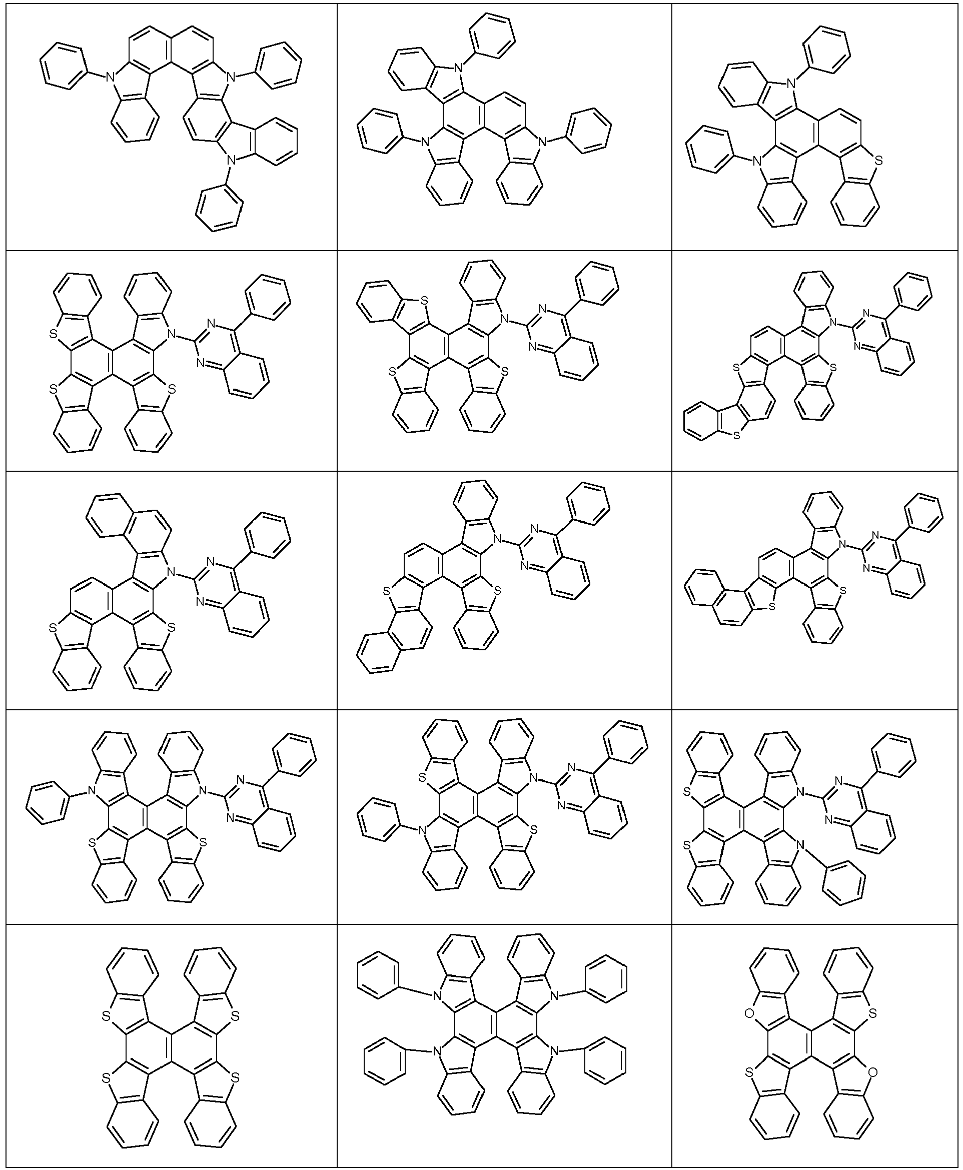

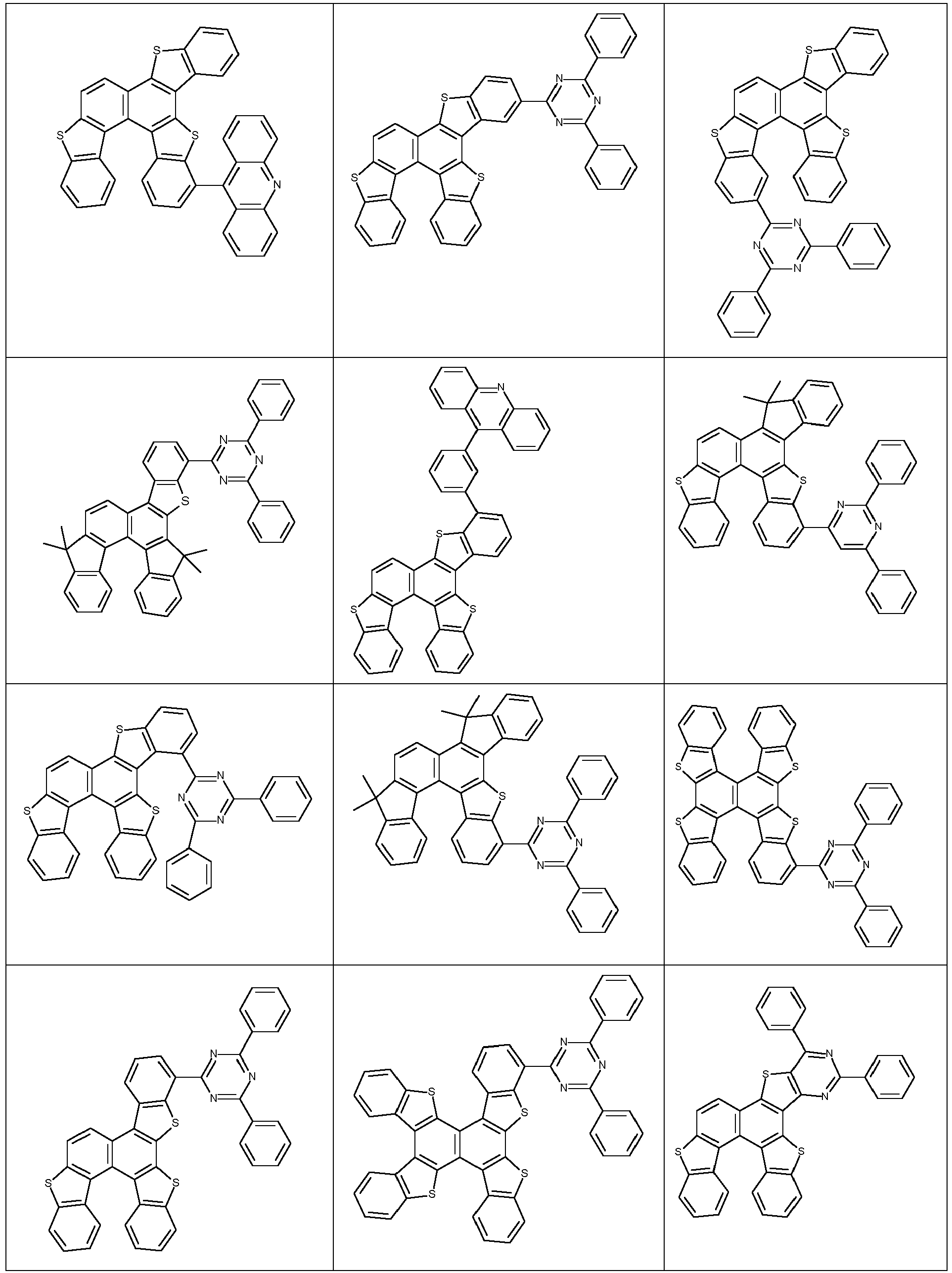

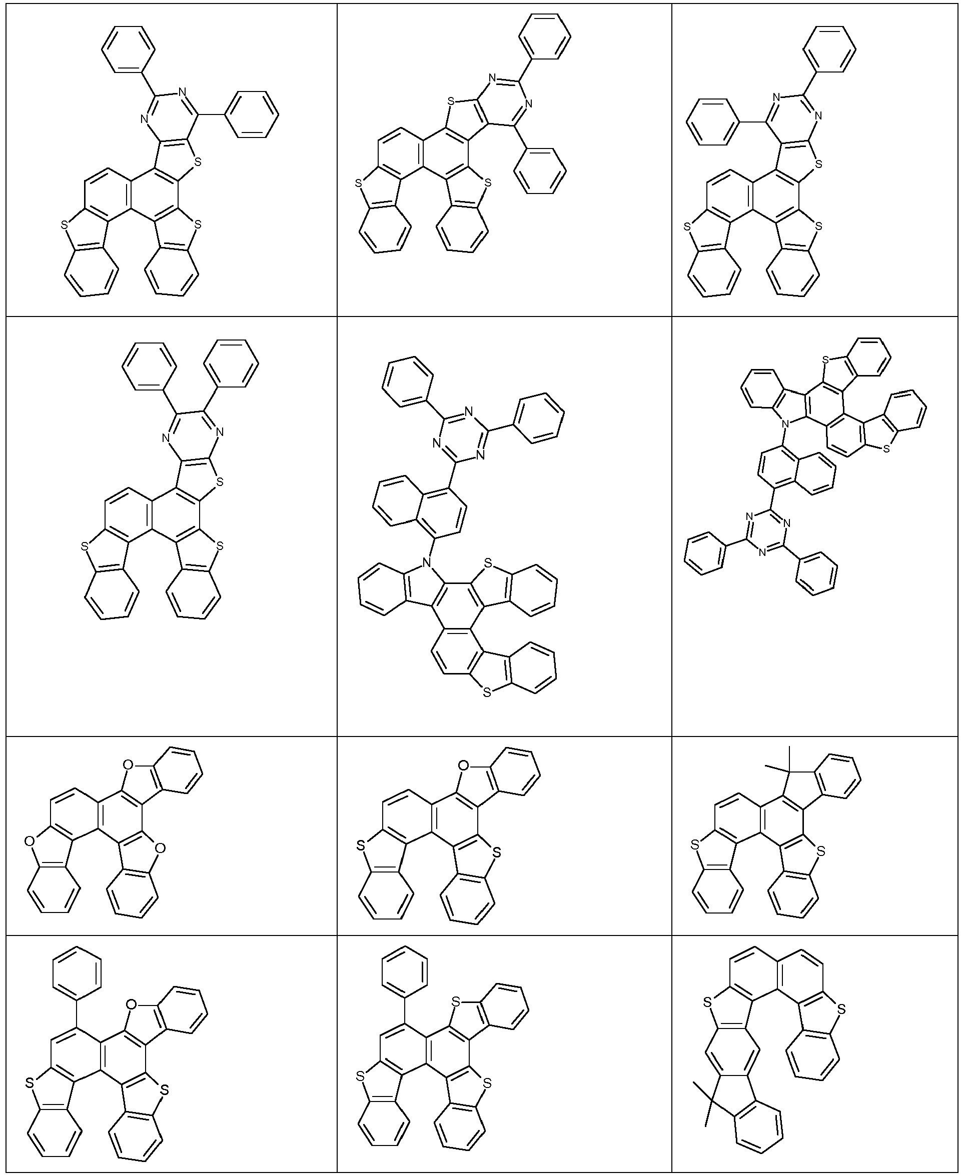

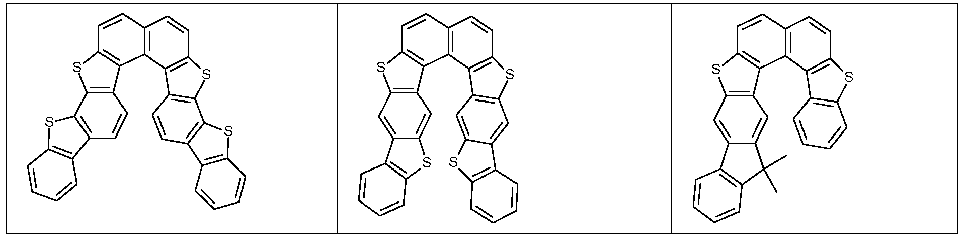

Die Grundstruktur der erfindungsgemäßen Verbindungen kann nach den in Schema 1 bis 4 skizzierten Wegen dargestellt werden. Dabei zeigt Schema 1 die Synthese des Grundgerüsts, an das noch keine Gruppe der Formel (2) ankondensiert ist. An dieses Grundgerüst kann durch Kupplungs- und Ringbildungsreaktion die Gruppe der Formel (2) ankondensiert werden (Schema 2 bis 4), wobei für Z¹, Z² und/oder Z³ gleich N-Ar die Gruppe Ar dann in einer nukleophilen aromatischen Substitution oder einer Kupplungsreaktion eingeführt werden kann.

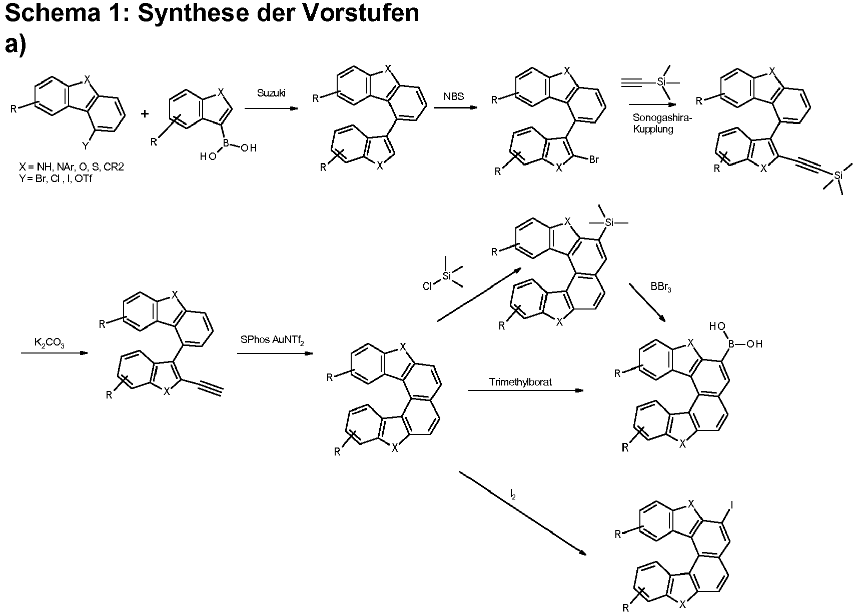

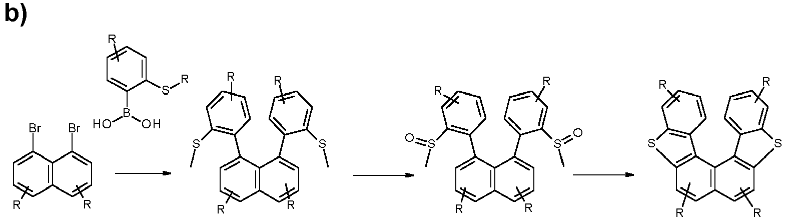

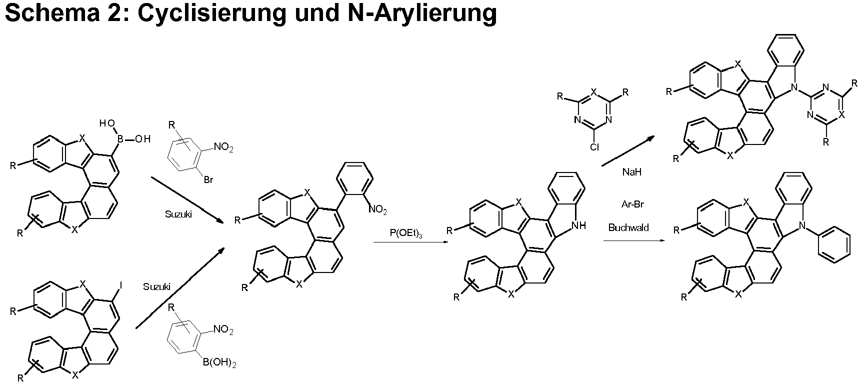

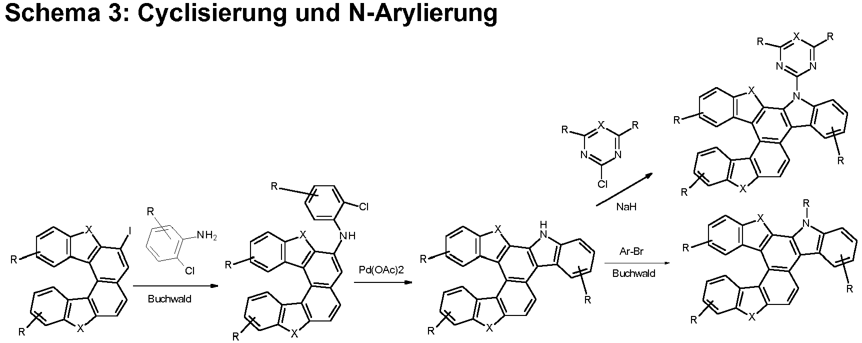

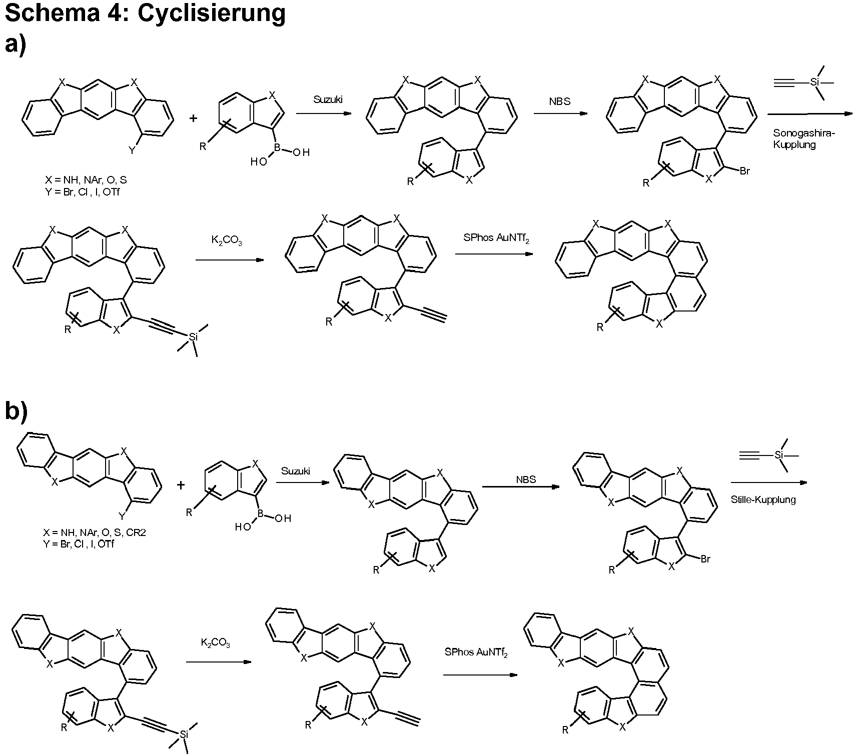

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Ein nochmals weiterer Gegenstand der vorliegenden Erfindung ist eine elektronische Vorrichtung enthaltend mindestens eine erfindungsgemäße Verbindung. Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur und je nach Wahl von Z¹, Z² und Z³. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrix-material für phosphoreszierende Emitter oder für Emitter, die TADF (thermally activated delayed fluorescence) zeigen, insbesondere für phosphoreszierende Emitter. Weiterhin kann die Verbindung der Formel (1) in einer emittierenden Schicht als fluoreszierender Emitter eingesetzt werden. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochblockierschicht und/oder in einer Lochtransportschicht und/oder in einer Exzitonenblockierschicht eingesetzt werden.

Wenn die erfindungsgemäßen Verbindungen als Matrixmaterial für phosphoreszierende Verbindungen eingesetzt werden, ist es bevorzugt, wenn mindestens eine der Gruppen Z¹, Z² und/oder Z³, insbesondere Z³, für N-Ar steht.

Wenn die erfindungsgemäßen Verbindungen als fluoreszierende Emitter eingesetzt werden, ist es bevorzugt, wenn die Gruppen Z¹, Z² und Z³ gleich oder verschieden, bevorzugt gleich, für O oder S stehen.

Wenn die erfindungsgemäßen Verbindungen als Elektronentransportmaterial eingesetzt werden, ist es bevorzugt, wenn mindestens zwei der Gruppen Z¹, Z² und Z³ gleich oder verschieden, bevorzugt gleich für O oder S stehen und der Grundkörper mit einem optional substituierten Triazin oder Pyrimidin substituiert ist.

Wenn die erfindungsgemäßen Verbindungen als Lochtransportmaterial eingesetzt werden, ist es bevorzugt, wenn mindestens zwei der Gruppen Z¹, Z² und Z³ für NAr stehen.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrix-material.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in WO 2006/130598, WO 2009/021126, WO 2009/124627 und WO 2010/006680.

In einer bevorzugten Ausführungsform der Erfindung werden die Materialien in Kombination mit einem weiteren Matrixmaterial eingesetzt. Bevorzugte Co-Matrixmaterialien sind gewählt aus der Gruppe der Biscarbazole, der verbrückten Carbazole, der Triarylamine, der Dibenzofuran-Carbazol-Derivate bzw. Dibenzofuran-Amin-Derivate und der Carbazolamine.

Bevorzugte Biscarbazole sind die Strukturen der folgenden Formeln (9) und (10), 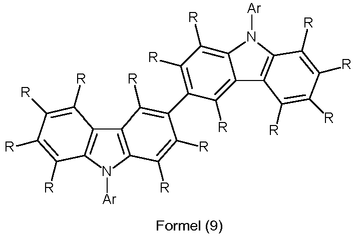 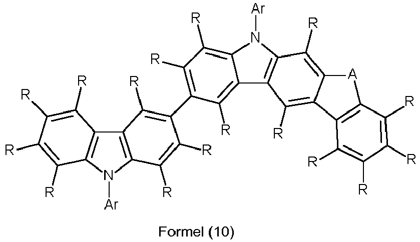 wobei Ar und A die oben genannten Bedeutungen aufweisen und R die oben genannten Bedeutungen aufweist, jedoch Reste R hier auch miteinander ein aromatisches oder heteroaromatisches Ringsystem bilden können. In einer bevorzugten Ausführungsform der Erfindung steht A für CR₂.

Bevorzugte Ausführungsformen der Verbindungen der Formeln (9) bzw. (10) sind die Verbindungen der folgenden Formeln (9a) bzw. (10a), 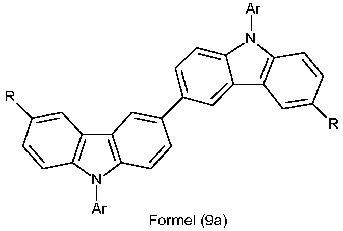 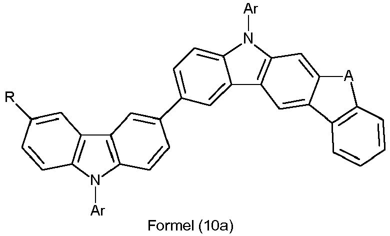 wobei die verwendeten Symbole die oben genannten Bedeutungen aufweisen.

Beispiele für geeignete Verbindungen gemäß Formel (9) oder (10) sind die nachfolgend abgebildeten Verbindungen.

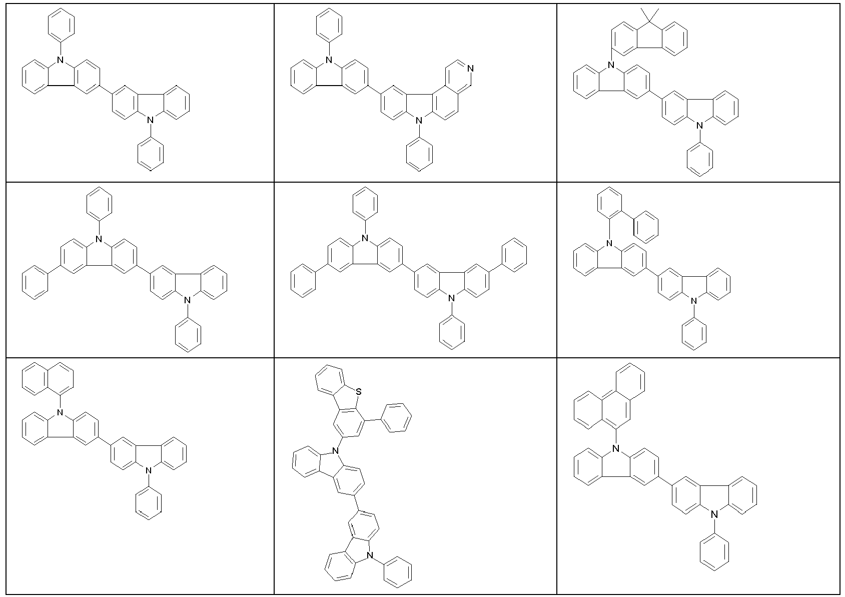

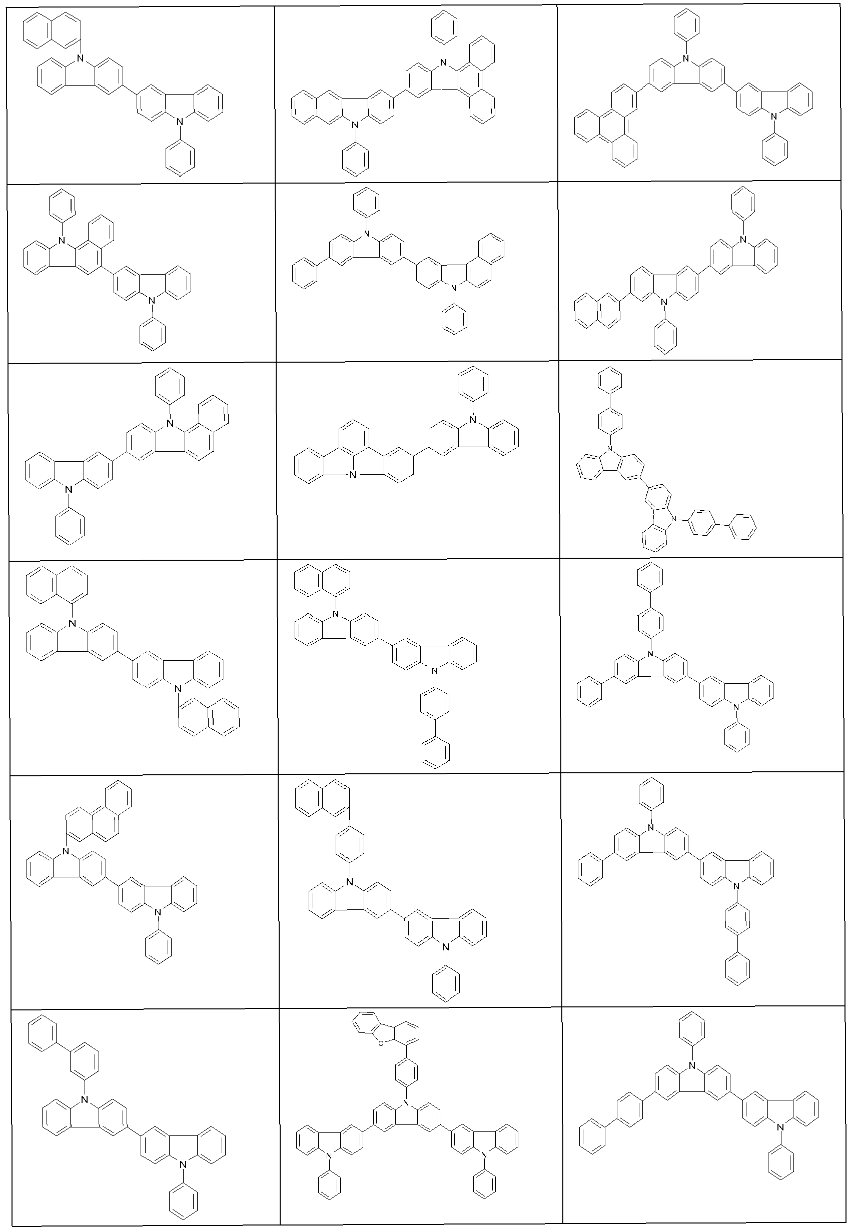

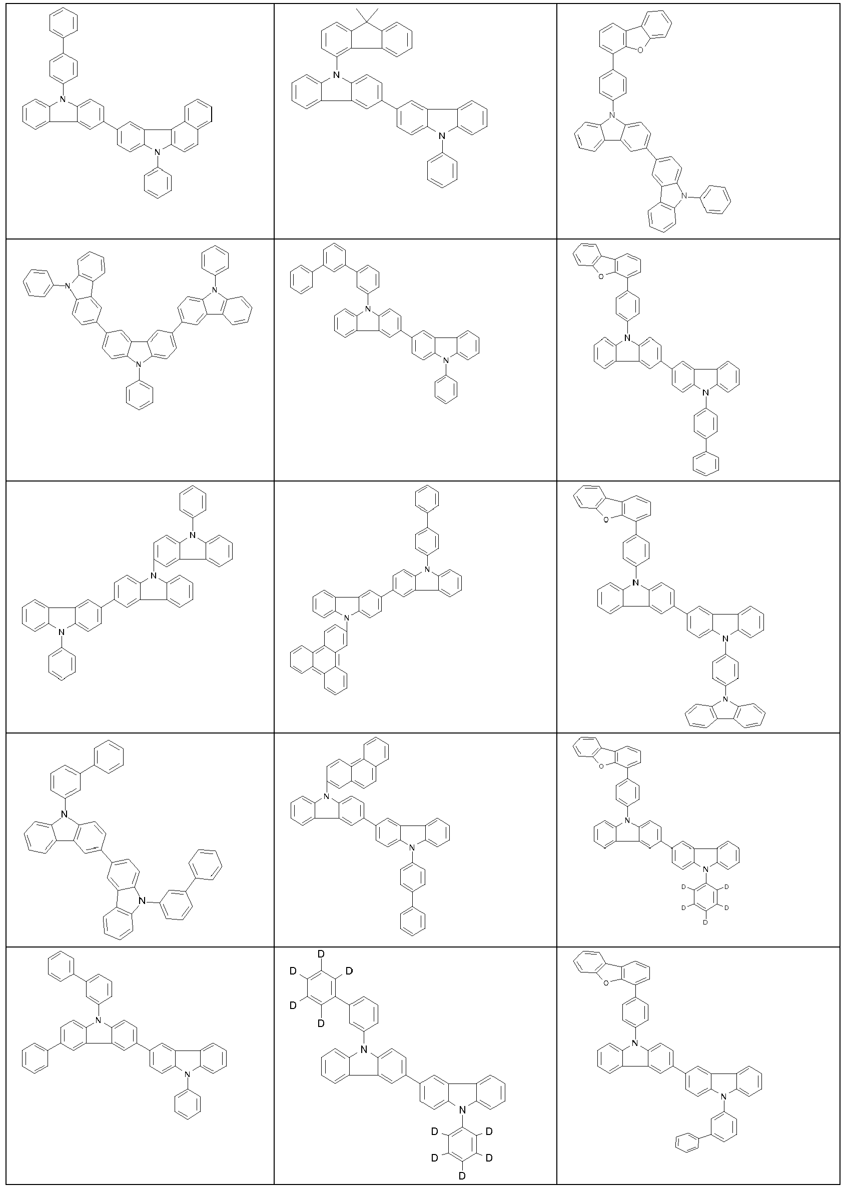

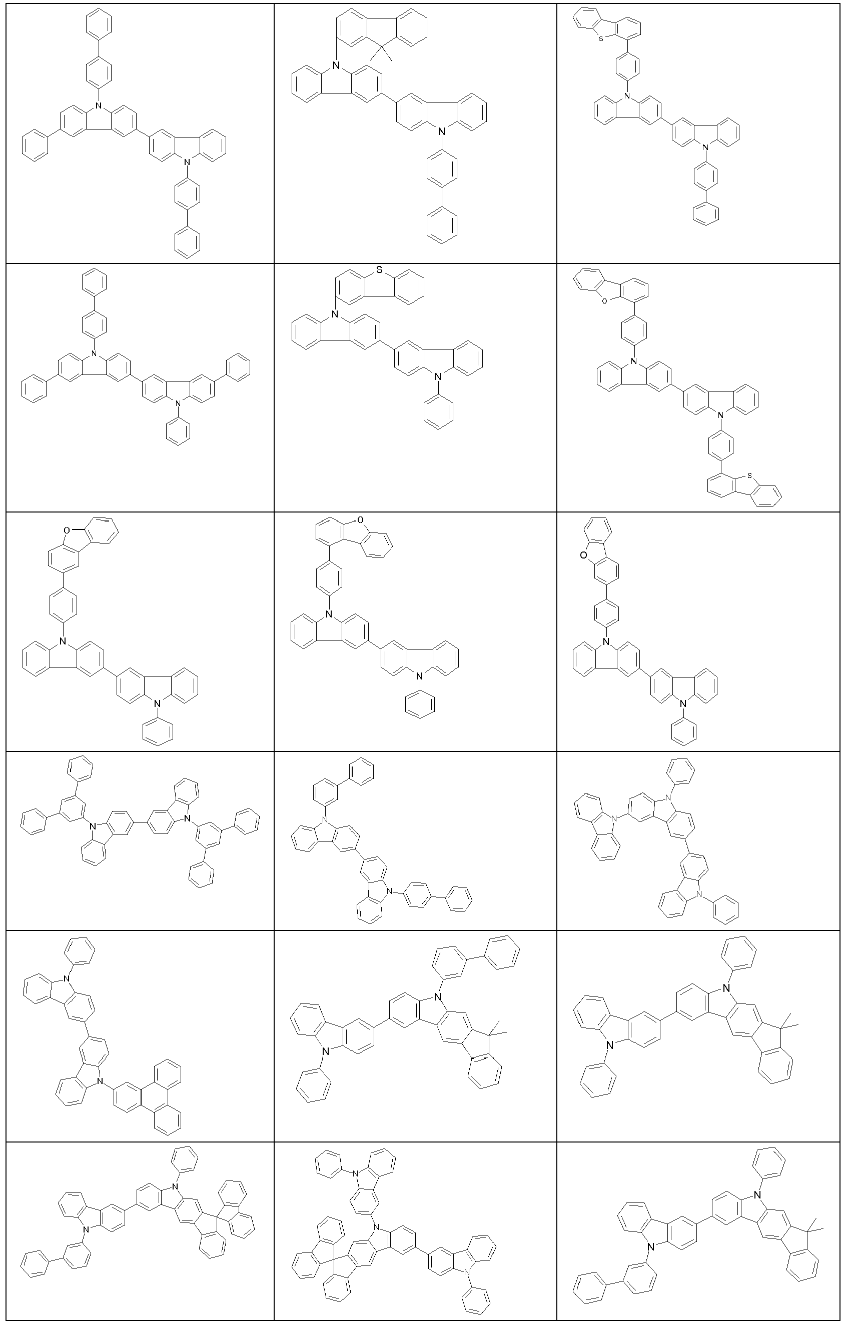

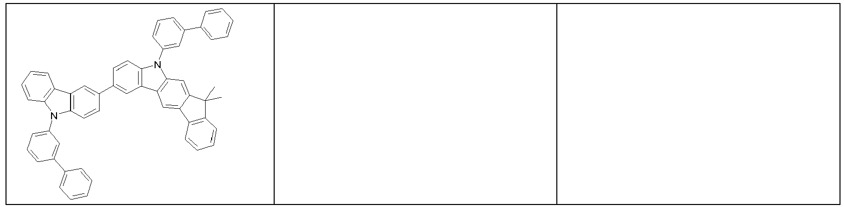

Bevorzugte verbrückte Carbazole sind die Strukturen der folgenden Formel (11), 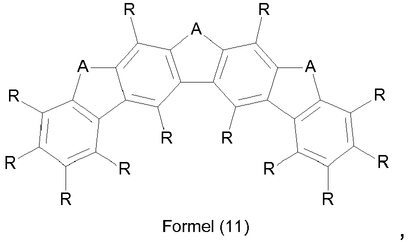 wobei A und R die oben genannten Bedeutungen aufweisen und A bevorzugt gleich oder verschieden bei jedem Auftreten ausgewählt ist aus der Gruppe bestehend aus NAr und CR₂.

Eine bevorzugte Ausführungsform der Formel (11) sind die Verbindungen der Formel (11a), besonders bevorzugt sind die Verbindungen der Formel (11b), und ganz besonders bevorzugt sind die Verbindungen der Formel (11c), 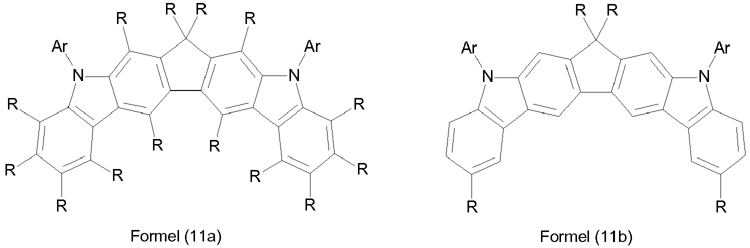 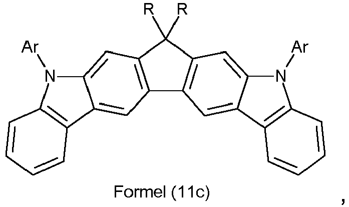 wobei Ar und R die oben genannten Bedeutungen aufweisen. Dabei steht Ar bevorzugt gleich oder verschieden bei jedem Auftreten für ein aromatisches Ringsystem mit 6 bis 18 aromatischen Ringatomen, beispielsweise für Phenyl, ortho-, meta- oder para-Biphenyl oder Terphenyl. Der Rest R am Inden-Kohlenstoffatom steht bevorzugt gleich oder verschieden bei jedem Auftreten für eine Alkylgruppe mit 1 bis 5 C-Atomen, insbesondere für Methyl, oder ein aromatisches Ringsystem mit 6 bis 18 aromatischen Ringatomen, insbesondere Phenyl.

Bevorzugte Triarylamine sind die Strukturen der folgenden Formel (12), 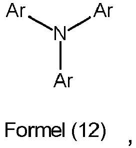 wobei Ar die oben genannten Bedeutungen aufweist.

Bevorzugte Dibenzofuran-Derivate sind die Verbindungen der folgenden Formel (13), 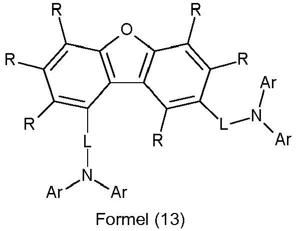 wobei der Sauerstoff auch durch Schwefel ersetzt sein kann, so dass ein Dibenzothiophen entsteht, L für eine Einfachbindung oder ein aromatisches oder heteroaromatisches Ringsystem mit 5 bis 30 aromatischen Ringatomen steht, welches auch durch eine oder mehrere Reste R substituiert sein kann, und R und Ar die oben genannten Bedeutungen aufweisen. Dabei können die beiden Gruppen Ar, die an dasselbe Stickstoffatom binden, oder eine Gruppe Ar und eine Gruppe L, die an dasselbe Stickstoffatom binden, auch miteinander verbunden sein, beispielsweise zu einem Carbazol.

Bevorzugte Ausführungsformen der Verbindungen der Formel (13) sind die Verbindungen der folgenden Formeln (13a) und (13b), 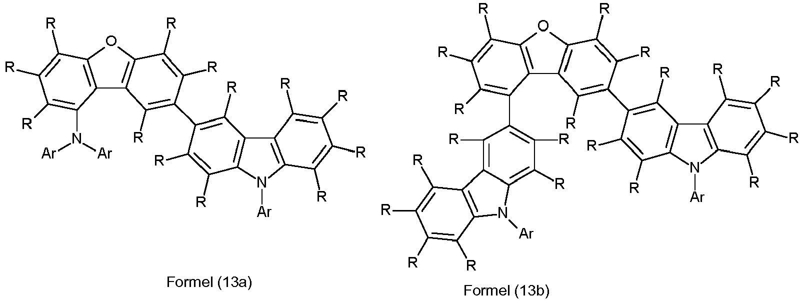 wobei der Sauerstoff auch durch Schwefel ersetzt sein kann, so dass ein Dibenzothiophen entsteht, und R und Ar die oben genannten Bedeutungen aufweisen, wobei zwei benachbarte Reste R, insbesondere am Carbazol, hier auch miteinander ein aromatisches oder heteroaromatisches Ringsystem bilden können.

Besonders bevorzugte Ausführungsformen sind die Verbindungen der folgenden Formeln (13c) und (13d), 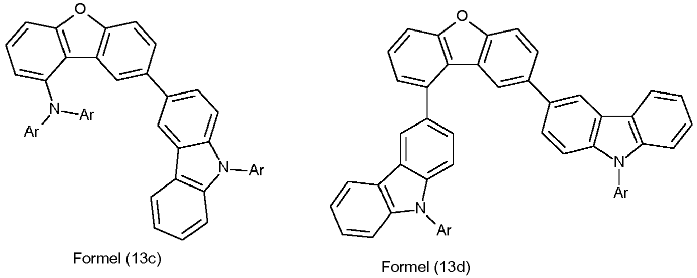 wobei der Sauerstoff auch durch Schwefel ersetzt sein kann, so dass ein Dibenzothiophen entsteht, und Ar die oben genannten Bedeutungen aufweist, wobei zwei benachbarte Reste R, insbesondere am Carbazol, hier auch miteinander ein aromatisches oder heteroaromatisches Ringsystem bilden können.

Beispiele für geeignete Dibenzofuran-Derivate sind die nachfolgend abgebildeten Verbindungen.

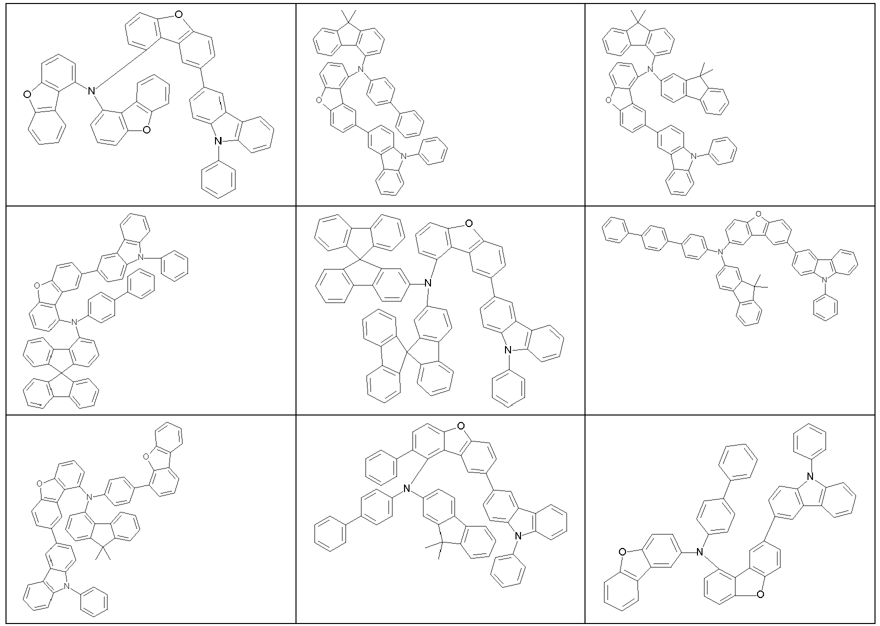

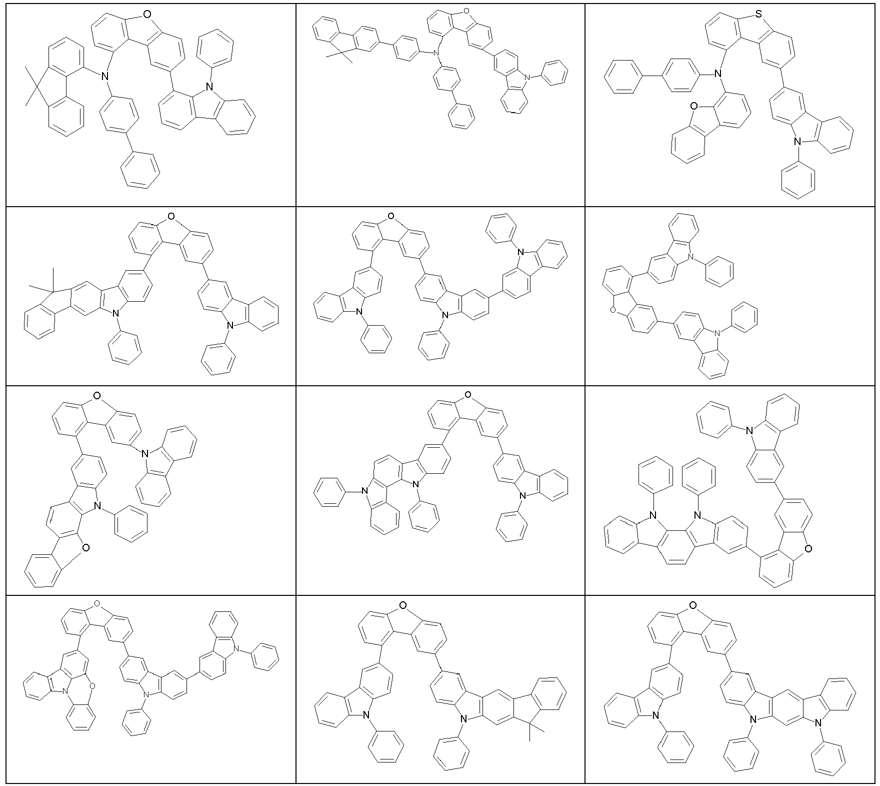

Bevorzugte Carbazolamine sind die Strukturen der folgenden Formeln (14), (15) und (16), 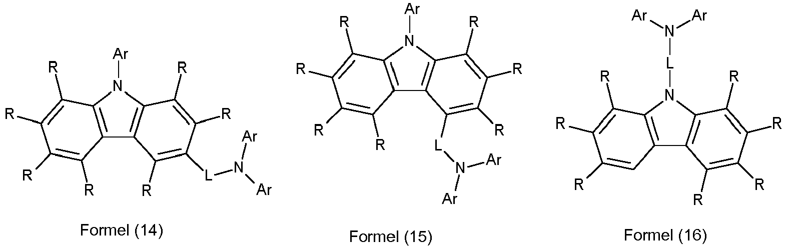 wobei L für ein aromatisches oder heteroaromatisches Ringsystem mit 5 bis 30 aromatischen Ringatomen steht, welches mit einem oder mehreren Resten R substituiert sein kann, und R und Ar die oben genannten Bedeutungen aufweisen, wobei zwei benachbarte Reste R hier auch ein aromatisches Ringsystem bilden können.

Beispiele für geeignete Carbazolamin-Derivate sind die nachfolgend abgebildeten Verbindungen.

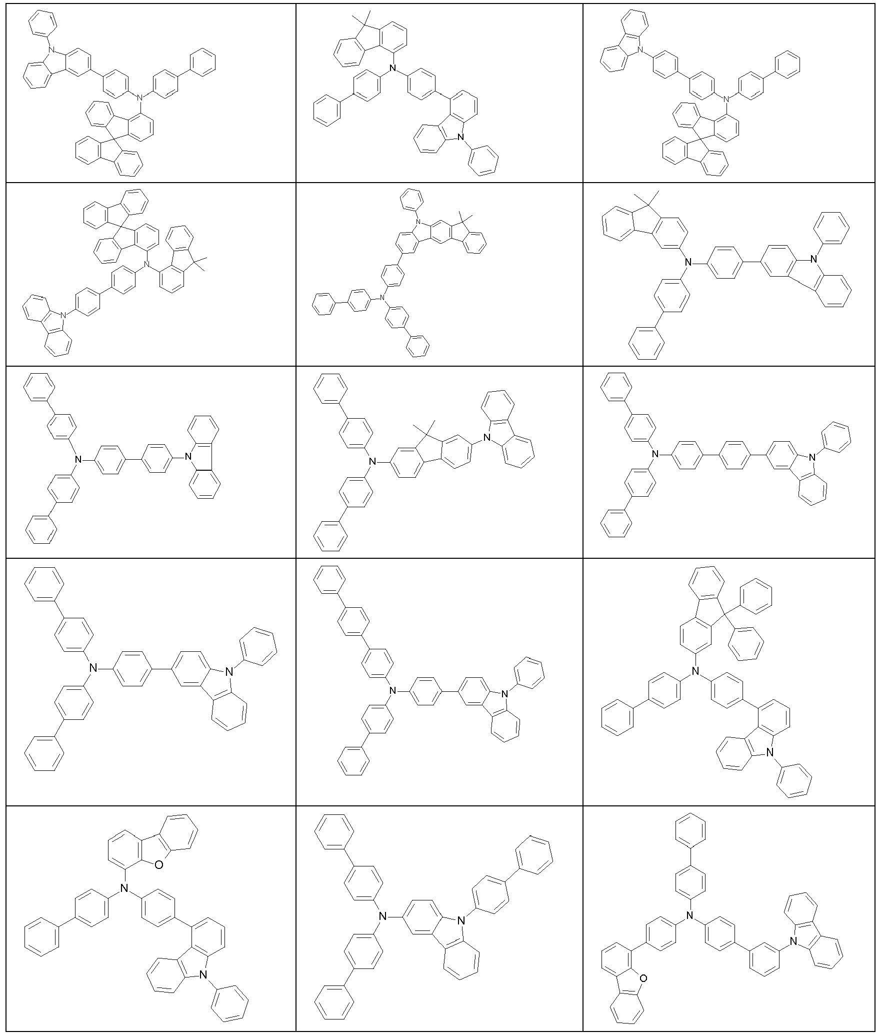

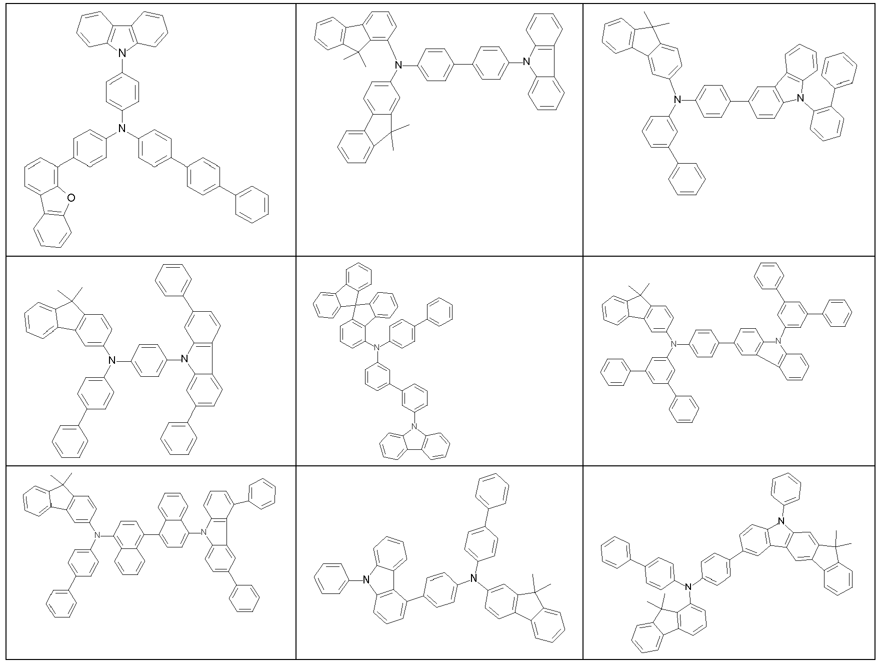

Bevorzugte Indeno- und Indolocarbazolderivate sind die Strukturen der folgenden Formeln (17), (18), (19) und (20), 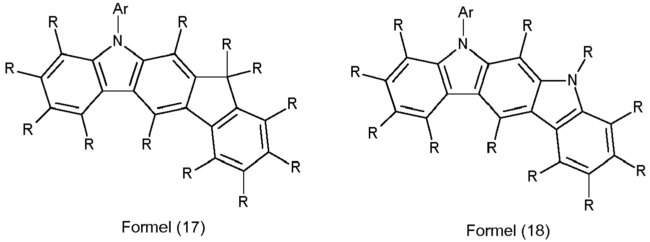 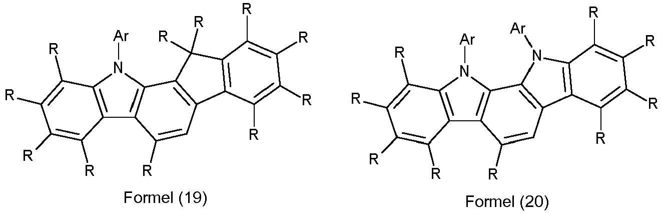 wobei die verwendeten Symbole die oben genannten Bedeutungen aufweisen.

Beispiele für Indolo- und Indenocarbazolderivate, welche je nach Substitutionsmuster als loch- oder elektronentransportierende Matrixmaterialien eingesetzt werden können, sind die folgenden Strukturen:

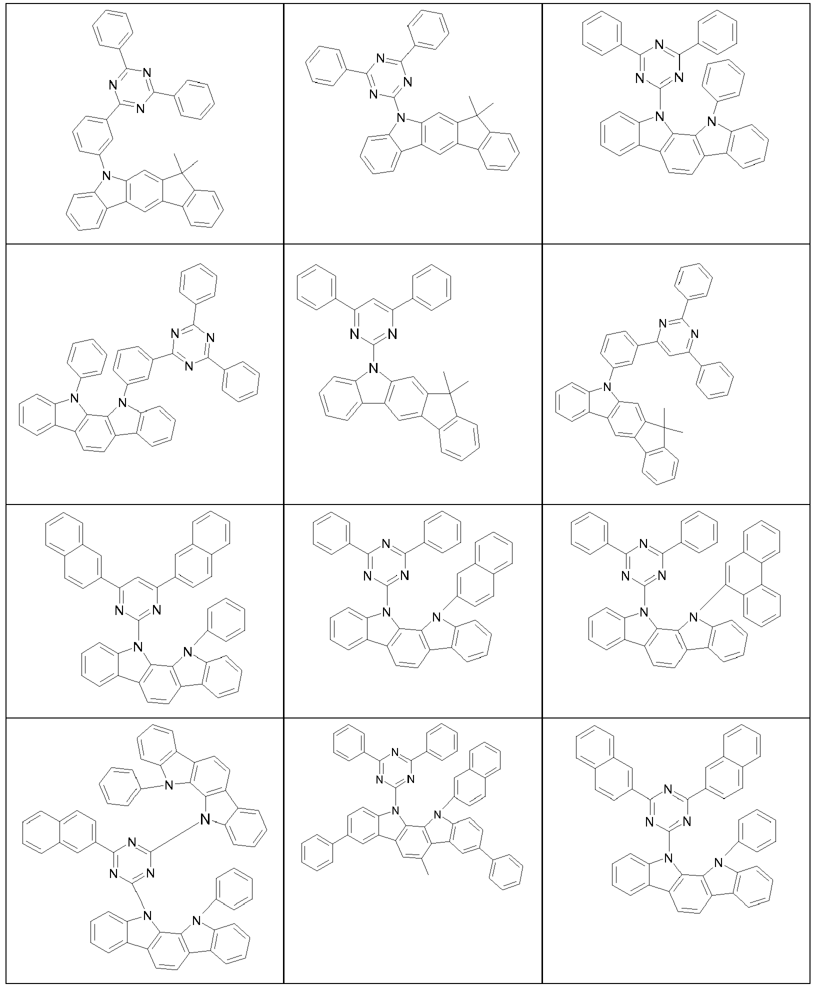

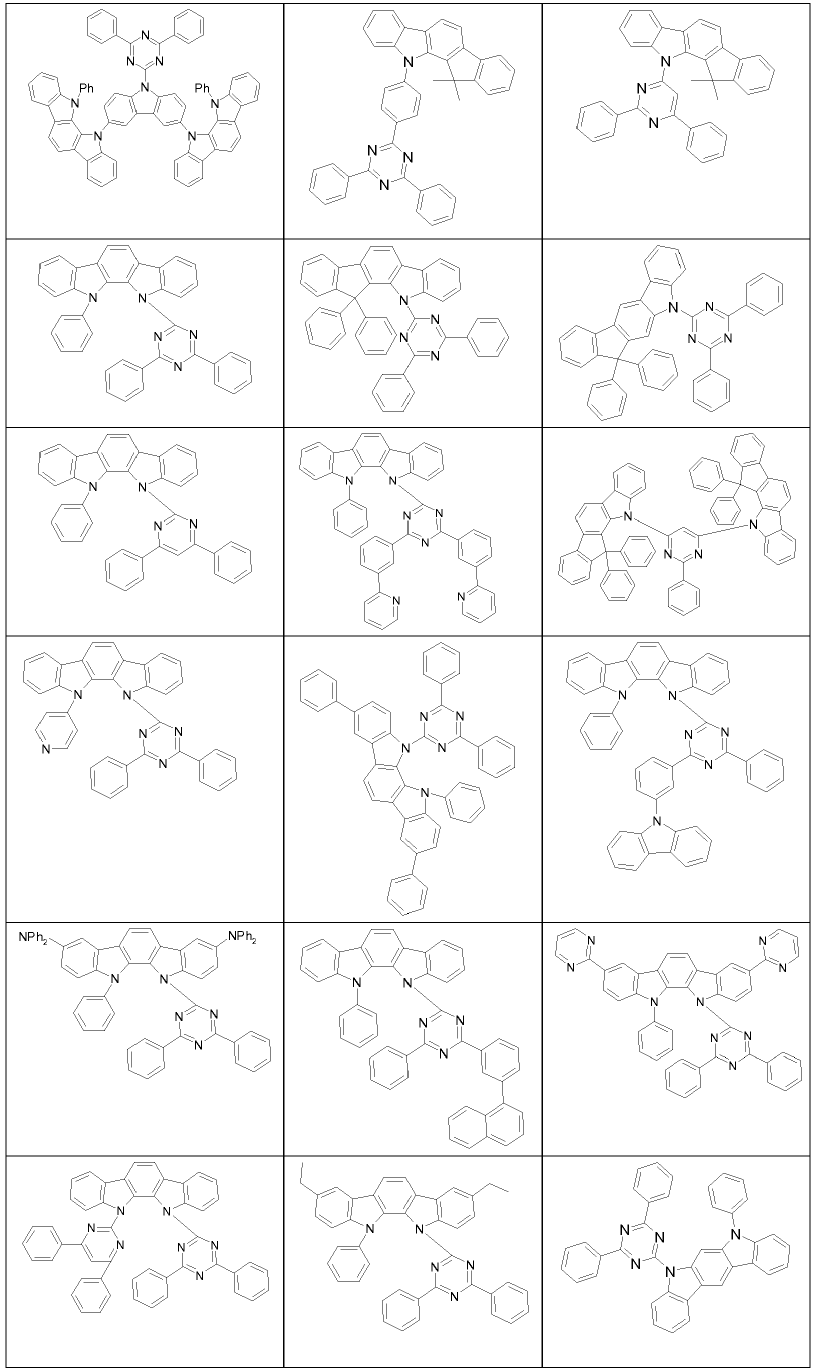

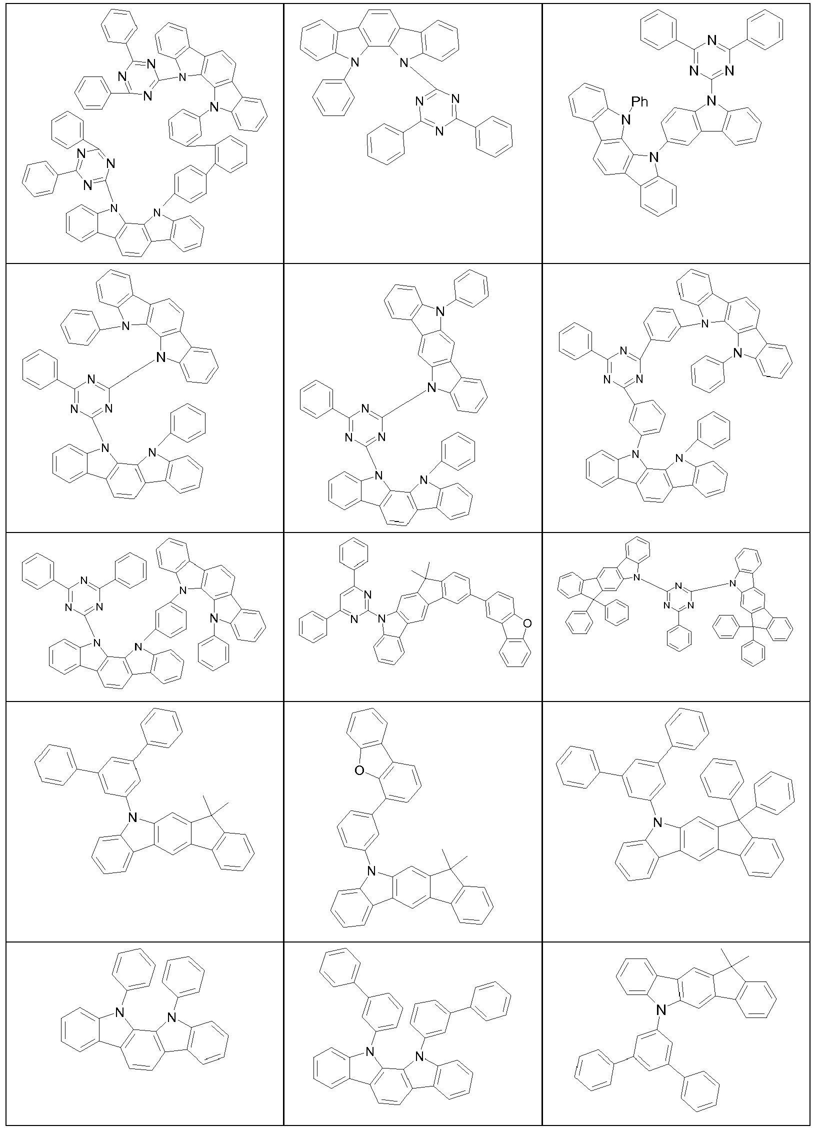

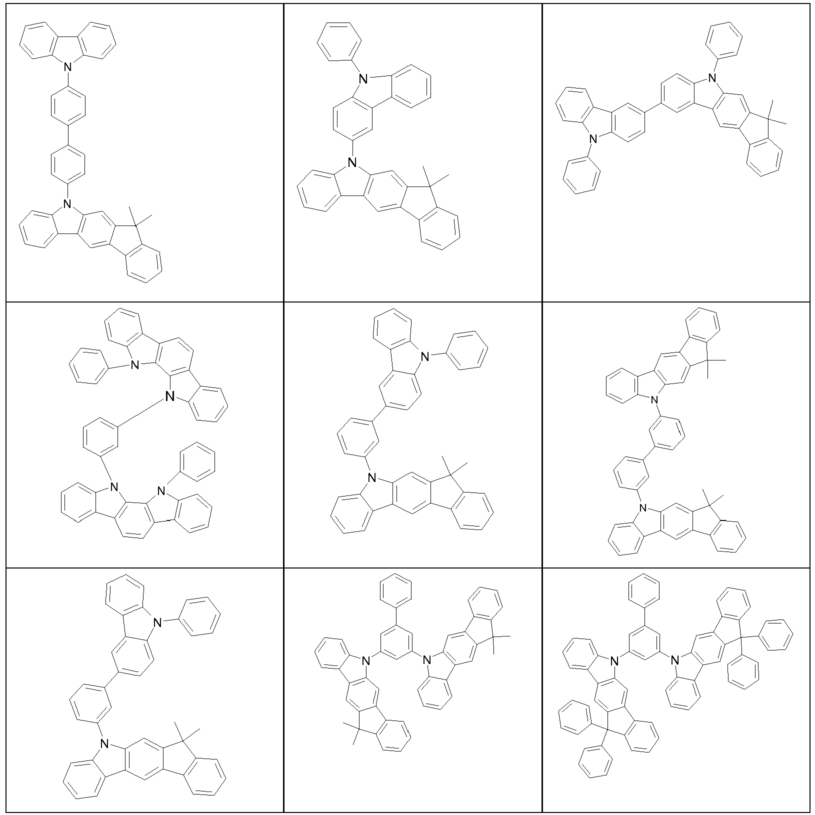

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und WO 2018/041769, sowie den noch nicht offen gelegten Patentanmeldungen EP 17182995.5, EP 17205103.9, EP 17206950.2 und EP 18156388.3 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

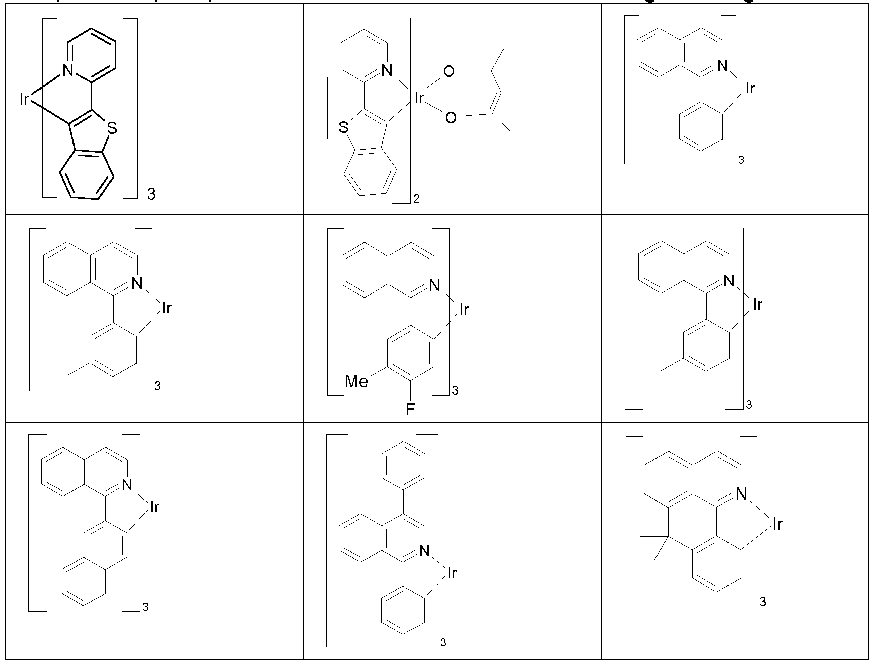

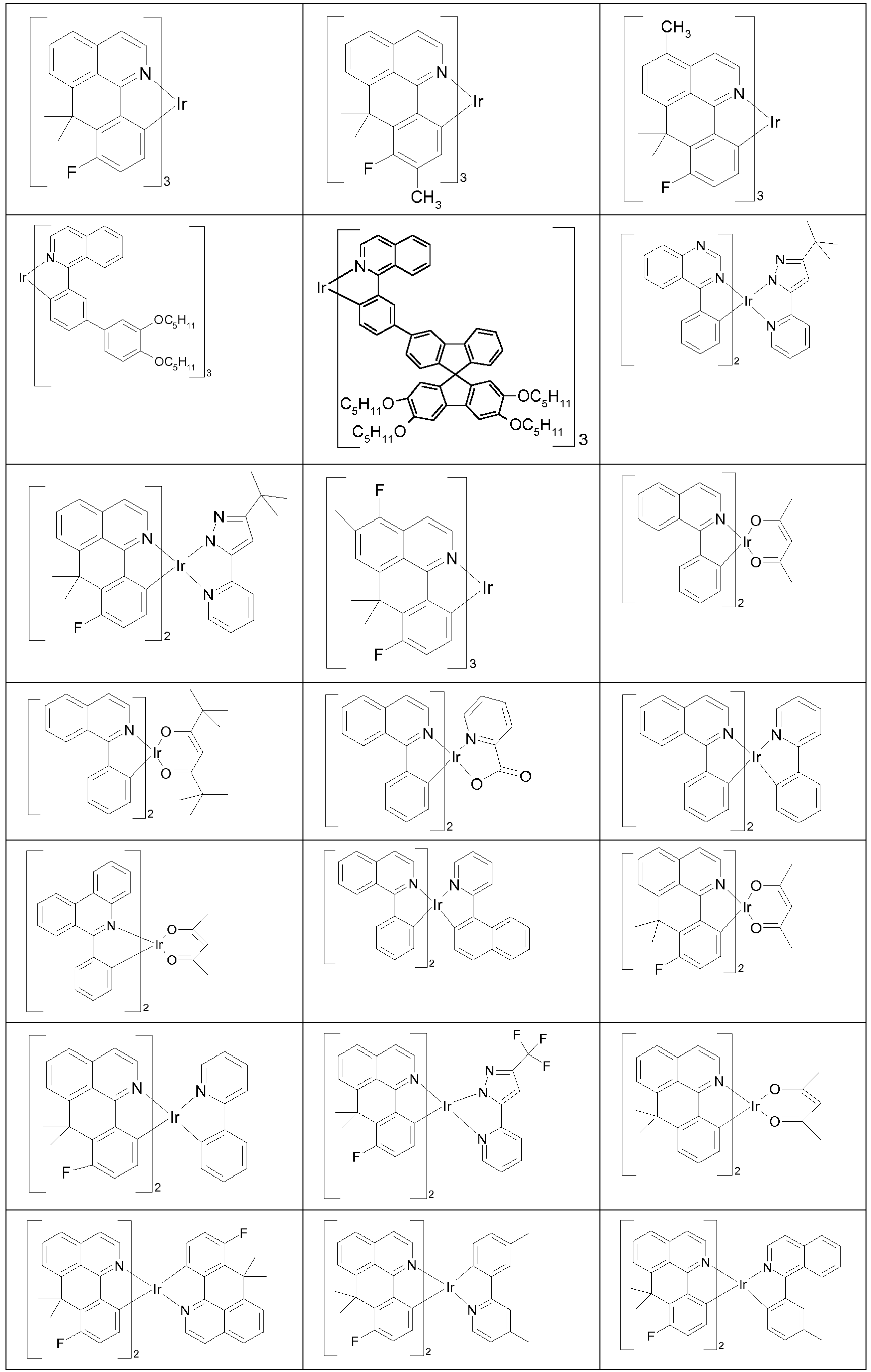

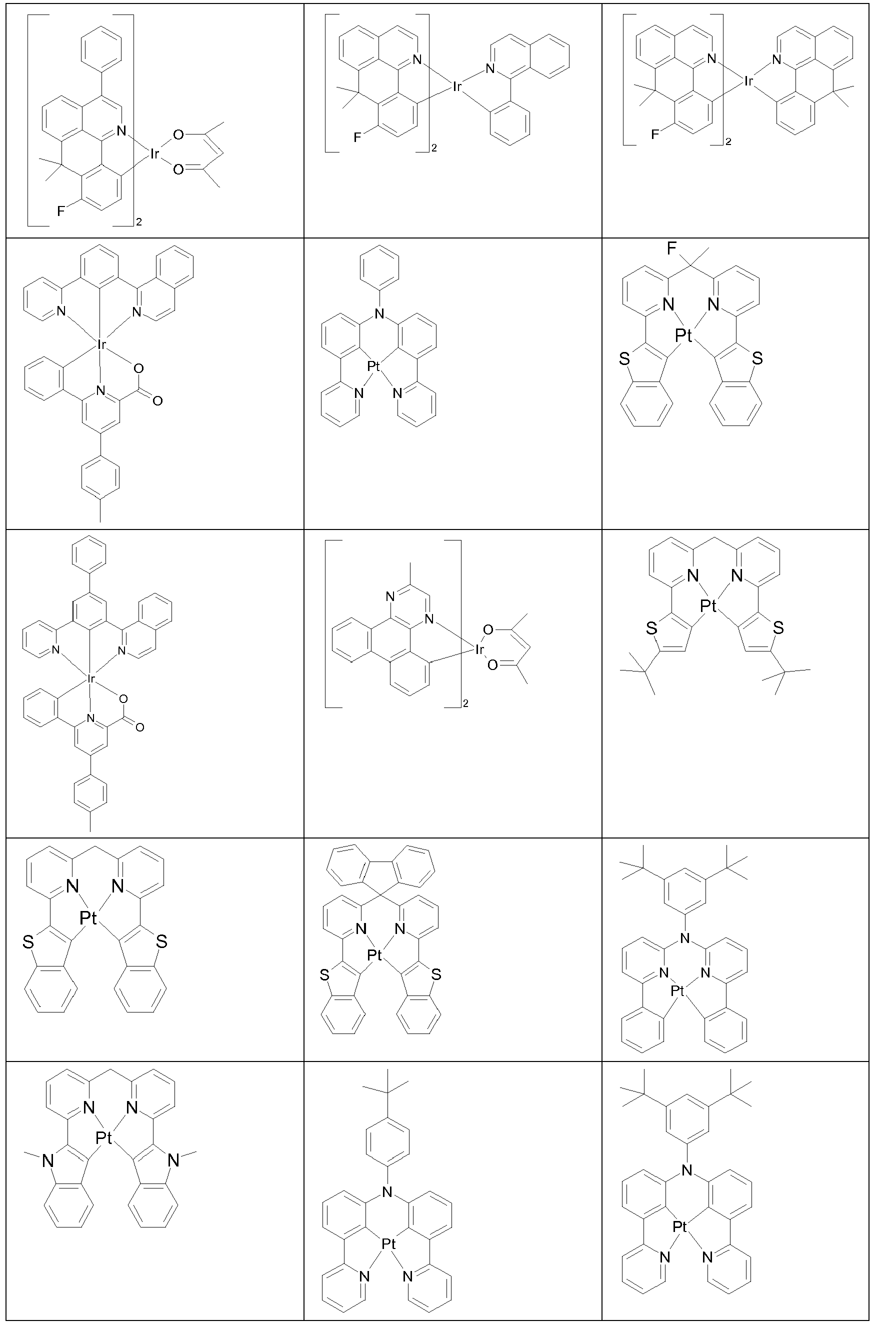

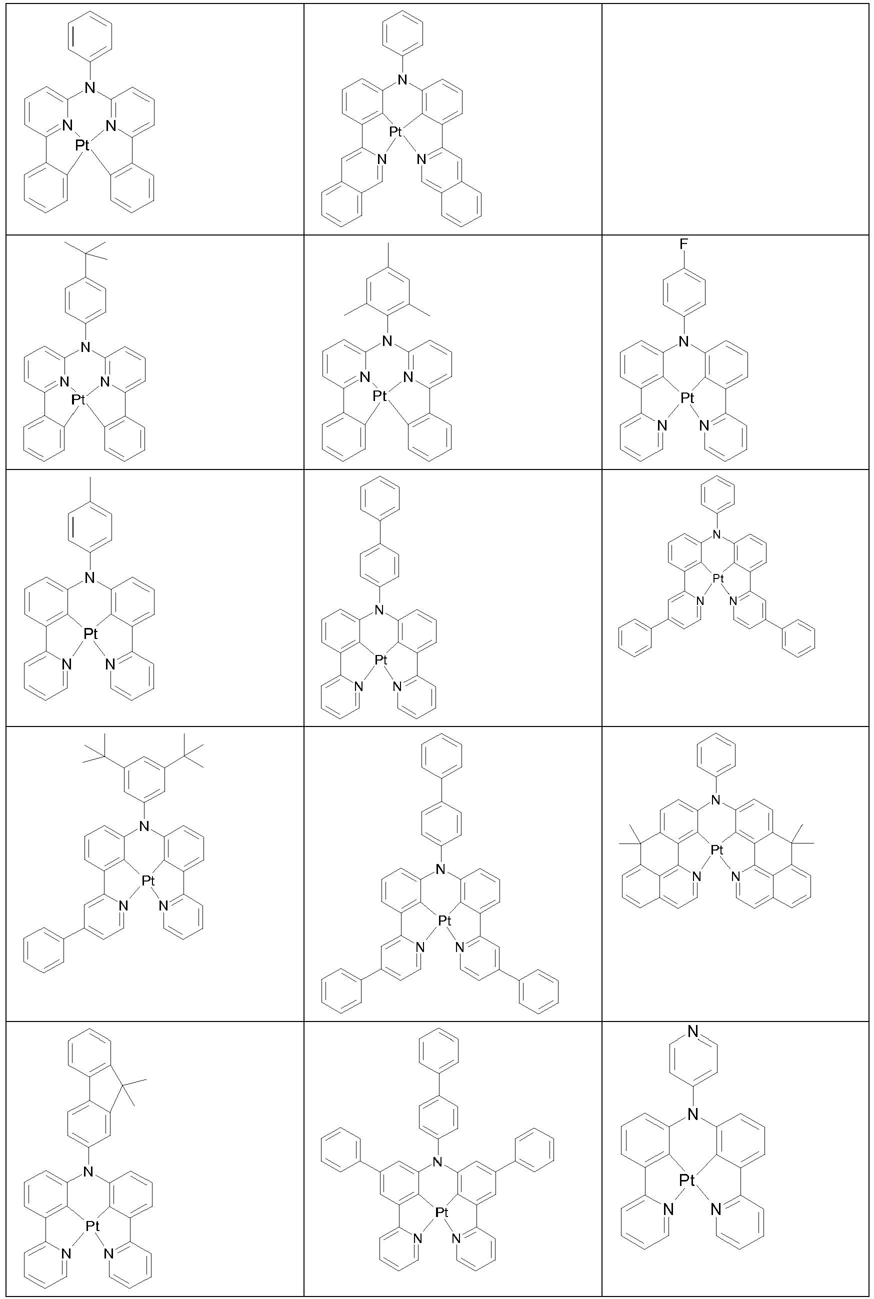

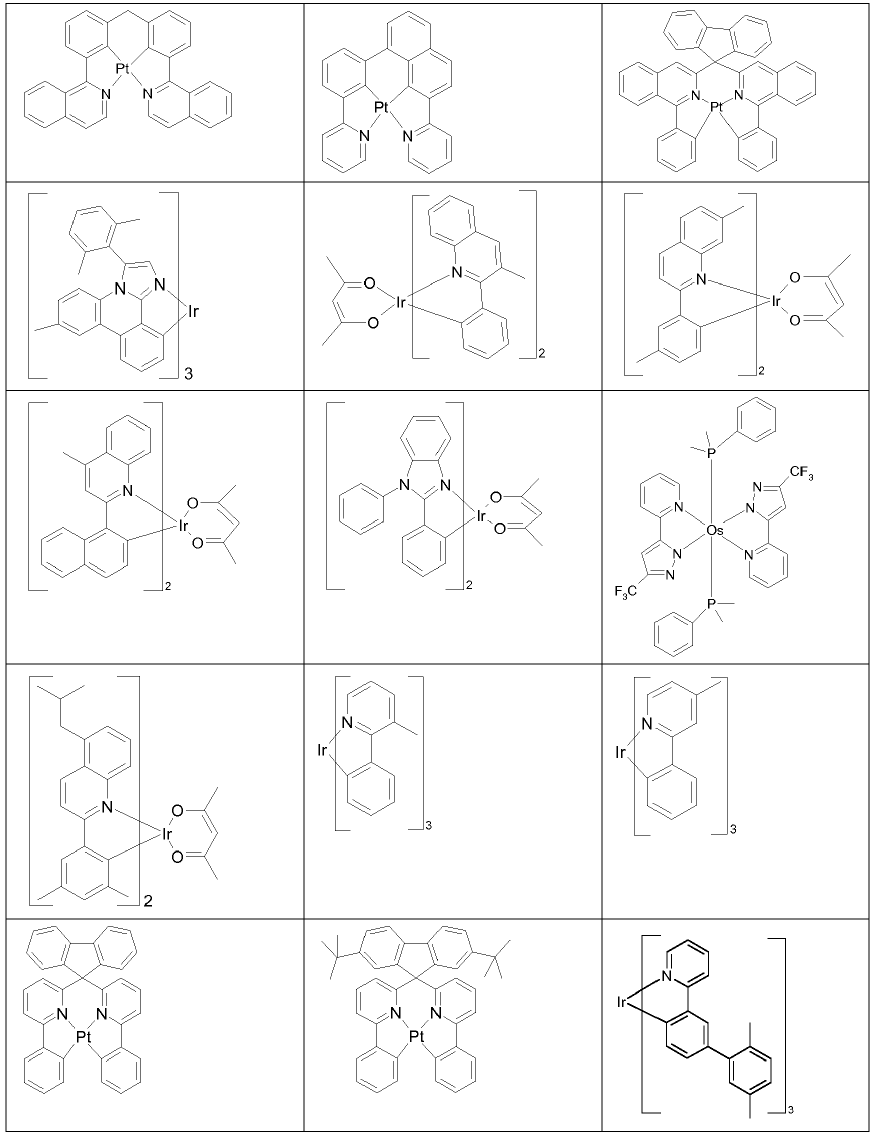

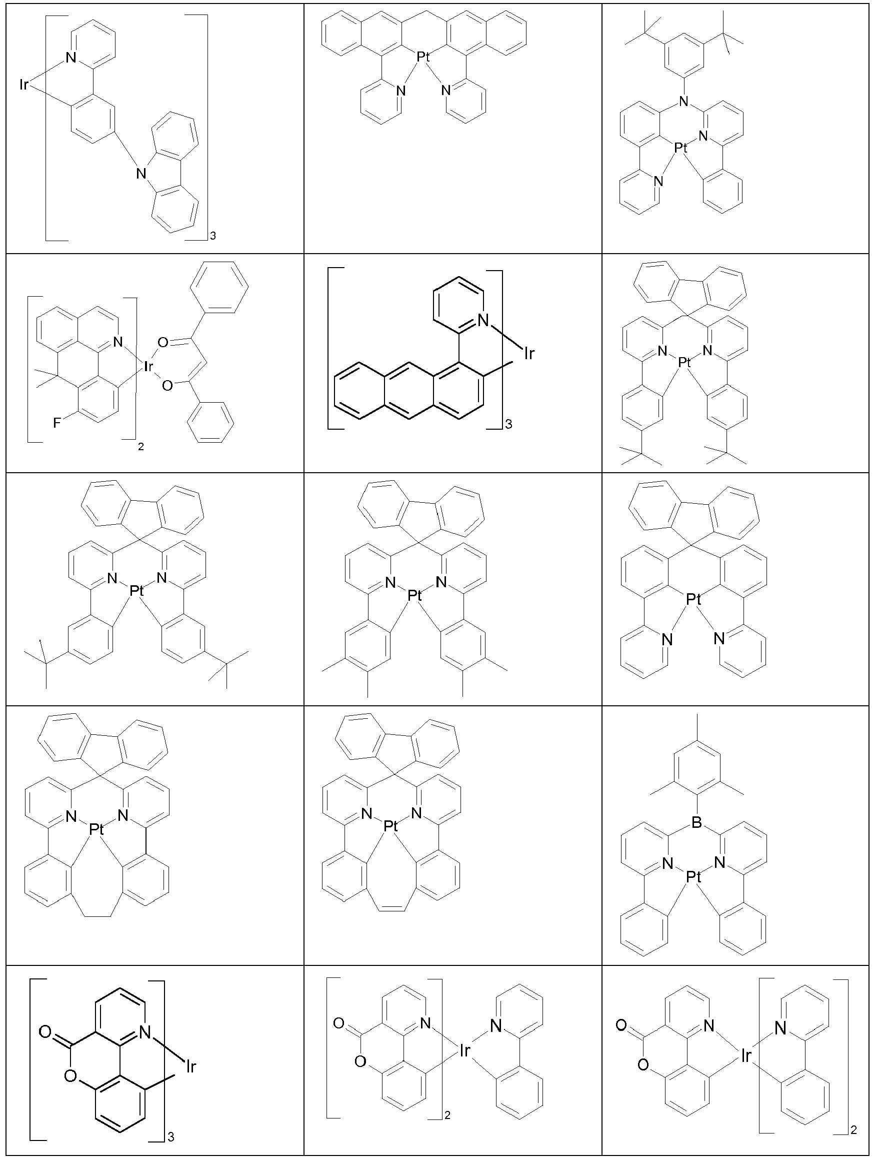

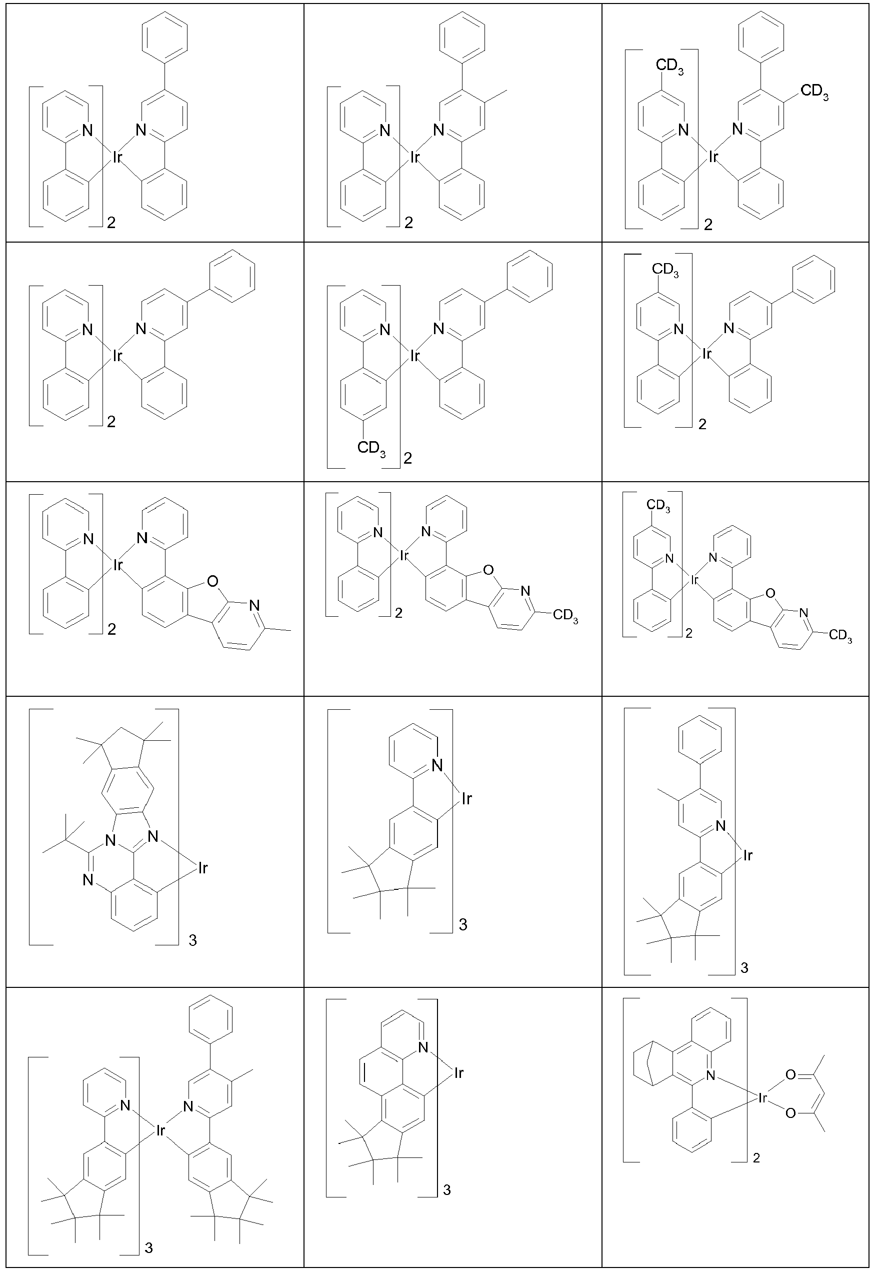

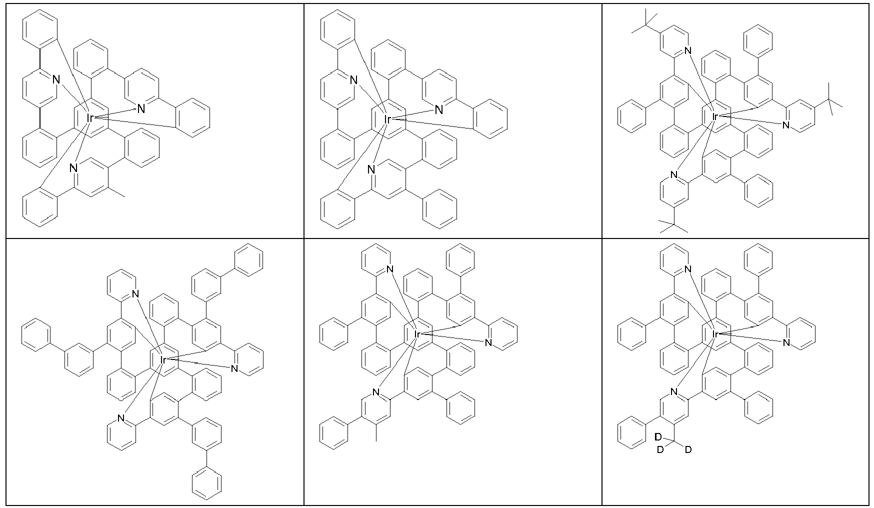

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu geringen Einsatz- und Betriebsspannungen. Dies gilt insbesondere, wenn die Verbindungen als Matrixmaterial für einen phosphoreszierenden Emitter eingesetzt werden.
3. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
4. Die erfindungsgemäßen Verbindungen könnten als blau fluoreszierende Emitter eingesetzt werden und führen in dieser Funktion zu hohen Effizienzen und Lebensdauern.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

### Synthesebeispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegebenen Nummern sind die entsprechenden CAS-Nummern.

### a) 1-(Benzofuran-3-yl)dibenzo[b,d]furan

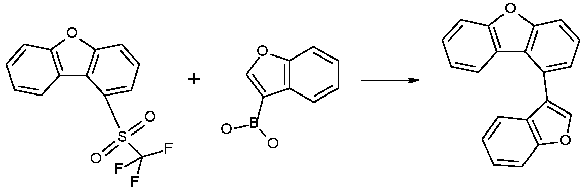

Unters Schutzatmosphäre werden 20 g (63.2 mmol) (Trifluormethyl)-sulfonyl)dibenzo[*b*,*d*]furan, 11.3 g (69.6 mmol) Benzofuran-3-ylboron-säure, 33.6 g (158.1 mmol) Kaliumphosphat, 0.3 g (1.3 mmol) Palladiumacetat und 1.2 g (2.5 mmol) XPhos in einem Gemisch aus 100 ml Wasser und 100 ml THF 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Das Öl wird mit Heptan chromatographisch gereinigt. Ausbeute: 15 g (52.8 mmol); 83% der Theorie.

### b )1-(2-Brombenzofuran-3-yl)dibenzo[b,d]furan

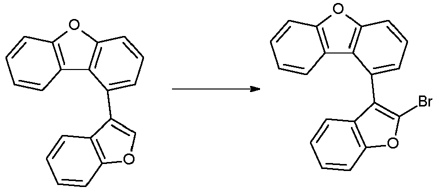

15 g (57 mmol) 1-(Benzofuran-3-yl)dibenzo[*b*,*d*]furan werden in 150 mL Dichlormethan gelöst. Zu dieser Lösung werden portionsweise 9.4 g (52.7 mmol) NBS zugegeben und 9 h in Dunkelheit gerührt. Danach wird mit Wasser/Eis versetzt, der Feststoff abgetrennt und mit Ethanol nachgewaschen. Der Rückstand wird über Alox filtriert. Ausbeute: 16.2 g (44 mmol); 84% der Theorie.

### c) ((3-(Dibenzo[b,d]furan-1-yl)benzofuran-2-yl)ethynyl)trimethylsilan

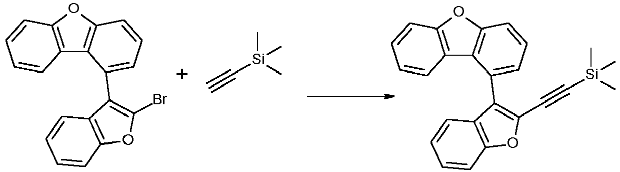

Unter Schutzatmosphäre werden 16 g (362 mmol) 1-(2-Brombenzofuran-3-yl)dibenzo[*b*,*d*]furan, 0.3 g (1.3 mmol) Kupferiodid, 0.6 g (0.9 mmol) Bis(triphenylphosphin)palladium(II)chlorid und 18.9 ml (133.8 mmol) Trimethylsilylacetylen mit 500 ml Triethylamin versetzt und über Nacht unter Rückfluss gekocht. Das Produkt wird chromatographisch mit Heptan gereinigt. Ausbeute: 13.6 g (35.7 mmol); 80% der Theorie.

### d) 1-(2-Ethynylbenzofuran-3-yl)dibenzo[b,d]furan

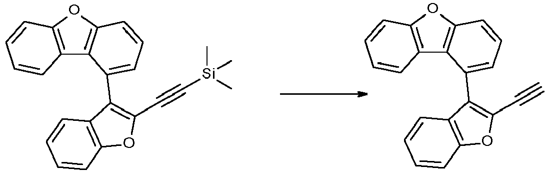

10 g (26.3 mmol) ((3-(Dibenzo[*b*,*d*]furan-1-yl)benzofuran-2-yl)ethynyl)-trimethylsilan, 0,7 g (5.3 mmol) Kaliumcarbonat werden in 100 ml Methanol 1 h unter Rückfluss gerührt. Das Lösungsmittel wird unter Vakuum entfernt. Die Mischung wird mit 100 ml Dichlormethan und 100 ml Wasser versetzt. Danach die organische Phase abgetrennt und anschließend eingeengt. Ausbeute: 8.1 g (26.3 mmol); 100 % der Theorie.

### e) Naphthobisbenzofuran

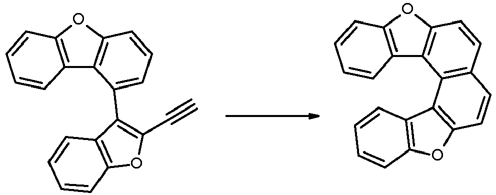

Unter Schutzatmosphäre werden 8.1 g (26 mmol) 1-(2-Ethynylbenzofuran-3-yl)dibenzo[*b*,*d*]furan und 690 mg (2.6 mmol) Dichlorplatinum in 500 ml Toluol über Nach unter Rückfluss gekocht. Das Produkt wird chromatographisch mit Toluol gereinigt. Ausbeute: 3.1 g (10 mmol); 38.7 % der Theorie.

### f) 1,8-Bis(2-methylsulfanylphenyl)naphthalin

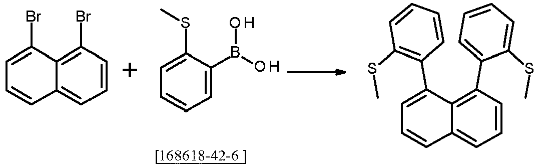

Eine gut gerührte, entgaste Suspension aus 5 g (17.4 mmol) 1,8-Dibromnaphthalin, 7 g (43.7 mmol) [2-(Methylsulfanylphenyl]boronsäure und 28 g (87 mmol) Caesiumcarbonat in einem Gemisch aus 200 ml Wasser und 200 ml N,N-Dimethylformamid wird mit 0.71 g (1.7 mmol) SPhos und 1.68 g (1.7 mmol) Pd₂(dba)₃ versetzt und 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet. Ausbeute: 5.9 g (15.9 mmol); 91 % der Theorie.

### g) 1,8-bis(2-Methylsulfinylphenyl)naphthalin

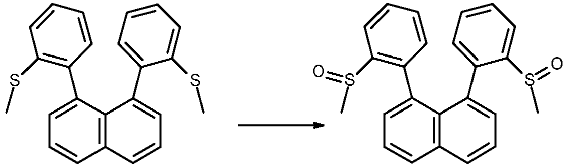

30 g (80 mmol) 1,8-Bis[2-methylsulfanylphenyl)naphthalin wird in 60 ml Eisessig vorgelegt und auf 0 °C gekühlt. Zu dieser Lösung werden 18.2 ml (160 mmol) einer 30 %igen H₂O₂-Lösung zugetropft und über Nacht gerührt. Das Gemisch wird mit Na₂SO₃ Lösung versetzt, die Phasen werden getrennt, und das Lösungsmittel wird im Vakuum entfernt. Ausbeute: 26 g (65 mmol); 80 % der Theorie; Reinheit: 92 % n. HPLC.

### h) Naphtho[2,1-b:7,8-b']bis[1]benzothiophen

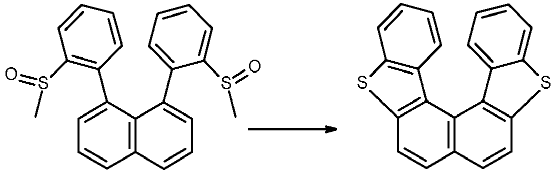

Eine Mischung aus 54 g (134 mmol) 1,8-Bis(2-methylsulfinylphenyl)-naphthalin und 737 ml Trifluormethansulfonsäure wird 48 h bei 5 °C gerührt. Anschließend wird die Mischung mit 2.4 L Wasser/Pyridin 5:1 versetzt und 20 min. unter Rückfluss erhitzt. Nach Abkühlen auf Raumtemperatur werden vorsichtig 500 ml Wasser und 1000 ml Dichlormethan zugesetzt. Die organische Phase wird mit 4 × 50 mL H₂O gewaschen, über MgSOa getrocknet und die Lösungsmittel im Vakuum entfernt. Das reine Produkt erhält man durch Umkristallisation. Ausbeute: 40 g (117 mmol); 80 % der Theorie; Reinheit: 96 % n. HPLC.

Analog dazu können folgende Verbindungen erhalten werden:

| | **Edukt 1** | **Produkt** | **Ausbeute** |
|---|---|---|---|
| h1 | 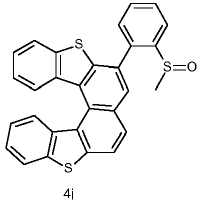 | 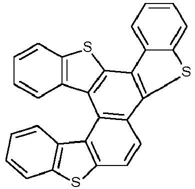 | 73% |
| h2 | 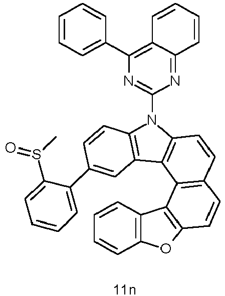 | 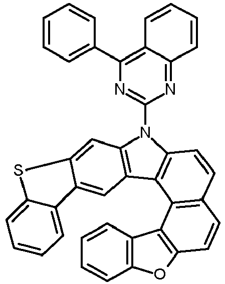 | 77% |
| h3 | 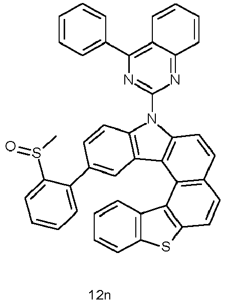 | 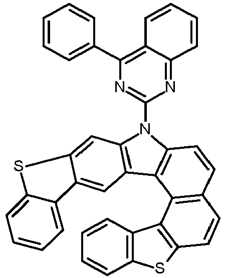 | 73% |
| h4 | 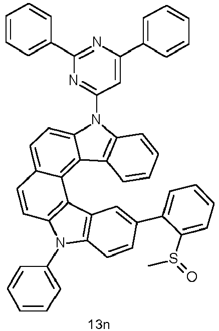 | 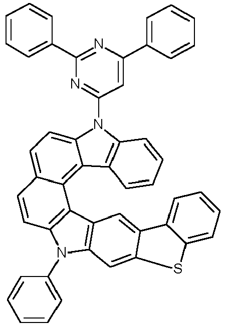 | 68% |
| h5 | 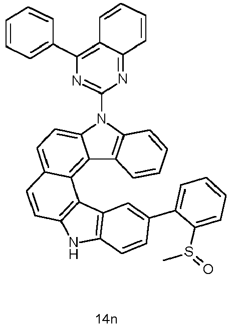 | 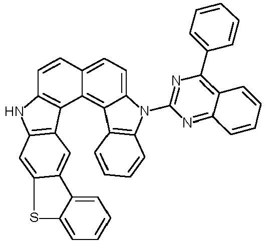 | 80% |
| h6 | 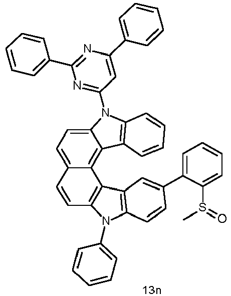 | 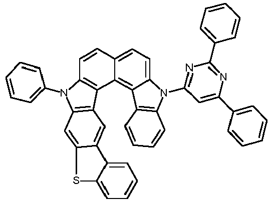 | 71% |
| h7 | 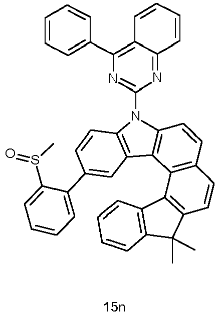 | 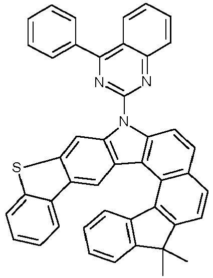 | 69% |

h1-h4 und h6-h7 werden mit Toluol heiß extrahiert, aus Toluol / n-Heptan umkristallisiert und abschließend im Hochvakuum sublimiert. Die Reinheit beträgt 99.9 %.

### i) Silylierung

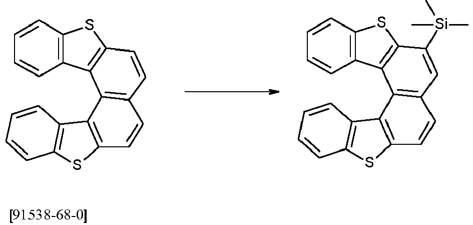

Eine auf 15 °C gekühlte Lösung von 82 g (240 mmol) Naphtho[2,1-b:7,8-b']bis[1]benzothiophen und 28.3 g (245 mmol) TMEDA in 1000 ml THF wird tropfenweise mit 138 ml (245 mmol) n-Buthyllithium (2.5 M in Hexan) versetzt. Die Reaktionsmischung wird 3 h bei Raumtemperatur gerührt, dann kühlt man auf 0 °C, tropft innerhalb 30 min. 27 g (251 mmol) Chlortrimethylsilan hinzu und rührt 16 h bei Raumtemperatur. Anschließend wird das Lösungsmittel im Vakuum entfernt und der Rückstand chromatographisch über Kieselgel mit Toluol:Dichlormethan 2:2 gereinigt. Ausbeute: 64 g (154 mmol); 65 % der Theorie.

Analog dazu kann die folgende Verbindung erhalten werden:

| | **Edukt** | **Produkt** | **Ausbeute** |
|---|---|---|---|
| 1i | 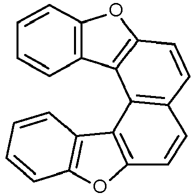 | 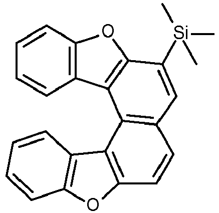 | 71% |

### j) Borylierung

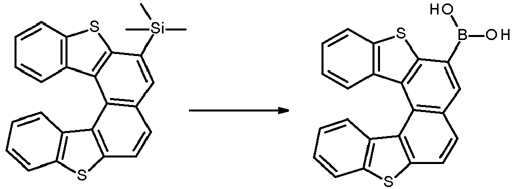

Unter Schutzgas wird eine Lösung von 10.3 g (26 mmol) Verbindung i in 100 mL Dichlormethan tropfenweise mit 7.8 g (31 mmol) Bortribromid versetzt und 10 h bei Raumtemperatur gerührt. Danach wird die Mischung langsam mit etwas Wasser versetzt und der ausgefallene Rückstand abfiltriert und mit Heptan gewaschen. Die Ausbeute beträgt 9.1 g (24 mmol), entsprechend 95 % der Theorie.

Analog dazu kann die folgende Verbindung erhalten werden:

| | **Edukt 1** | **Produkt** | **Ausbeute** |
|---|---|---|---|
| 1j | 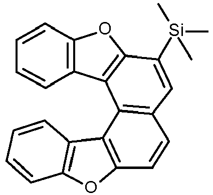 | 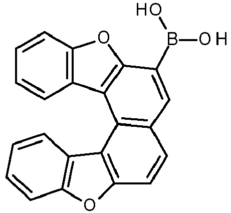 | 92% |

### k) lodierung

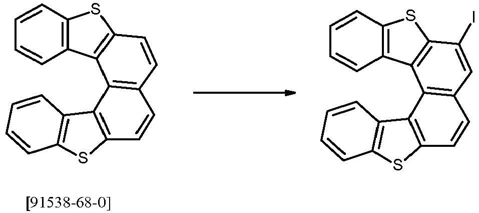

Eine auf 15 °C gekühlte Lösung von 82 g (240 mmol) Naphtho[2,1-b:7,8-b']bis[1]benzothiophen wird in 1000 ml THF wird tropfenweise bei -78 °C mit 138 ml (245 mmol) n-Butyllithium (2.5 M in Hexan) versetzt. Die Reaktionsmischung wird 3 h bei -78 °C gerührt. Dazu wird anschließend 95 g (377 mmol) lod gelöst in 400 ml THF langsam zugetropft. Man lässt auf Raumtemperatur kommen, rührt 2 h nach, vesetzt die Mischung mit 100 ml gesättigter Na₂SO₃-Lösung und 500 ml Essigsäureethylester, trennt die Phasen, entfernt das Lösungsmittel im Vakuum entfernt reinigt den Rückstand chromatographisch über Kieselgel mit Toluol:Dichlormethan 2:2. Ausbeute: 76 g (164 mmol); 68 % der Theorie.

Analog dazu können die folgenden Verbindungen erhalten werden:

| | **Edukt** | **Produkt** | **Ausbeute** |
|---|---|---|---|
| 1k | 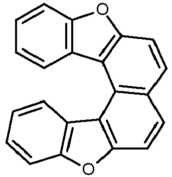 | 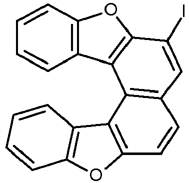 | 64% |
| 2k | 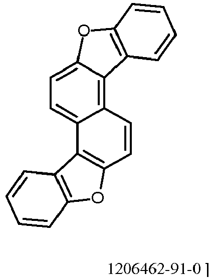 | 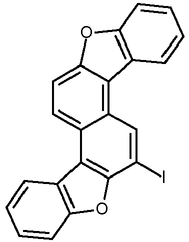 | 60% |
| 3k | 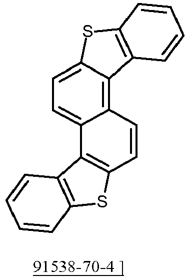 | 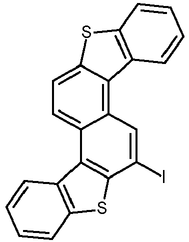 | 65% |
| 4k | 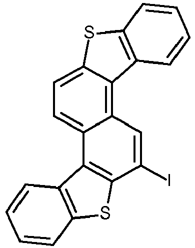 | 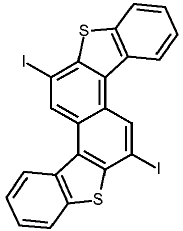 | 82% |

### l) Aminierung

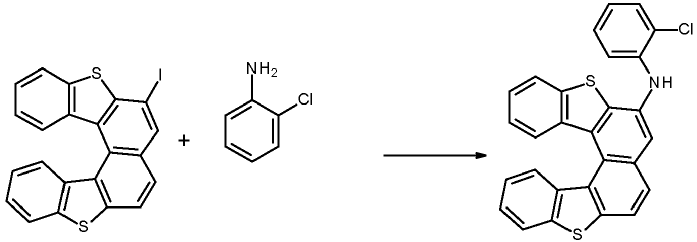

55.2 g (140 mmol) Verbindung **k**, 18.2 g (142 mmol) 2-Chloranilin, 68.2 g (710 mmol) Natrium-tert-butylat, 613 mg (3 mmol) Palladium(II)acetat und 3.03 g (5 mmol) dppf werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 44 g (96 mmol); 81% der Theorie.

Analog können folgende Verbindungen hergestellt werden:

| | **Edukt 1** | **Edukt 2** | **Produkt** | **Ausbeute** |
|---|---|---|---|---|
| 1l | 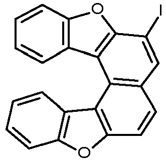 | 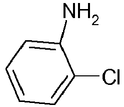 | 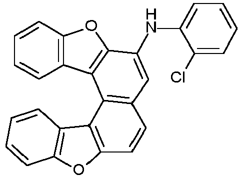 | 79% |
| 2l | 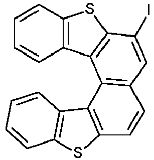 | 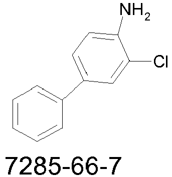 | 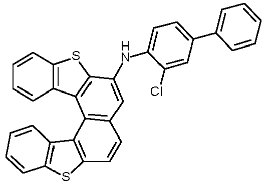 | 74% |
| 3l | 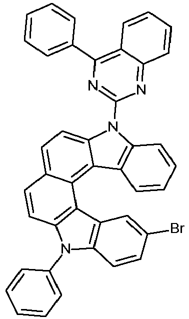 | 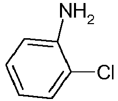 | 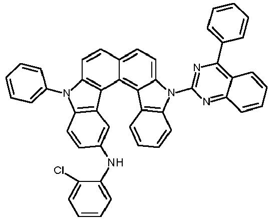 | 76% |
| 4l | 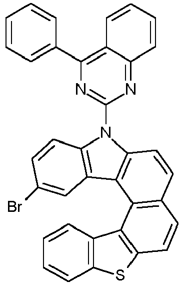 | 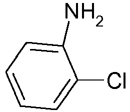 | 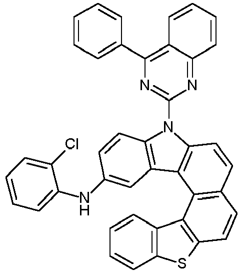 | 81% |
| 5l | 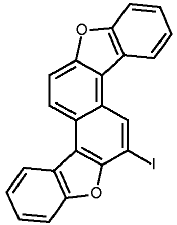 | 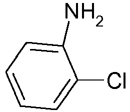 | 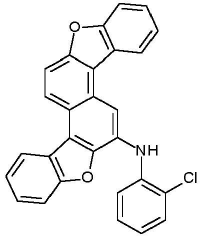 | 82% |
| 6l | 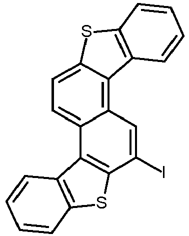 | 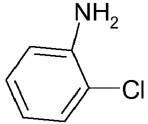 | 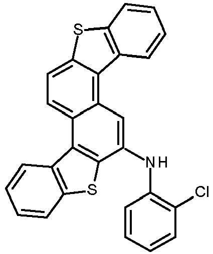 | 84% |
| 7l | 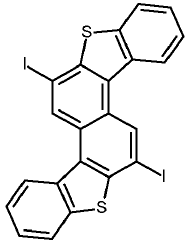 | 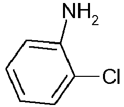 | 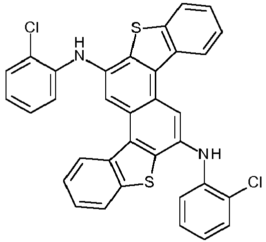 | 63% |

### m) Cyclisierung

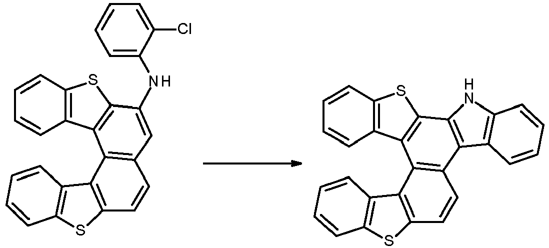

41.5 g (102 mmol) Verbindung c, 56 g (409 mmol) Kaliumcarbonat, 4.5 g (12 mmol) Tricyclohexylphosphintetrafluoroborat und 1.38 g (6 mmol) Palladium(II)acetat werden in 500 mL Dimethylacetamid suspendiert und 6 h unter Rückfluss gerührt. Nach Erkalten wird die Reaktionmischung mit 300 ml Wasser und 400 mL Dichlormethan versetzt. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert. Ausbeute: 27.5 g (64 mmol); 74 % der Theorie.

Analog können folgende Verbindungen hergestellt werden:

| | **Edukt** | **Produkt** | **Ausbeute** |
|---|---|---|---|
| 1m | 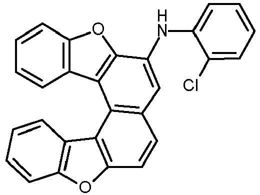 | 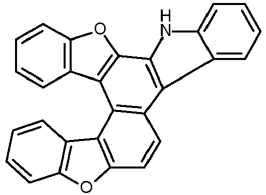 | 68% |
| 2m | 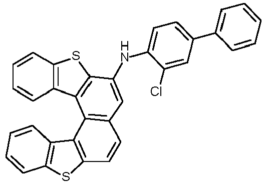 | 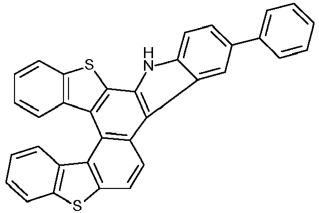 | 61% |
| 3m | 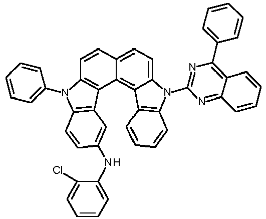 | 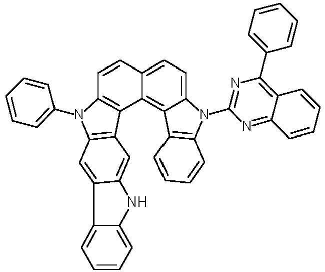 | 60% |
| 4m | 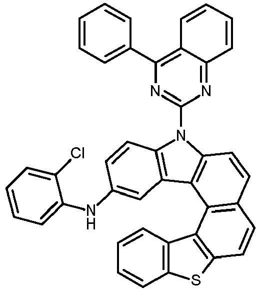 | 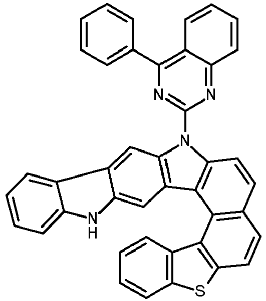 | 60% |
| 5m | 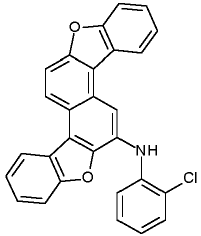 | 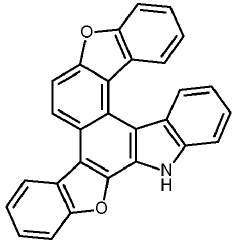 | 62% |
| 6m | 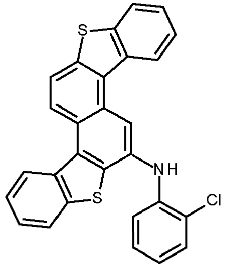 | 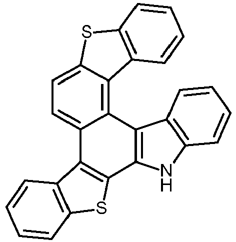 | 71% |
| 7m | 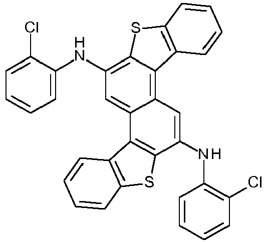 | 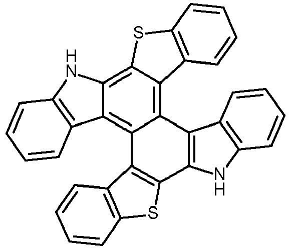 | 55% |

### n) Suzuki-Kupplung

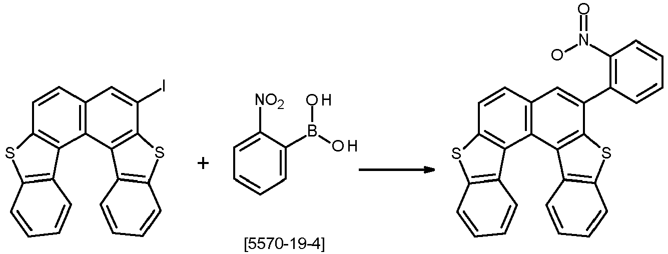

Eine gut gerührte, entgaste Suspension aus 43 g (92 mmol) Verbindung **d**, 153 g (92 mmol) 2-Nitrophenylboronsäure und 33.2 g (106 mmol) Kaliumcarbonat in einem Gemisch aus 200 ml Wasser und 200 ml THF wird mit 0.85 g (0.74 mmol) Pd(PPh₃)₄ versetzt und 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet. Ausbeute: 35.5 g (76.5 mmol); 91% der Theorie.

Analog können folgende Verbindungen hergestellt werden:

| | **Edukt 1** | **Edukt 2** | **Produkt** | **Ausbeute** |
|---|---|---|---|---|
| 1n | 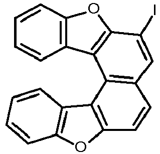 | 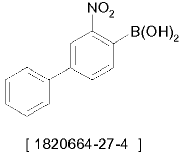 | 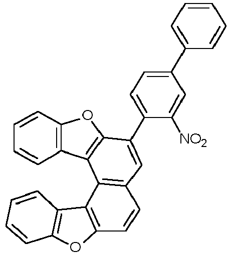 | 83% |
| 2n | 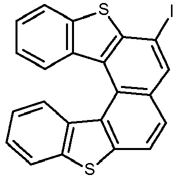 | 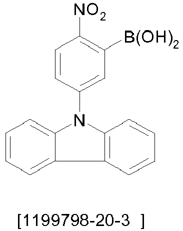 | 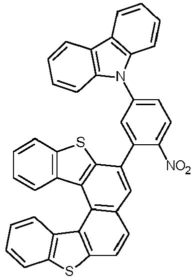 | 87% |
| 3n | 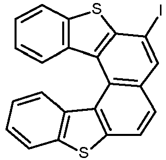 | 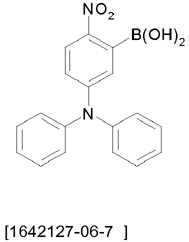 | 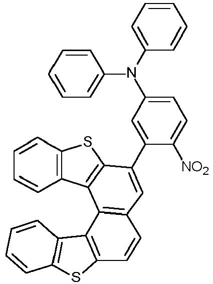 | 81% |
| 4n | 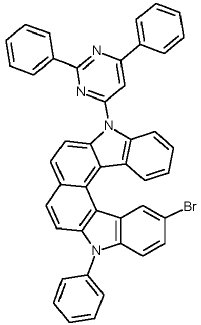 | 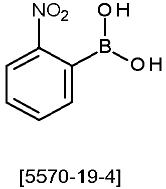 | 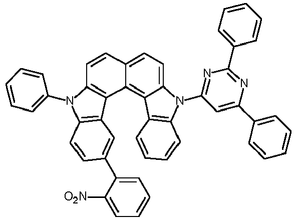 | 73% |
| 5n | 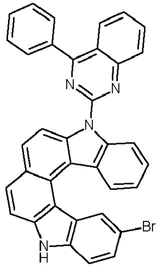 | 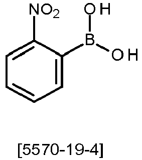 | 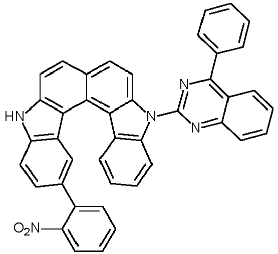 | 75% |
| 6n | 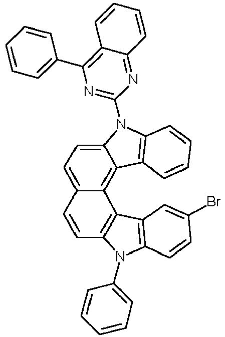 | 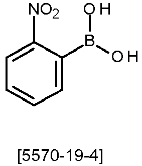 | 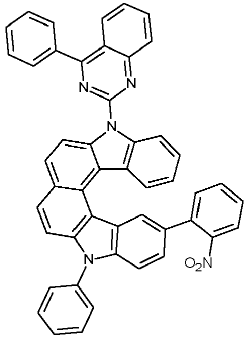 | 79% |
| 7n | 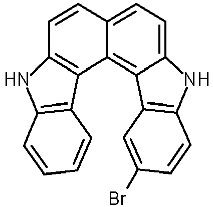 | 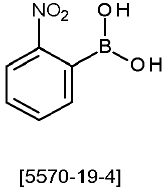 | 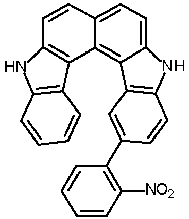 | 70% |
| 8n | 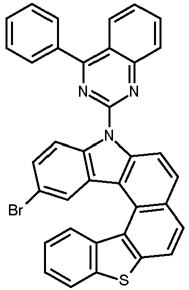 | 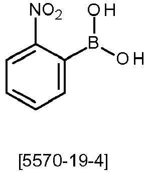 | 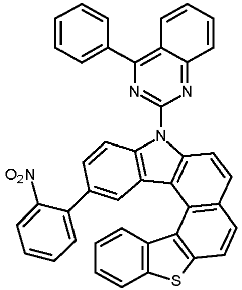 | 65% |
| 9n | 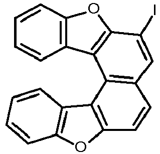 | 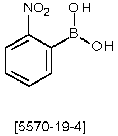 | 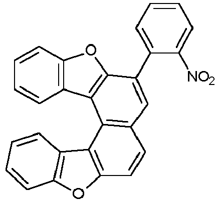 | 70% |
| 10n | 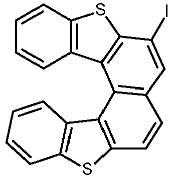 | 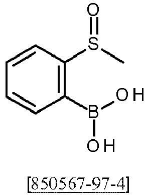 | 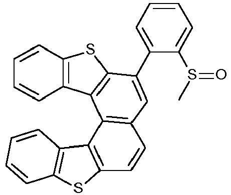 | 87% |
| 11n | 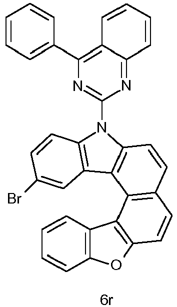 | 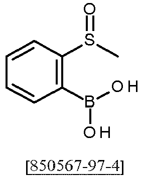 | 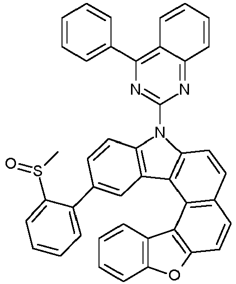 | 79% |
| 12n | 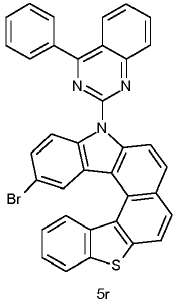 | 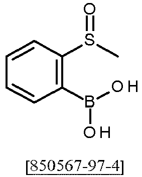 | 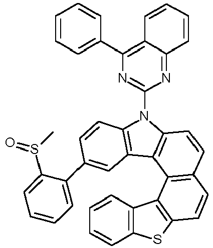 | 71% |
| 13n | 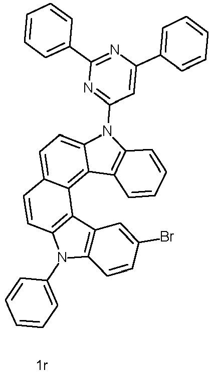 | 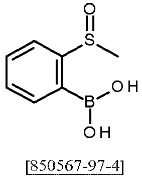 | 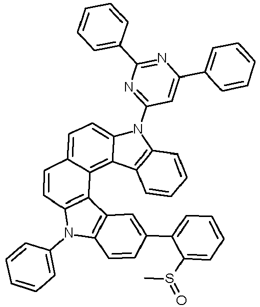 | 65% |
| 14n | 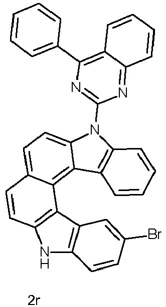 | 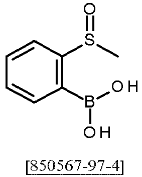 | 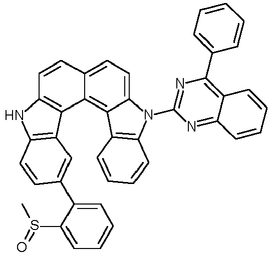 | 72% |
| 15n | 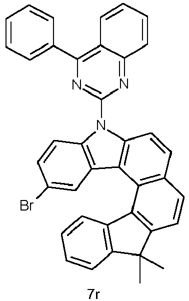 | 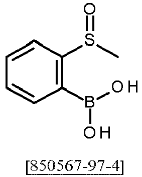 | 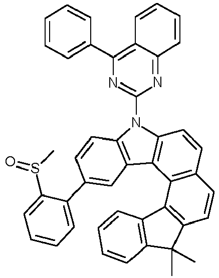 | 76% |

### o) Carbazolsynthese

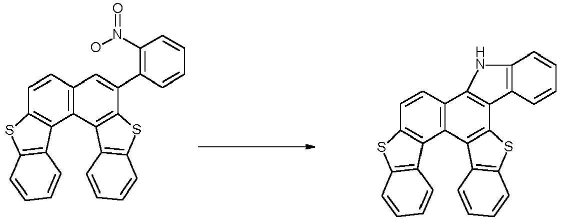

Eine Mischung aus 111 g (240 mmol) Verbindung n und 290.3 ml (1.7 mol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und umkristallisiert. Ausbeute: 75 g (175 mmol); 73 % der Theorie.

Analog können folgende Verbindungen hergestellt werden:

| | **Edukt** | **Produkt** | **Ausbeute** |
|---|---|---|---|
| 1o | 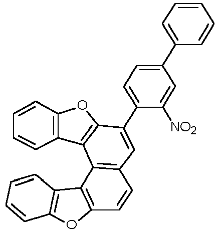 | 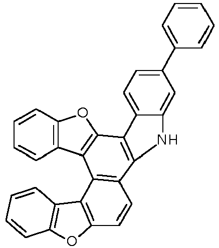 | 74% |
| 2o | 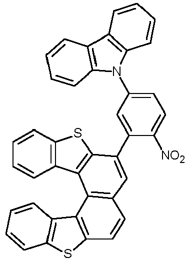 | 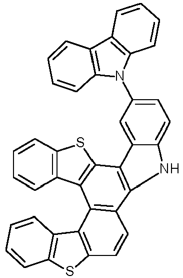 | 72% |
| 3o | 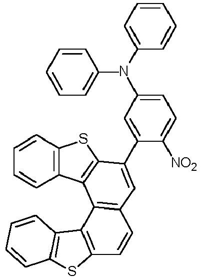 | 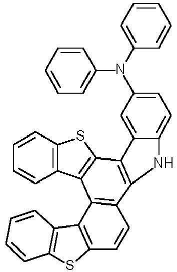 | 81% |
| 4o | 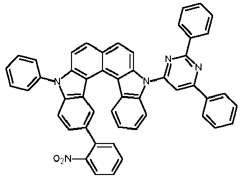 | 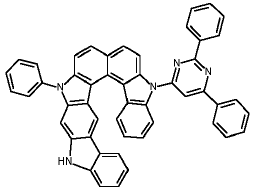 | 74% |
| 5o | 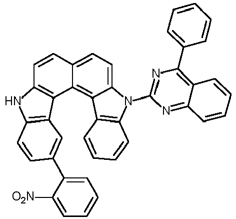 | 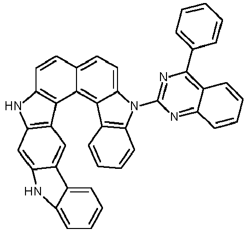 | 70% |
| 6o | 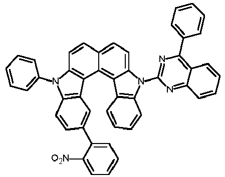 | 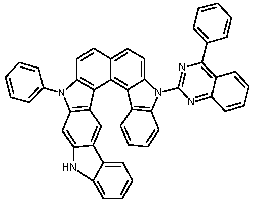 | 71% |
| 7o | 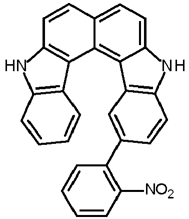 | 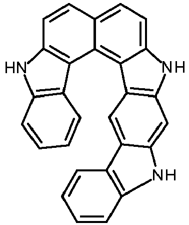 | 65% |
| 8o | 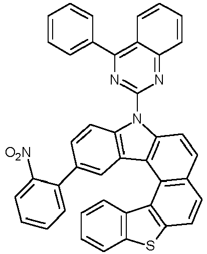 | 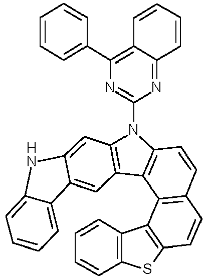 | 76% |
| 9o | 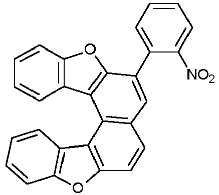 | 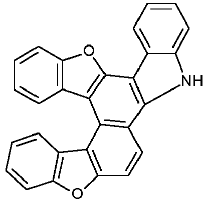 | 77% |

### p) Nukleophile Substitution

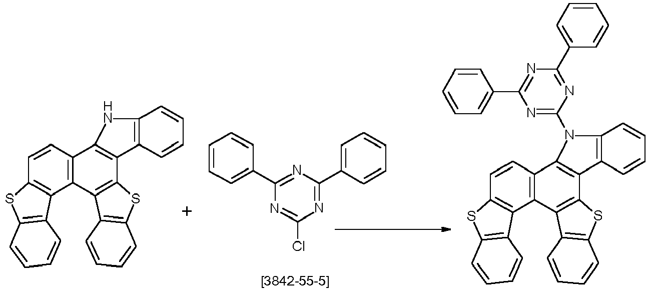

4.2 g NaH, 60%ig in Mineralöl (106 mmol) werden in 300 mL Dimethylformamid unter Schutzatmosphäre gelöst. 54.4 g (106 mmol) Verbindung **f** werden in 250 mL DMF gelöst und zu der Reaktionsmischung zugetropft. Nach 1 h bei Raumtemperatur wird eine Lösung von 2-Chlor-4,6-diphenyl-[1,3,5]triazin (34.5 g, 0.122 mol) in 200 mL THF zugetropft. Das Reaktionsgemisch wird 12 h bei Raumtemperatur gerührt und dann auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert, aus Toluol / n-Heptan umkristallisiert und abschließend im Hochvakuum sublimiert. Die Reinheit beträgt 99.9 %. Ausbeute: 44 g (66 mmol); 63 % der Theorie.

Analog können folgende Verbindungen hergestellt werden:

| | **Edukt 1** | **Edukt 2** | **Produkt** | **Ausbeute** |
|---|---|---|---|---|
| 1p | 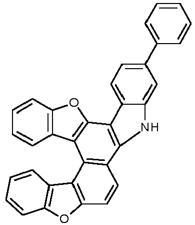 | 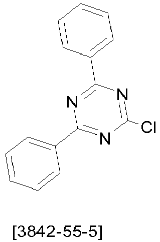 | 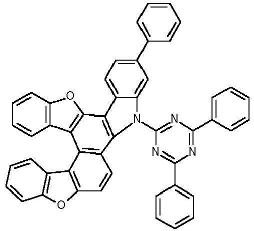 | 63% |
| 2p | 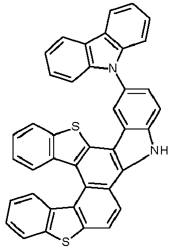 | 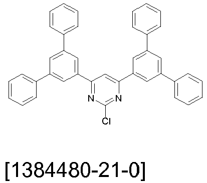 | 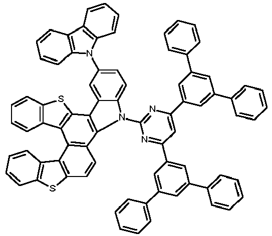 | 62% |
| 3p | 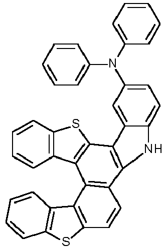 | 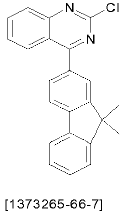 | 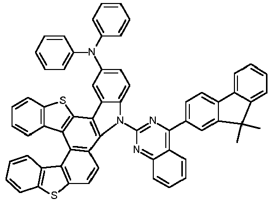 | 59% |
| 4p | 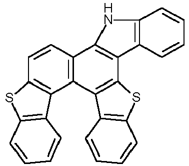 | 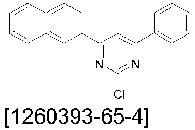 | 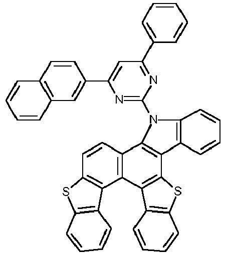 | 53% |
| 5p | 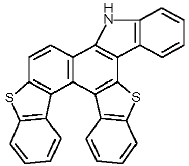 | 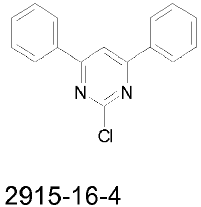 | 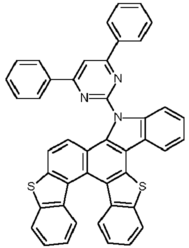 | 60% |
| 6p | 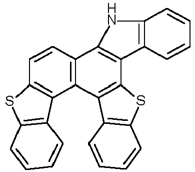 | 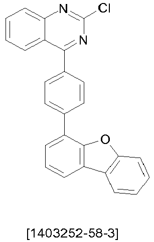 | 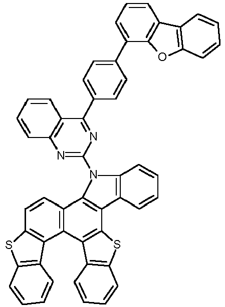 | 57% |
| 7p | 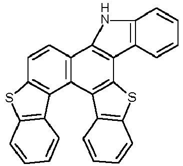 | 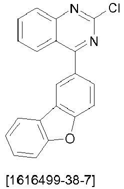 | 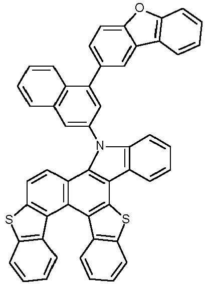 | 62% |
| 8p | 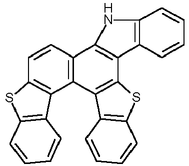 | 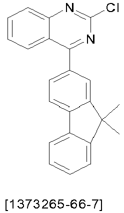 | 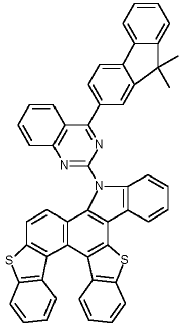 | 64% |
| 9p | 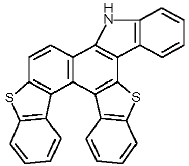 | 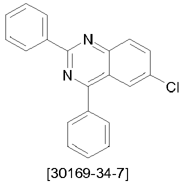 | 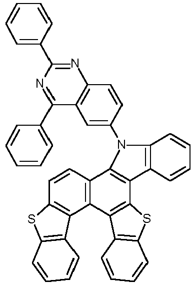 | 65% |
| 10p | 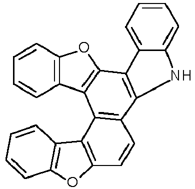 | 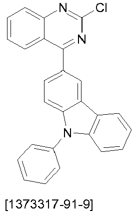 | 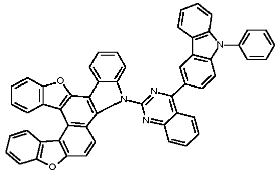 | 67% |
| 11p | 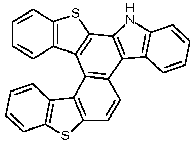 | 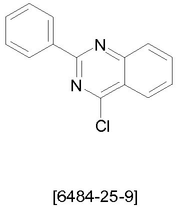 | 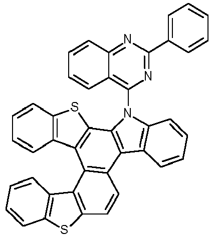 | 65% |
| 12p | 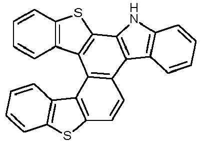 | 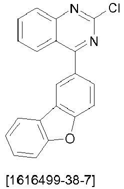 | 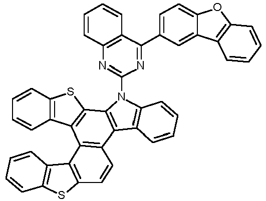 | |
| 13p | 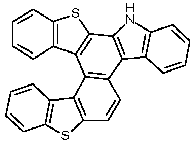 | 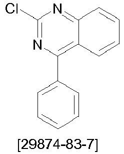 | 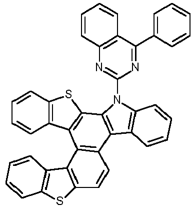 | 63% |
| 14p | 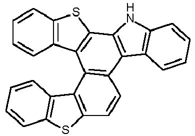 | 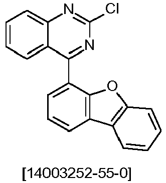 | 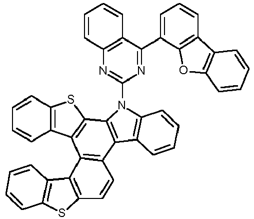 | 67% |
| 15g | 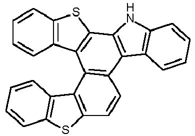 | 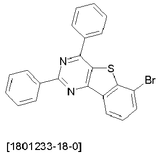 | 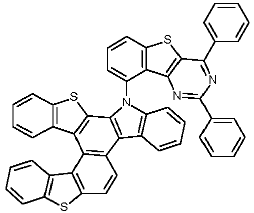 | 66% |
| 16p | 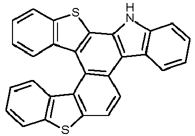 | 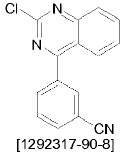 | 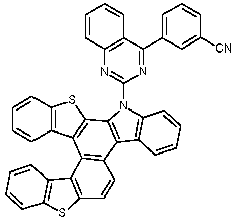 | 61% |
| 17p | 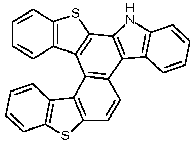 | 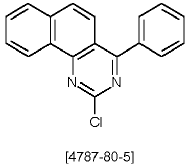 | 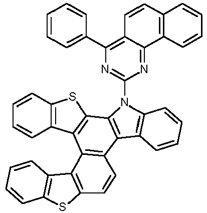 | 62% |
| 18p | 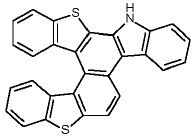 | 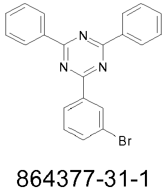 | 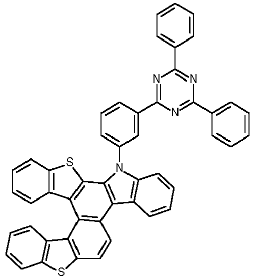 | 60% |
| 19p | 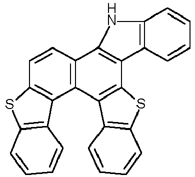 | 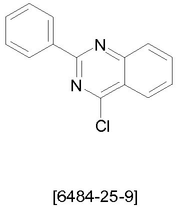 | 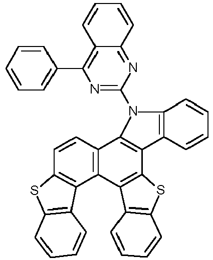 | 64% |
| 20p | 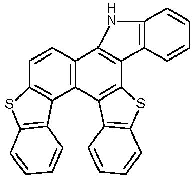 | 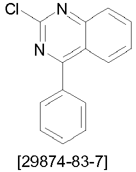 | 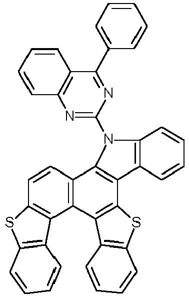 | 67% |
| 21p | 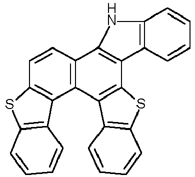 | 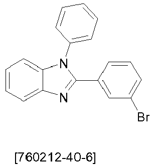 | 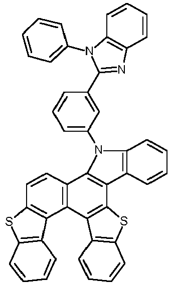 | 70% |
| 22p | 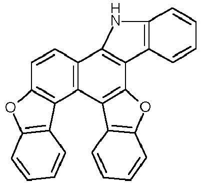 | 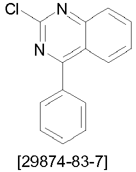 | 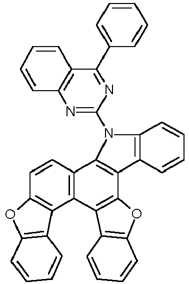 | 68% |
| 23p | 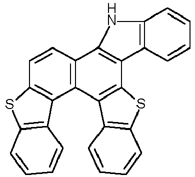 | 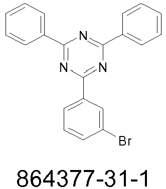 | 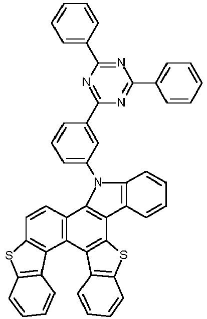 | 66% |
| 24p | 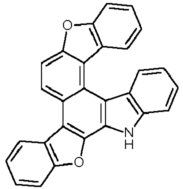 | 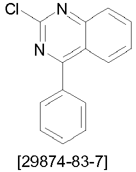 | 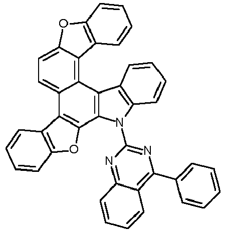 | 64% |
| 25p | 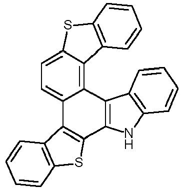 | 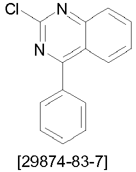 | 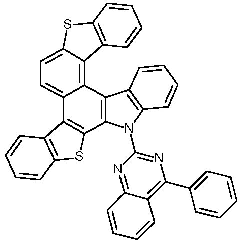 | 74% |
| 26p | 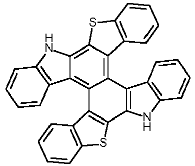 | 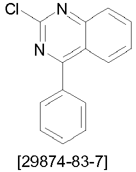 | 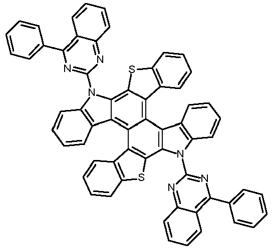 | 61% |
| 27p | 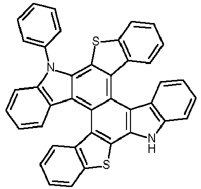 | 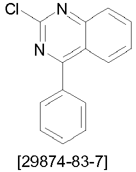 | 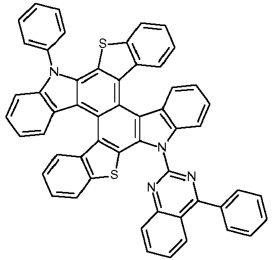 | 60% |
| 28p | 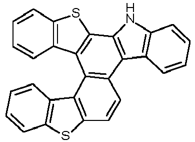 | 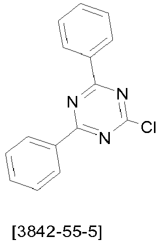 | 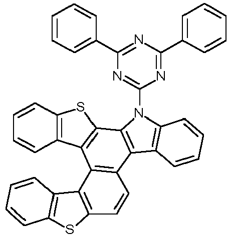 | 72% |

### q) Buchwald-Kupplung

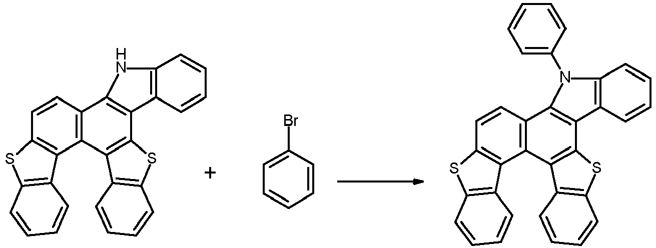

21.4 g (50 mmol) Verbindung **f** und 8.4 g (54 mmol) Brombenzol werden in 400 ml Toluol unter Argonatmosphäre geloest. 1.0 g (5 mmol) Tri-tert-butyl-phosphin werden zugegeben und unter Argonatmosphäre gerührt. 0.6 g (2 mmol) Pd(OAc)₂ werden zugegeben und unter Argonatmosphäre gerührt, wonach 9.5 g (99 mmol) Natium-tert-butanolat zugegeben werden. Das Reaktionsgemisch wird unter Rückfluss 24 h gerührt. Nach dem Abkühlen wird die organische Phase getrennt, dreimal mit 200 ml Wasser gewaschen, über MgSOa getrocknet, filtriert und das Lösungsmittel im Vakuum entfernt. Der Rückstand wird durch Säulenchromatographie über Kieselgel (Eluent: DCM/Heptan (1:3)) gereinigt, mit Toluol heiß extrahiert, aus Toluol / n-Heptan umkristallisiert und abschließend im Hochvakuum sublimiert. Ausbeute: 22.7 g (45 mmol); 90 % der Theorie.

Analog werden die folgenden Verbindungen erhalten:

| | **Edukt 1** | **Edukt 2** | **Produkt** | **Ausbeute** |
|---|---|---|---|---|
| 1q | 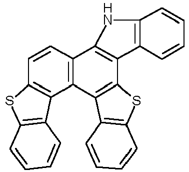 | 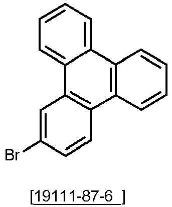 | 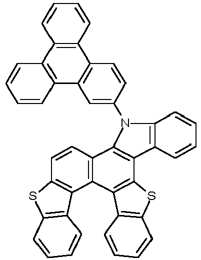 | 70% |
| 2q | 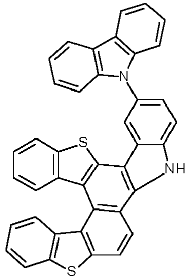 | 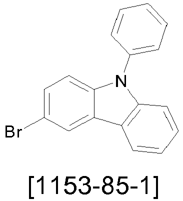 | 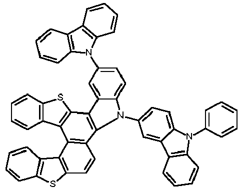 | 73% |
| 3q | 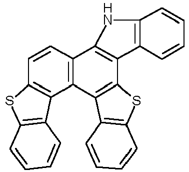 | 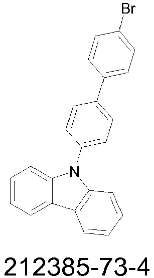 | 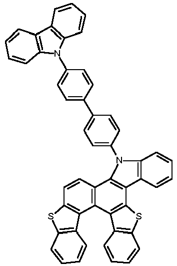 | 75% |
| 4q | 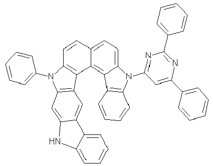 | 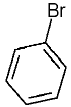 | 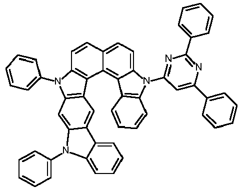 | 76% |
| 5q | 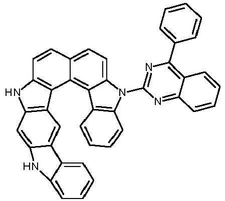 | 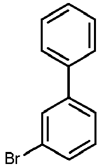 | 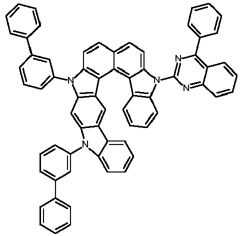 | 78% |
| 6q | 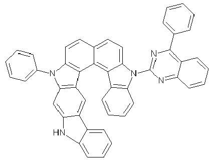 | 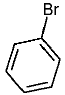 | 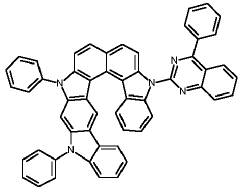 | 70% |
| 7q | 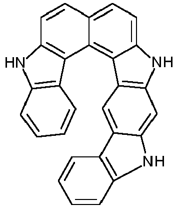 | 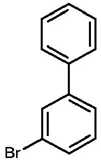 | 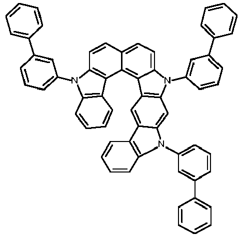 | 73% |
| 8q | 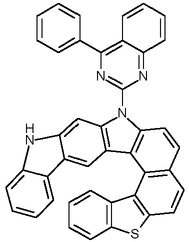 | 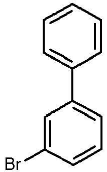 | 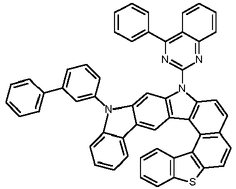 | 75% |
| 9q | 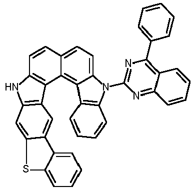 | 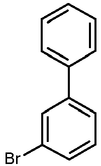 | 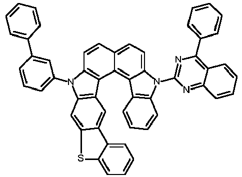 | 70% |
| 10q | 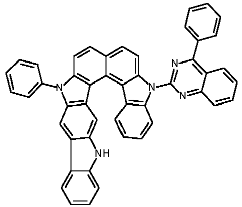 | 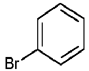 | 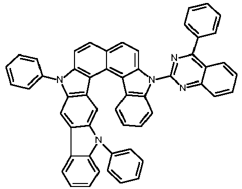 | 77% |
| 11q | 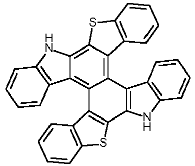 | 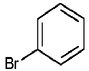 | 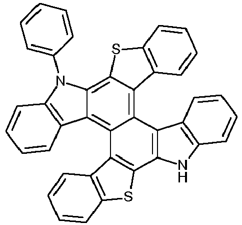 | 51% |

### r) Bromierung

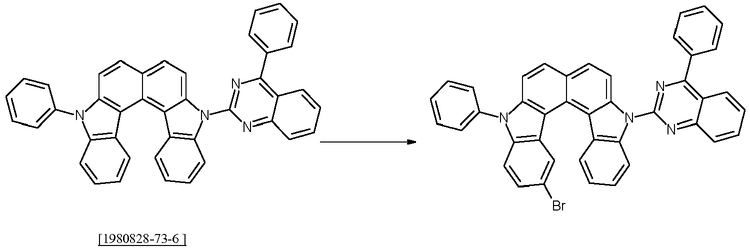

111.3 g (190 mmol) 5,10-Dihydro-5-phenyl-10-(4-phenyl-2-chinazolinyl)-carbazolo[3,4-c]carbazol werden in 1800 mL DMF suspendiert. Zu dieser Suspension werden portionsweise 34 g (190 mmol) NBS zugegeben und 2 h in Dunkelheit gerührt. Danach wird mit Wasser/Eis versetzt, der Feststoff abgetrennt und mit Ethanol nachgewaschen. Die Isomere werden durch Umkristallisation getrennt. Die Ausbeute beträgt 82 g (123 mmol); 65 % der Theorie.

Analog dazu werden die folgenden Verbindungen hergestellt:

| | **Edukt** | **Produkt** | **Ausbeute** |
|---|---|---|---|
| 1r | 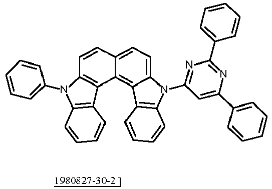 | 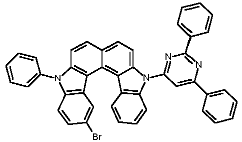 | 54% |
| 2r | 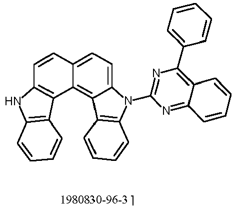 | 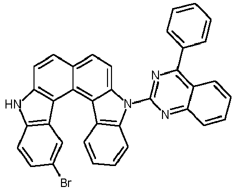 | 52% |
| 3r | 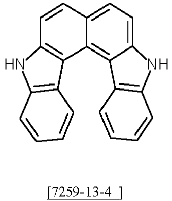 | 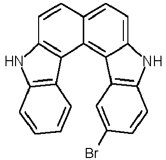 | 77% |
| 4r | 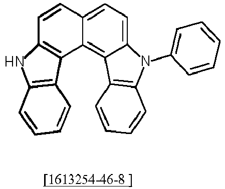 | 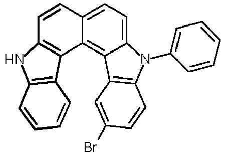 | 41% |
| 5r | 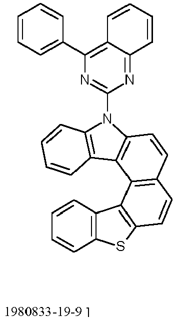 | 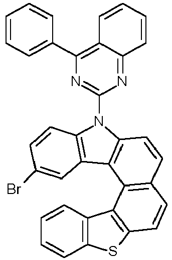 | 55% |
| 6r | 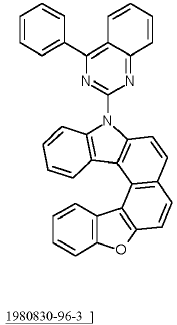 | 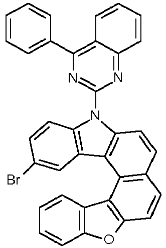 | 67% |
| 7r | 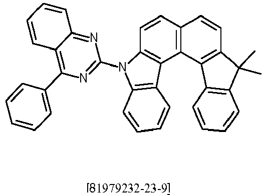 | 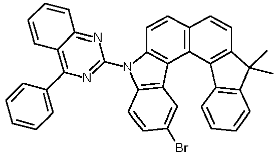 | 56% |

### Herstellung der OLEDs

In den folgenden Beispielen E1 bis E11 (siehe Tabelle 1) wird der Einsatz der erfindungsgemäßen Materialien in OLEDs vorgestellt.

**Vorbehandlung für die Beispiele E1-E11:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt. Die Daten der OLEDs sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie EG1:IC2:TER5 (55%:35%:10%) bedeutet hierbei, dass das Material EG1 in einem Volumenanteil von 55%, IC2 in einem Volumenanteil von 35% und TER5 in einem Volumenanteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Strom-Spannungs-Kennlinien sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 3 bezeichnet die Spannung, die für eine Leuchtdichte von 1000 cd/m² benötigt wird. Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=95% inTabelle 3 bedeutet, dass die in Spalte LD angegebene Lebensdauer der Zeit entspricht, nach der die Leuchtdichte auf 95% ihres Anfangswertes absinkt.

### Verwendung der erfindungsgemäßen Materialien in OLEDs

Die erfindungsgemäßen Materialien EG1 bis EG7 werden in den Beispielen E1 bis E11 als Matrixmaterial in der Emissionsschicht von phosphoreszierenden roten OLEDs eingesetzt. Die besonderen Vorteile dieser Verbindungen sind die vergleichbar geringe Einsatzspannung, sowie eine lange Lebensdauer, die in einer Mischung aus einem erfindungsgemäßen Material und einer Co-Matrix, im vorliegenden Fall in einer Mischung aus EG1 und IC3, nochmal deutlich verlängert werden kann.

**Tabelle 1: Aufbau der OLEDs**

| Bsp | HIL | HTL | EBL | EML | HBL | ETL | EIL |
|---|---|---|---|---|---|---|---|
| | Dicke | Dicke | Dicke | Dicke | Dicke | Dicke | Dicke |
| E1 | HATCN | SpMA1 | SpMA3 | EG1:TER5 | ST2 | ST2:LiQ (50%:50%) | LiQ |
| | 5nm | 125nm | 10nm | (97%:3%) 35nm | 10nm | 30nm | 1nm |
| E2 | HATCN | SpMA1 | SpMA3 | EG1:IC2:TER5 | ST2 | ST2:LiQ (50%:50%) | LiQ |
| | 5nm | 125nm | 10nm | (72%:25%:3%) 35nm | 10nm | 30nm | 1nm |
| E3 | HATCN | SpMA1 | SpMA3 | EG1:IC3:TER5 | ST2 | ST2:LiQ (50%:50%) | LiQ |
| | 5nm | 125nm | 10nm | (72%:25%:3%) 35nm | 10nm | 30nm | 1nm |
| E4 | HATCN | SpMA1 | SpMA3 | EG2:IC3:TER5 | ST2 | ST2:LiQ (50%:50%) | LiQ |
| | 5nm | 125nm | 10nm | (72%:25%:3%) 35nm | 10nm | 30nm | 1nm |
| E5 | HATCN | SpMA1 | SpMA3 | EG3:IC3:TER5 | ST2 | ST2:LiQ (50%:50%) | LiQ |
| | 5nm | 125nm | 10nm | (72%:25%:3%) 35nm | 10nm | 30nm | 1nm |
| E6 | HATCN | SpMA1 | SpMA3 | EG4:IC3:TER5 | ST2 | ST2:LiQ (50%:50%) | LiQ |
| | 5nm | 125nm | 10nm | (72%:25%:3%) 35nm | 10nm | 30nm | 1nm |
| E7 | HATCN | SpMA1 | SpMA3 | EG5:IC3:TER5 | ST2 | ST2:LiQ (50%:50%) | LiQ |
| | 5nm | 125nm | 10nm | (72%:25%:3%) 35nm | 10nm | 30nm | 1nm |
| E8 | HATCN | SpMA1 | SpMA3 | EG6:TER5 | ST2 | ST2:LiQ (50%:50%) | LiQ |
| | 5nm | 125nm | 10nm | (97%:3%) 35nm | 10nm | 30nm | 1nm |
| E9 | HATCN | SpMA1 | SpMA3 | EG6:IC2:TER5 | ST2 | ST2:LiQ (50%:50%) | LiQ |
| | 5nm | 125nm | 10nm | (72%:25%:3%) 35nm | 10nm | 30nm | 1nm |
| E20 | HATCN | SpMA1 | SpMA3 | EG7:TER5 | ST2 | ST2:LiQ (50%:50%) | LiQ |
| | 5nm | 125nm | 10nm | (97%:3%) 35nm | 10nm | 30nm | 1nm |
| E11 | HATCN | SpMA1 | SpMA3 | EG7:IC2:TER5 | ST2 | ST2:LiQ (50%:50%) | LiQ |
| | 5nm | 125nm | 10nm | (72%:25%:3%) 35nm | 10nm | 30nm | 1nm |

**Tabelle 2: Strukturformeln der Materialien für die OLEDs**

| | |
|---|---|
| 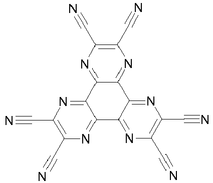 | 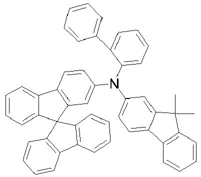 |
| HATCN | SpMA1 |
| 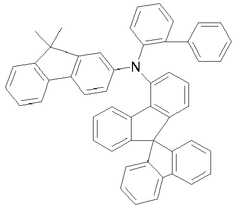 | 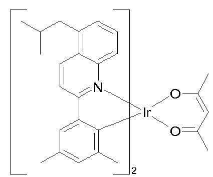 |
| SpMA3 | TER5 |
| 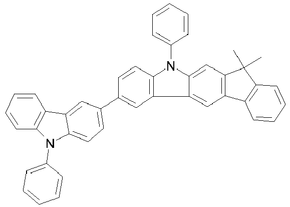 | 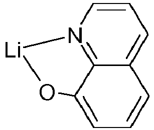 |
| IC2 | LiQ |
| 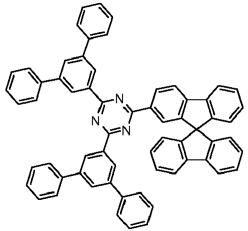 | 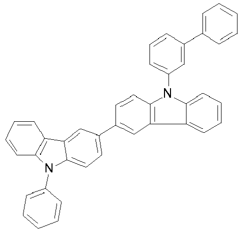 |
| ST2 | IC3 |
| 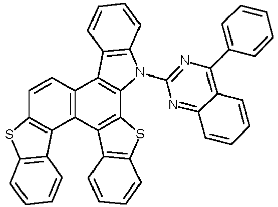 | 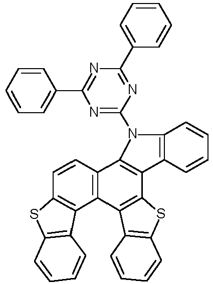 |
| EG1 | EG2 |
| 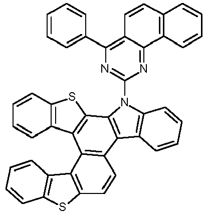 | 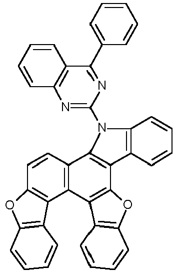 |
| EG3 | EG4 |
| 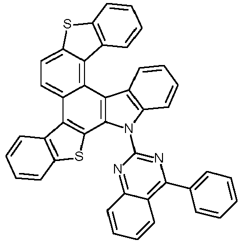 | 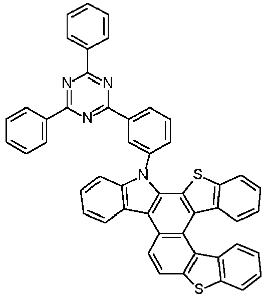 |
| EG5 | EG6 |
| 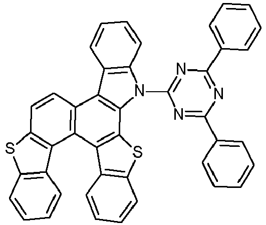 | |
| EG7 | |

**Tabelle 3: Daten der OLEDs**

| Bsp. | U1000 | CIE x/y bei | j₀ | EQE 1000 | L1 | LD |
|---|---|---|---|---|---|---|
| | (V) | 1000 cd/m² | (mA/cm²) | % | (%) | (h) |
| E1 | 3.3 | 0.67/0.33 | 20 | 19 | 95 | 520 |
| E2 | 3.5 | 0.67/0.34 | 20 | 20 | 95 | 410 |
| E3 | 3.4 | 0.67/0.33 | 20 | 21.2 | 95 | 375 |
| E4 | 3.3 | 0.67/0.34 | 20 | 21.1 | 95 | 400 |
| E5 | 3.3 | 0.67/0.33 | 20 | 20.5 | 95 | 337 |
| E6 | 3.4 | 0.67/0.33 | 20 | 20.3 | 95 | 391 |
| E7 | 3.3 | 0.67/0.33 | 20 | 20.1 | 95 | 430 |
| E8 | 3.3 | 0.67/0.33 | 20 | 24.0 | 95 | 220 |
| E9 | 3.4 | 0.67/0.33 | 20 | 25.6 | 95 | 230 |
| E10 | 3.1 | 0.67/0.33 | 20 | 22.7 | 95 | 300 |
| E11 | 3.2 | 0.67/0.33 | 20 | 26.3 | 95 | 490 |

Mit den weiteren in den Synthesebeispielen beschriebenen Verbindungen werden in einem Device mit einem analogen Aufbau zu den Beispielen E1 und E2 vergleichbare Ergebnisse erhalten.

## Patentansprüche

1. Verbindung gemäß Formel (1), 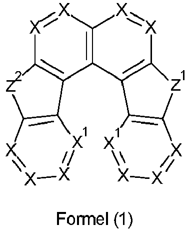 wobei für die verwendeten Symbole gilt:
X ist bei jedem Auftreten gleich oder verschieden CR oder N oder zwei benachbarte X stehen für eine Gruppe der folgenden Formel (2), 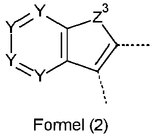 wobei die gestrichelten Bindungen die Verknüpfung dieser Gruppe in der Formel (1) kennzeichnen, mit der Maßgabe, dass die Verbindung der Formel (1) eine oder zwei Gruppen der Formel (2) enthält;
X¹ ist CH;
Y ist bei jedem Auftreten gleich oder verschieden CR oder N;
Z¹, Z², Z³ sind bei jedem Auftreten gleich oder verschieden O, S, N-Ar oder CR₂;
Ar ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann;
R ist bei jedem Auftreten gleich oder verschieden H, D, F, N(Ar')₂, CN, OR¹, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl- oder Alkenylgruppe jeweils mit einem oder mehreren Resten R¹ substituiert sein kann, wobei eine oder mehrere nicht benachbarte CH₂-Gruppen durch O ersetzt sein können, oder ein aromatisches oder heteroaromatisches Ringsystem mit 6 bis 30 aromatischen Ring-atomen, das jeweils durch einen oder mehrere Reste R¹ substituiert sein kann; dabei können zwei Reste R auch miteinander ein aliphatisches Ringsystem bilden;
Ar' ist bei jedem Auftreten gleich oder verschieden ein aromatisches oder heteroaromatisches Ringsystem mit 6 bis 30 aromatischen Ringatomen, das durch einen oder mehrere Reste R¹ substituiert sein kann;
R¹ ist bei jedem Auftreten gleich oder verschieden H, D, F, CN, OR², eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl- oder Alkenylgruppe jeweils mit einem oder mehreren Resten R² substituiert sein kann, wobei eine oder mehrere nicht benachbarte CH₂-Gruppen durch O ersetzt sein können, oder ein aromatisches oder heteroaromatisches Ringsystem mit 6 bis 30 aromatischen Ring-atomen, das jeweils durch einen oder mehrere Reste R² substituiert sein kann; dabei können zwei oder mehrere Reste R¹ miteinander ein aliphatisches Ringsystem bilden;
R² ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können.

2. Verbindung nach Anspruch 1, ausgewählt aus den Verbindungen der Formeln (3) bis (8), 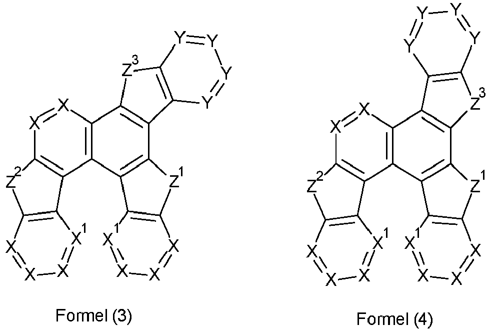 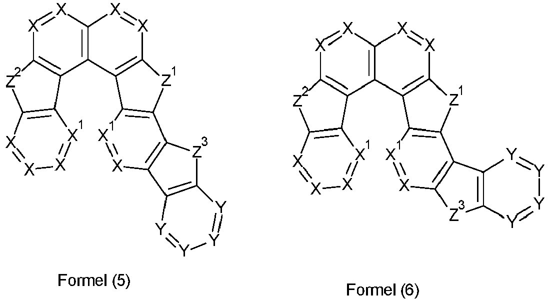 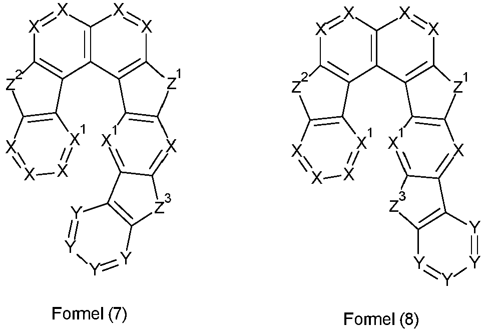 wobei die verwendeten Symbole die in Anspruch 1 genannten Bedeutungen aufweisen.

3. Verbindung nach Anspruch 1 oder 2, **dadurch gekennzeichnet, dass** die Verbindung genau eine Gruppe der Formel (2) enthält.

4. Verbindung nach einem oder mehreren der Ansprüche 1 bis 3, **dadurch gekennzeichnet, dass** die Gruppe der Formel (2) für eine Gruppe der Formel (2a) steht, 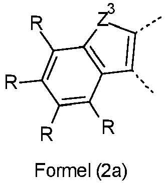 wobei die verwendeten Symbole die in Anspruch 1 genannten Bedeutungen aufweisen.

5. Verbindung nach einem oder mehreren der Ansprüche 1 bis 4, ausgewählt aus den Strukturen der Formeln (3a) bis (8a), 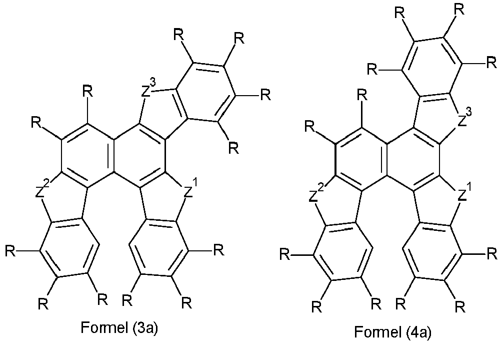 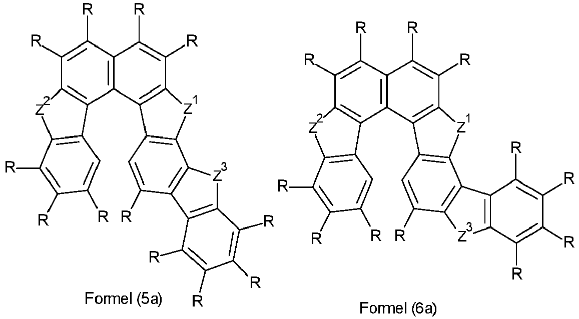 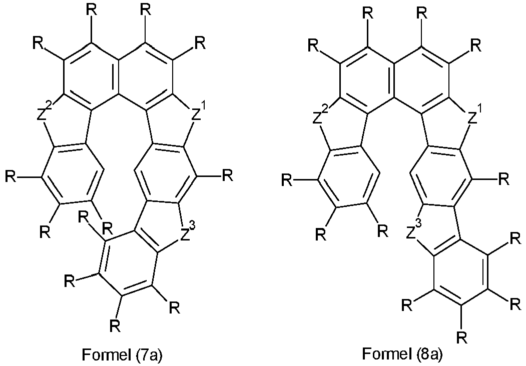 wobei die verwendeten Symbole die in Anspruch 1 genannten Bedeutungen aufweisen.

6. Verbindung nach einem oder mehreren der Ansprüche 1 bis 5, ausgewählt aus den Strukturen der Formeln (3b) bis (8b), 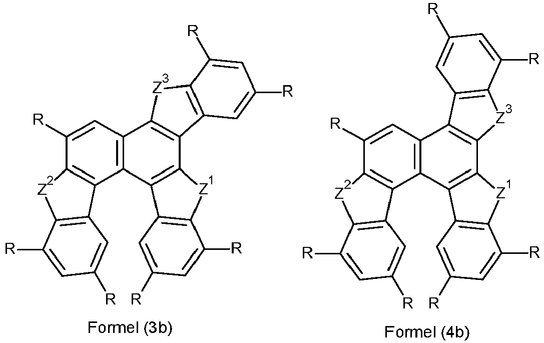 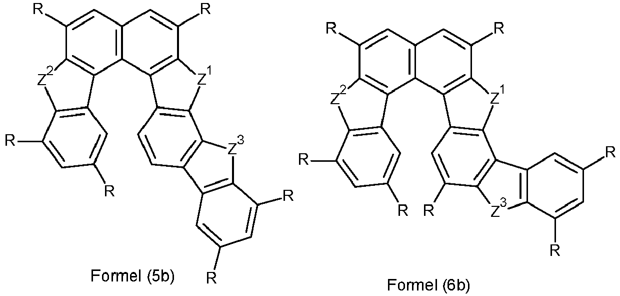 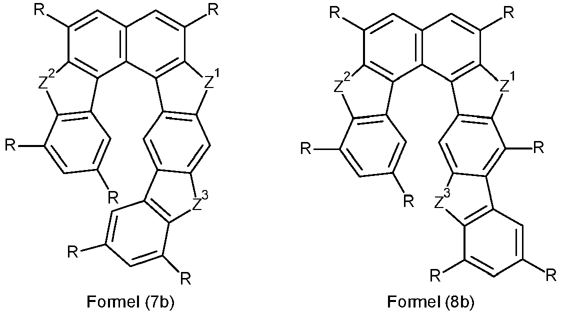 wobei die verwendeten Symbole die in Anspruch 1 genannten Bedeutungen aufweisen.

7. Verbindung nach einem oder mehreren der Ansprüche 1 bis 6, **dadurch gekennzeichnet, dass** Z¹ und Z² gleich gewählt sind.

8. Verbindung nach einem oder mehreren der Ansprüche 1 bis 7, **dadurch gekennzeichnet, dass** mindestens eines der Symbole Z¹, Z² und/oder Z³ für N-Ar steht.

9. Verbindung nach einem oder mehreren der Ansprüche 1 bis 8, ausgewählt aus den Strukturen der Formeln (3d) bis (8d), 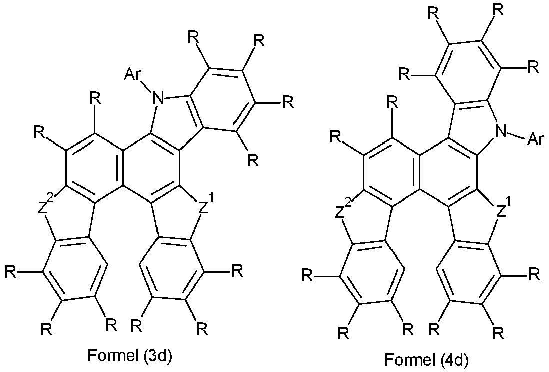 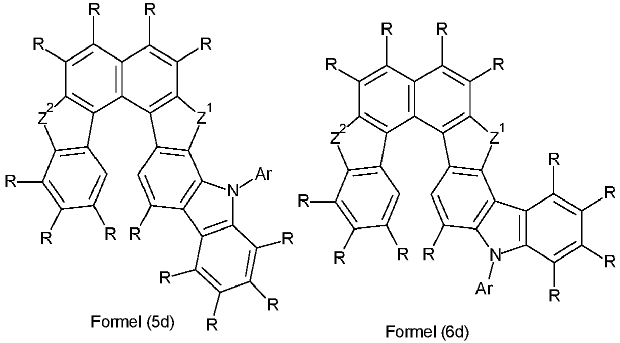 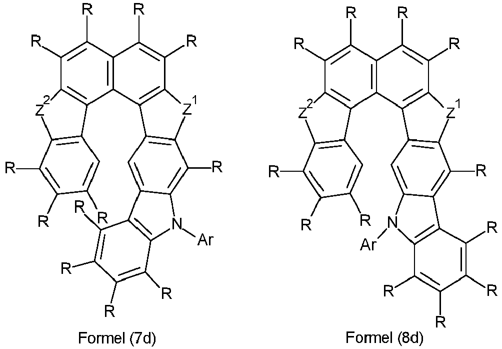 wobei die verwendeten Symbole die in Anspruch 1 genannten Bedeutungen aufweisen.

10. Verbindung nach einem oder mehreren der Ansprüche 1 bis 9, ausgewählt aus den Strukturen der Formeln (3e) bis (8e), 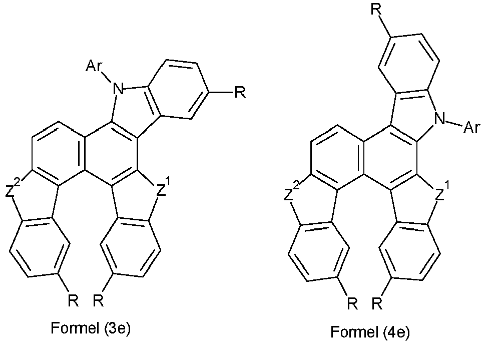 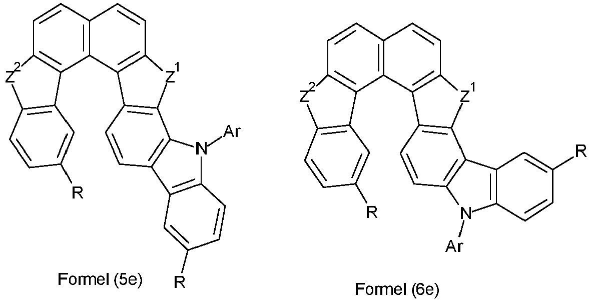 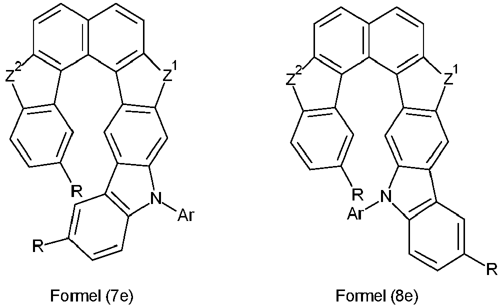 wobei die verwendeten Symbole die in Anspruch 1 genannten Bedeutungen aufweisen.

11. Formulierung, enthaltend mindestens eine Verbindung nach einem oder mehreren der Ansprüche 1 bis 10 und mindestens eine weitere Verbindung und/oder ein Lösemittel.

12. Verwendung einer Verbindung nach einem oder mehreren der Ansprüche 1 bis 10 in einer elektronischen Vorrichtung.

13. Elektronische Vorrichtung enthaltend mindestens eine Verbindung nach einem oder mehreren der Ansprüche 1 bis 10.

14. Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 10 in einer emittierenden Schicht als Matrixmaterial für phosphoreszierende Emitter oder für Emitter, die TADF zeigen, und/oder in einer emittierenden Schicht als fluoreszierender Emitter und/oder in einer Elektronentransportschicht und/oder in einer Lochblockierschicht und/oder in einer Lochtransportschicht und/oder in einer Exzitonenblockierschicht eingesetzt werden.

15. Elektronische Vorrichtung nach Anspruch 14, **dadurch gekennzeichnet, dass** die Verbindung nach einem oder mehreren der Ansprüche 1 bis 10 als Matrixmaterial für einen phosphoreszierenden Emitter in Kombination mit einem weiteren Matrixmaterial eingesetzt wird, wobei das weitere Matrixmaterial ausgewählt ist aus der Gruppe bestehend aus Biscarbazolen, verbrückten Carbazolen, Triarylaminen, Dibenzofuran-Carbazol-Derivaten, Dibenzofuran-Amin-Derivaten, Carbazolaminen, Indenocarbazolen und Indolocarbazolen.

## Claims

1. Compound of the formula (1), 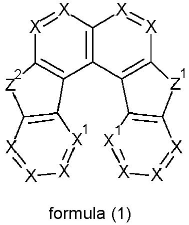 where the following applies to the symbols used:
X is on each occurrence, identically or differently, CR or N or two adjacent X stand for a group of the following formula (2), 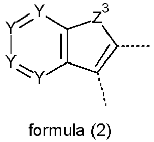 where the dashed bonds denote the linking of this group in the formula (1), with the proviso that the compound of the formula (1) contains one or two groups of the formula (2);
X¹ is CH;
Y is on each occurrence, identically or differently, CR or N;
Z¹, Z², Z³ are on each occurrence, identically or differently, O, S, N-Ar or CR₂;
Ar is on each occurrence, identically or differently, an aromatic or heteroaromatic ring system having 5 to 40 aromatic ring atoms, which may be substituted by one or more radicals R;
R is on each occurrence, identically or differently, H, D, F, N(Ar')₂, CN, OR¹, a straight-chain alkyl group having 1 to 10 C atoms or an alkenyl group having 2 to 10 C atoms or a branched or cyclic alkyl group having 3 to 10 C atoms, where the alkyl or alkenyl group may in each case be substituted by one or more radicals R¹, where one or more non-adjacent CH₂ groups may be replaced by O, or an aromatic or heteroaromatic ring system having 6 to 30 aromatic ring atoms, which may in each case be substituted by one or more radicals R¹; two radicals R may also form an aliphatic ring system with one another;
Ar' is on each occurrence, identically or differently, an aromatic or heteroaromatic ring system having 6 to 30 aromatic ring atoms, which may be substituted by one or more radicals R¹;
R¹ is on each occurrence, identically or differently, H, D, F, CN, OR², a straight-chain alkyl group having 1 to 10 C atoms or an alkenyl group having 2 to 10 C atoms or a branched or cyclic alkyl group having 3 to 10 C atoms, where the alkyl or alkenyl group may in each case be substituted by one or more radicals R², where one or more non-adjacent CH₂ groups may be replaced by O, or an aromatic or heteroaromatic ring system having 6 to 30 aromatic ring atoms, which may in each case be substituted by one or more radicals R²; two or more radicals R¹ may form an aliphatic ring system with one another;
R² is on each occurrence, identically or differently, H, D, F, CN or an aliphatic, aromatic or heteroaromatic organic radical, in particular a hydrocarbon radical, having 1 to 20 C atoms, in which, in addition, one or more H atoms may be replaced by F.

2. Compound according to Claim 1, selected from the compounds of the formulae (3) to (8), 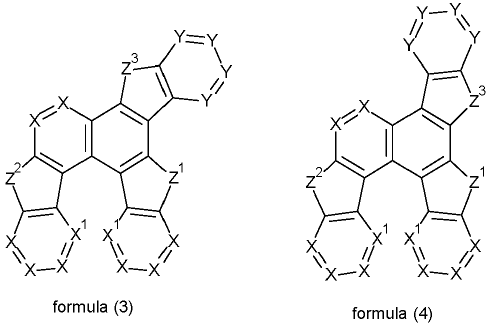 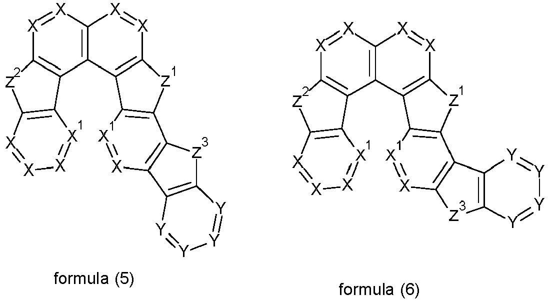 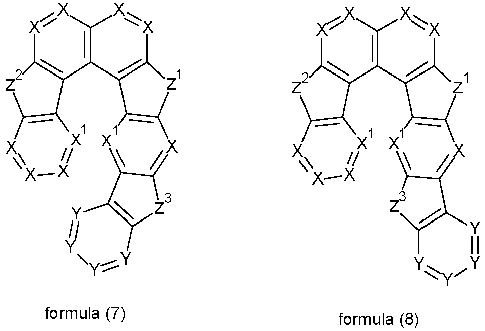 where the symbols used have the meanings given in Claim 1.

3. Compound according to Claim 1 or 2, **characterised in that** the compound contains precisely one group of the formula (2).

4. Compound according to one or more of Claims 1 to 3, **characterised in that** the group of the formula (2) stands for a group of the formula (2a), 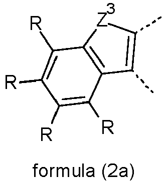 where the symbols used have the meanings given in Claim 1.

5. Compound according to one or more of Claims 1 to 4, selected from the structures of the formulae (3a) to (8a), 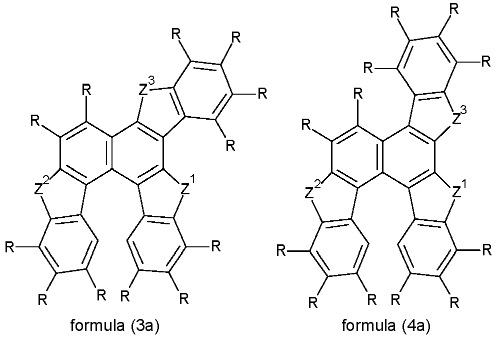 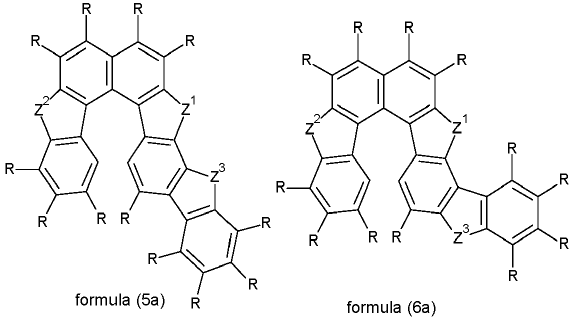 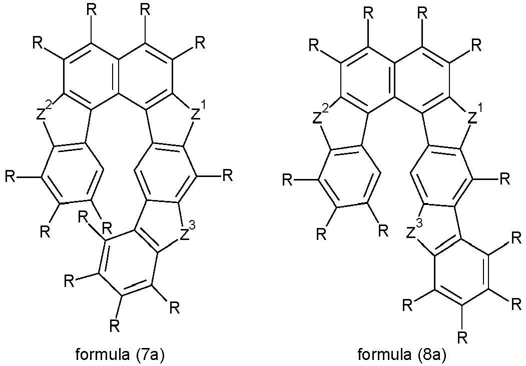 where the symbols used have the meanings given in Claim 1.

6. Compound according to one or more of Claims 1 to 5, selected from the structures of the formulae (3b) to (8b), 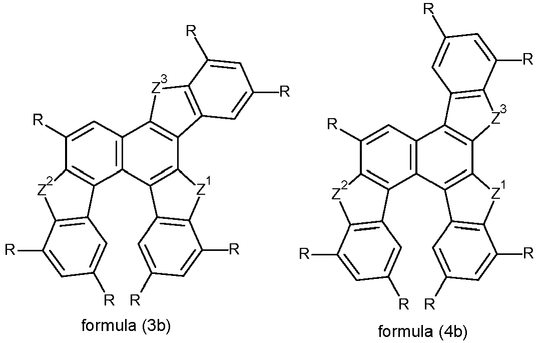 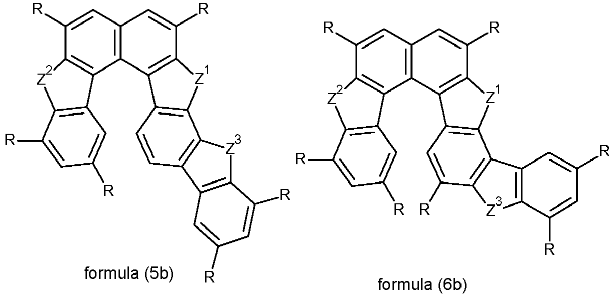 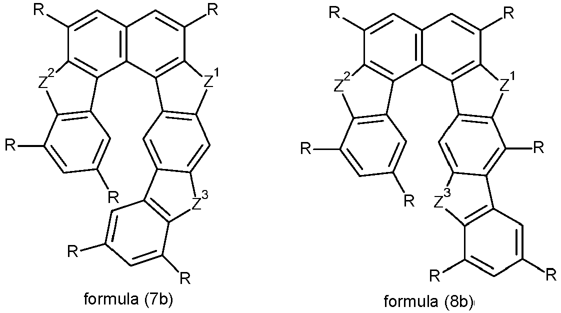 where the symbols used have the meanings given in Claim 1.

7. Compound according to one or more of Claims 1 to 6, **characterised in that** Z¹ and Z² are selected identically.

8. Compound according to one or more of Claims 1 to 7, **characterised in that** at least one of the symbols Z¹, Z² and/or Z³ stands for N-Ar.

9. Compound according to one or more of Claims 1 to 8, selected from the structures of the formulae (3d) to (8d), 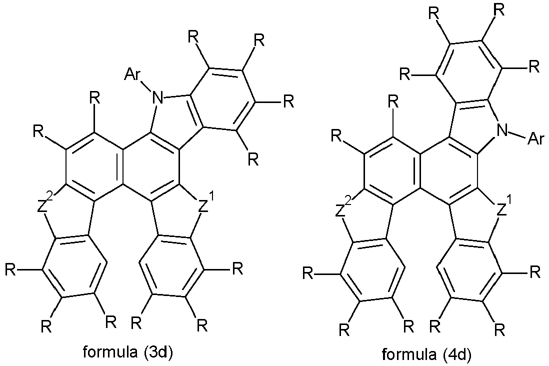 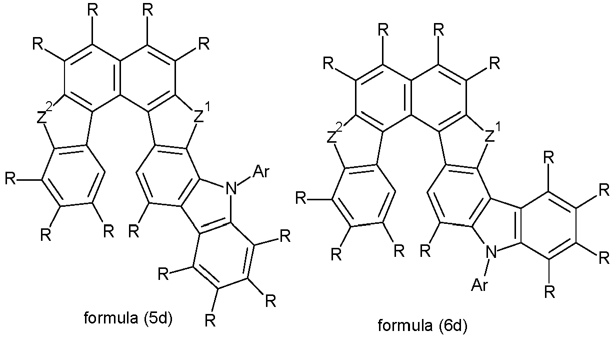 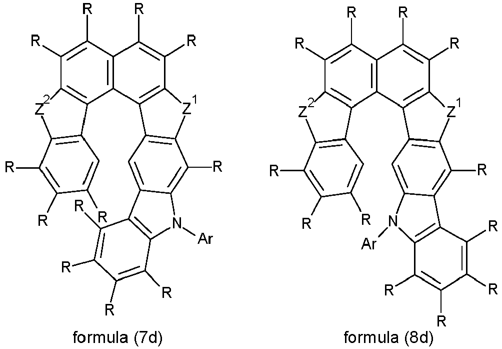 where the symbols used have the meanings given in Claim 1.

10. Compound according to one or more of Claims 1 to 9, selected from the structures of the formulae (3e) to (8e), 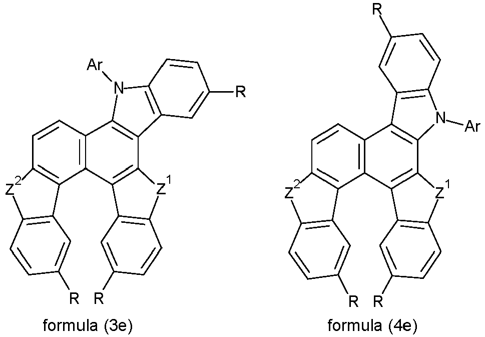 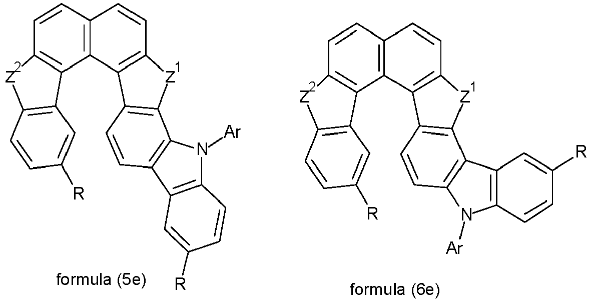 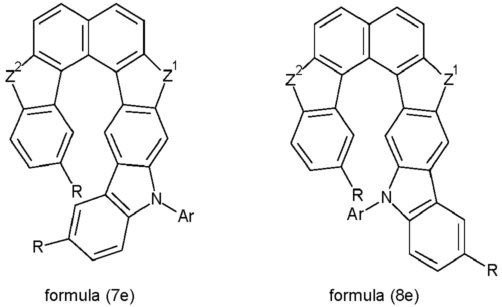 where the symbols used have the meanings given in Claim 1.

11. Formulation comprising at least one compound according to one or more of Claims 1 to 10 and at least one further compound and/or a solvent.

12. Use of a compound according to one or more of Claims 1 to 10 in an electronic device.

13. Electronic device containing at least one compound according to one or more of Claims 1 to 10.

14. Electronic device according to Claim 13, which is an organic electroluminescent device, **characterised in that** the compound according to one or more of Claims 1 to 10 is employed in an emitting layer as matrix material for phosphorescent emitters or for emitters which exhibit TADF, and/or in an emitting layer as fluorescent emitter and/or in an electron-transport layer and/or in a hole-blocking layer and/or in a hole-transport layer and/or in an exciton-blocking layer.

15. Electronic device according to Claim 14, **characterised in that** the compound according to one or more of Claims 1 to 10 is employed as matrix material for a phosphorescent emitter in combination with a further matrix material, where the further matrix material is selected from the group consisting of biscarbazoles, bridged carbazoles, triarylamines, dibenzofuran-carbazole derivatives, dibenzofuran-amine derivatives, carbazolamines, indenocarbazoles and indolocarbazoles.

## Revendications

1. Composé de formule (1), 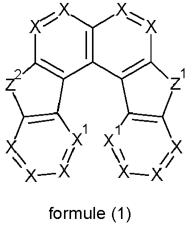 dans laquelle ce qui suit s'applique aux symboles utilisés :
X est à chaque occurrence, de manière identique ou différente, CR ou N ou deux X adjacents représentent un groupement de formule (2) suivante, 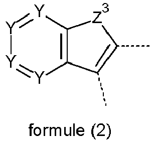 dans laquelle les liaisons en pointillés désignent la liaison de ce groupement dans la formule (1), à condition que le composé de formule (1) contienne un ou deux groupements de formule (2) ;
X¹ est CH ;
Y est à chaque occurrence, de manière identique ou différente, CR ou N ;
Z¹, Z², Z³ sont à chaque occurrence, de manière identique ou différente, O, S, N-Ar ou CR₂ ;
Ar est à chaque occurrence, de manière identique ou différente, un noyau aromatique ou hétéroaromatique ayant de 5 à 40 atomes de cycle aromatique, qui peut être substitué par un ou plusieurs radicaux R ;
R est à chaque occurrence, de manière identique ou différente, H, D, F, N(Ar')₂, CN, OR¹, un groupement alkyle à chaîne linéaire ayant de 1 à 10 atomes de C ou un groupement alcényle ayant de 2 à 10 atomes de C ou un groupement alkyle ramifié ou cyclique ayant de 3 à 10 atomes de C, où le groupement alkyle ou alcényle peut dans chaque cas être substitué par un ou plusieurs radicaux R¹, où un ou plusieurs groupements CH₂ non adjacents peuvent être remplacés par O, ou un noyau aromatique ou hétéroaromatique ayant de 6 à 30 atomes de cycle aromatique, qui peut dans chaque cas être substitué par un ou plusieurs radicaux R¹ ; deux radicaux R peuvent également former un noyau aliphatique l'un avec l'autre ;
Ar' est à chaque occurrence, de manière identique ou différente, un noyau aromatique ou hétéroaromatique ayant de 6 à 30 atomes de cycle aromatique, qui peut être substitué par un ou plusieurs radicaux R¹ ;
R¹ est à chaque occurrence, de manière identique ou différente, H, D, F, CN, OR², un groupement alkyle à chaîne linéaire ayant de 1 à 10 atomes de C ou un groupement alcényle ayant de 2 à 10 atomes de C ou un groupement alkyle ramifié ou cyclique ayant de 3 à 10 atomes de C, où le groupement alkyle ou alcényle peut dans chaque cas être substitué par un ou plusieurs radicaux R², où un ou plusieurs groupements CH₂ non adjacents peuvent être remplacés par O, ou un noyau aromatique ou hétéroaromatique ayant de 6 à 30 atomes de cycle aromatique, qui peut dans chaque cas être substitué par un ou plusieurs radicaux R² ; deux, ou plus, radicaux R¹ peuvent former un noyau aliphatique les uns avec les autres ;
R² est à chaque occurrence, de manière identique ou différente, H, D, F, CN ou un radical organique aliphatique, aromatique ou hétéroaromatique, en particulier un radical hydrocarboné, ayant de 1 à 20 atomes de C, où, de plus, un ou plusieurs atomes de H peuvent être remplacés par F.

2. Composé selon la revendication 1, choisi parmi les composés de formules (3) à (8), 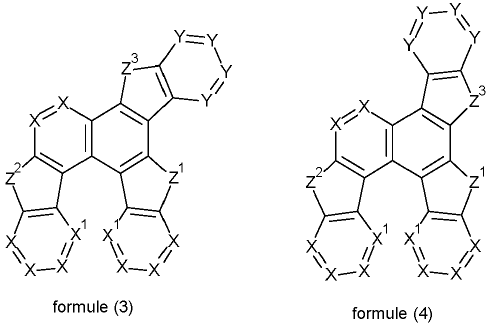 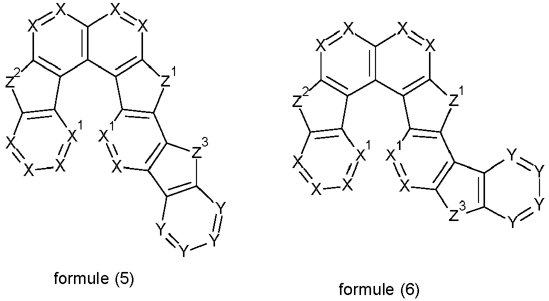 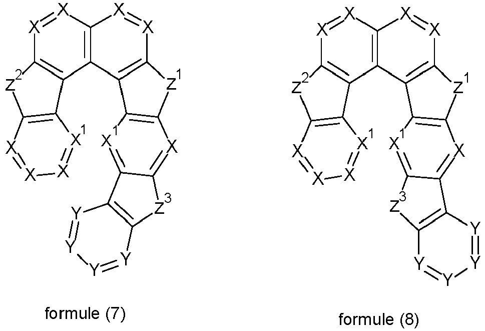 dans lesquelles les symboles utilisés revêtent les significations données selon la revendication 1.

3. Composé selon la revendication 1 ou 2, **caractérisé en ce que** le composé contient précisément un seul groupement de formule (2).

4. Composé selon l'une ou plusieurs parmi les revendications 1 à 3, **caractérisé en ce que** le groupement de formule (2) représente un groupement de formule (2a), 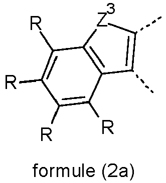 dans laquelle les symboles utilisés revêtent les significations données selon la revendication 1.

5. Composé selon l'une ou plusieurs parmi les revendications 1 à 4, choisi parmi les structures de formules (3a) à (8a), 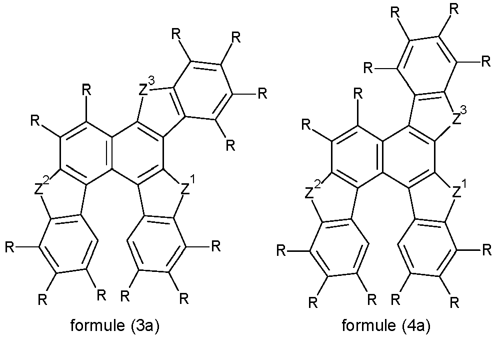 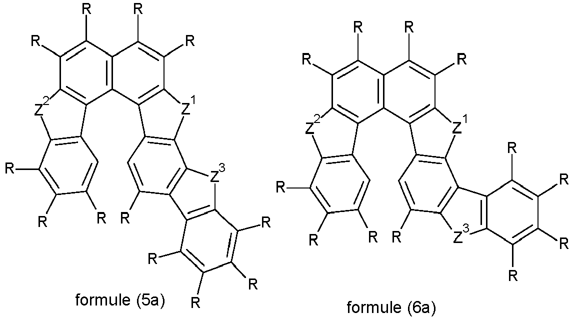 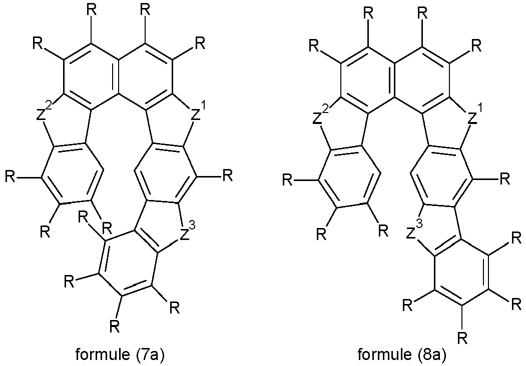 dans lesquelles les symboles utilisés revêtent les significations données selon la revendication 1.

6. Composé selon l'une ou plusieurs parmi les revendications 1 à 5, choisi parmi les structures de formules (3b) à (8b), 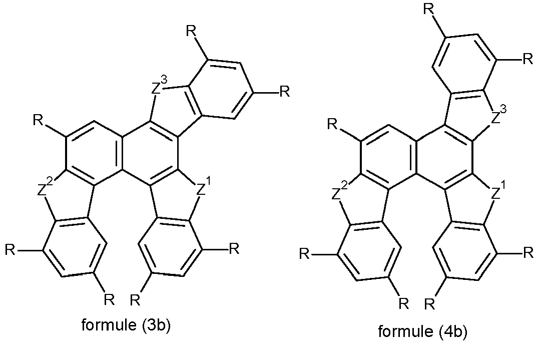 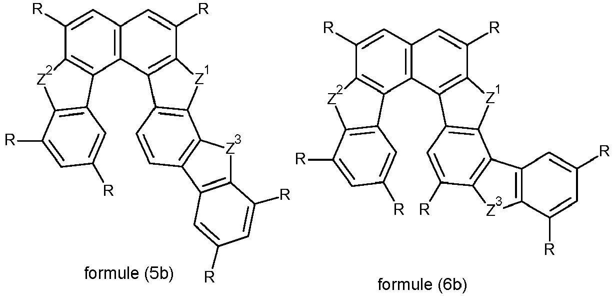 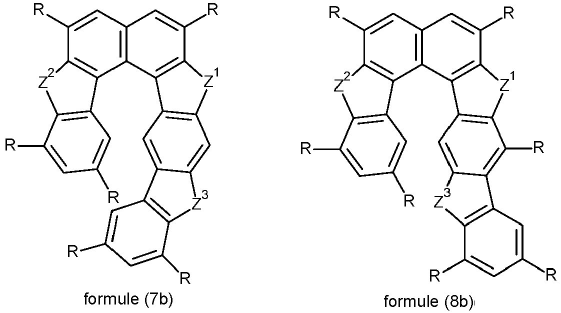 dans lesquelles les symboles utilisés revêtent les significations données selon la revendication 1.

7. Composé selon l'une ou plusieurs parmi les revendications 1 à 6, **caractérisé en ce que** Z¹ et Z² sont choisis de manière identique.

8. Composé selon l'une ou plusieurs parmi les revendications 1 à 7, **caractérisé en ce qu'**au moins l'un parmi les symboles Z¹, Z² et/ou Z³ représente N-Ar.

9. Composé selon l'une ou plusieurs parmi les revendications 1 à 8, choisi parmi les structures de formules (3d) à (8d), 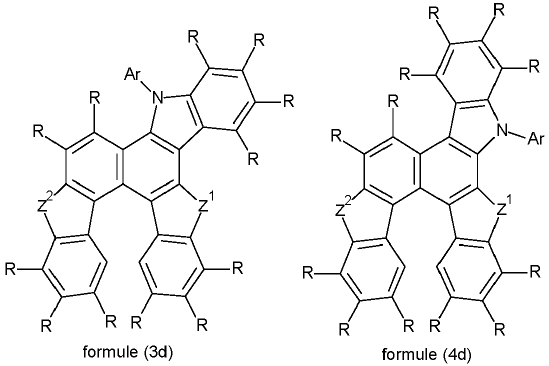 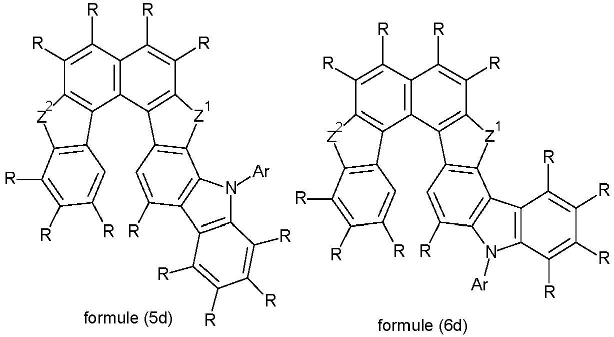 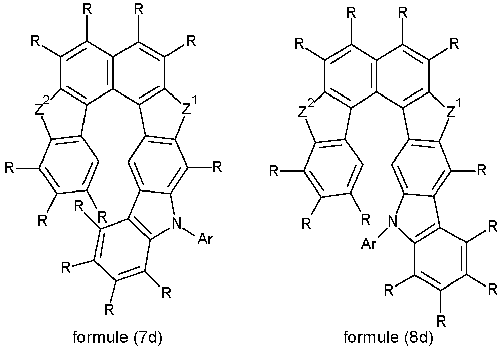 dans lesquelles les symboles utilisés revêtent les significations données selon la revendication 1.

10. Composé selon l'une ou plusieurs parmi les revendications 1 à 9, choisi parmi les structures de formules (3e) à (8e), 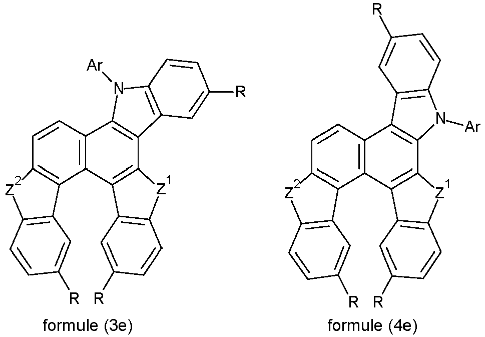 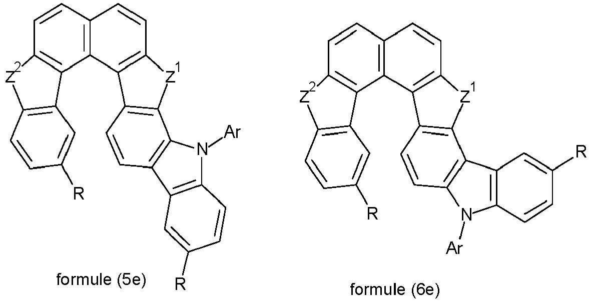 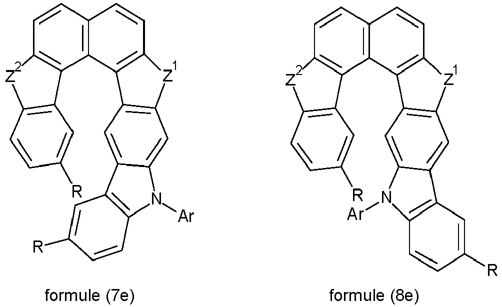 dans lesquelles les symboles utilisés revêtent les significations données selon la revendication 1.

11. Formulation comprenant au moins un composé selon l'une ou plusieurs parmi les revendications 1 à 10, et au moins un autre composé et/ou un solvant.

12. Utilisation d'un composé selon l'une ou plusieurs parmi les revendications 1 à 10, dans un dispositif électronique.

13. Dispositif électronique contenant au moins un composé selon l'une ou plusieurs parmi les revendications 1 à 10.

14. Dispositif électronique selon la revendication 13, qui est un dispositif électroluminescent organique, **caractérisé en ce que** le composé selon l'une ou plusieurs parmi les revendications 1 à 10 est employé dans une couche émettrice comme matériau de matrice pour des émetteurs phosphorescents ou pour des émetteurs présentant une TADF, et/ou dans une couche émettrice comme émetteur fluorescent et/ou dans une couche de transport d'électrons et/ou dans une couche de blocage de trous et/ou dans une couche de transport de trous et/ou dans une couche de blocage d'excitons.

15. Dispositif électronique selon la revendication 14, **caractérisé en ce que** le composé selon l'une ou plusieurs parmi les revendications 1 à 10 est employé comme matériau de matrice pour un émetteur phosphorescent en combinaison avec un autre matériau de matrice, où l'autre matériau de matrice est choisi dans le groupe constitué par les biscarbazoles, les carbazoles pontés, les triarylamines, les dérivés de dibenzofurane-carbazole, les dérivés de dibenzofurane-amine, les carbazolamines, les indénocarbazoles et les indolocarbazoles.
